# EUROPÄISCHE PATENTSCHRIFT

(11) **EP 0 192 180 B1**
(45) Veröffentlichungstag und Bekanntmachung des Hinweises auf die Patenterteilung: **09.08.1989**
(21) Anmeldenummer: 86101772.1
(22) Anmeldetag: 12.02.1986
(51) Int. Cl.: C07D 403/12, C07D 239/62, C07D 263/34, C07D 277/34, C07D 249/08, C07D 235/26, C07D 271/06, C07D 413/12, C07D 239/60, C07D 263/32, C07D 263/58

(54) **5-(Azolyloxyphenylcarbamoyl)barbitursäure-derivate als Wirkstoffe für Anthelmintika**
5-(Azolyloxyphenylcarbamoyl)-barbituric acid derivatives as active agents in anthelmics
Dérivés de l'acide (azolyloxyphénylcarbamyl)-5 barbiturique comme agents actifs pour antihelminthiques

(30) Priorität: 15.02.1985 CH 705/85
(43) Veröffentlichungstag der Anmeldung: 27.08.1986
(73) Patentinhaber: CIBA-GEIGY AG, 4002 Basel (CH)
(72) Erfinder: Sturm, Elmar, Dr., CH-4147 Aesch (CH); Gallay, Jean Jacques, Dr., CH-4312 Magden (CH); Kristinsson, Haukur, Dr., CH-4051 Basel (CH); Pissiotas, Georg, Dr., D-7850 Lörrach (DE)
(74) Vertreter: Zumstein, Fritz, Dr.

(56) Entgegenhaltungen:
- DE-A- 2 225 071
- DE-A- 2 641 979
- DE-A- 2 719 777
- DE-A- 2 936 457
- DE-A- 3 238 079
- US-A- 3 679 695
- US-A- 4 005 202
- CHEMICAL ABSTRACTS, Band 99, Nr. 10, 5. September 1973, Columbus, Ohio, USA, B. DE SOUSA; W. SCHMID; H. HEFTI; D. BELLUS "A biological evaluation of pyrethroids as potential moth-proofing agents. Part 3. A complete and highly effective moth-proofing agent comprising a pyrethroid and a hexahydropyrimidine", p. 63, Zusammenfassnung Nr. 72077x
- CHEMICAL ABSTRACTS, Band 79, Nr. 21, 26. November 1973, Columbus, Ohio, USA, T. NAGAI; Y. FUKUSHIMA; T. KURODA; H. SHIMIZU; S. SEKIGUCHI; K. MATSUI "Rearrangement of 2-aryloxybenzazoles", p. 400, Zusammenfassung Nr. 126394f

## Beschreibung

Die vorliegende Erfindung betrifft neue substituierte 5-(Azolyloxyphenylcarbamoyl)barbitursäurede- rivate mit anthelmintischer Wirksamkeit, Mittel, die diese Wirkstoffe als Aktivsubstanzen enthalten, sowie diese Wirkstoffe bzw. Mittel zur Verwendung in einem Verfahren zur Bekämpfung von Helminthen, insbesondere von Nematoden, Cestoden und Trematoden in Haus- und Nutztieren, vor allem in Warmblütern, insbesondere in Säugetieren. Die Erfindung betrifft ferner die Herstellung der neuen Wirkstoffe sowie der sie enthaltenden Mittel.

Die neuen Verbindungen entsprechen der allgemeinen Formel I 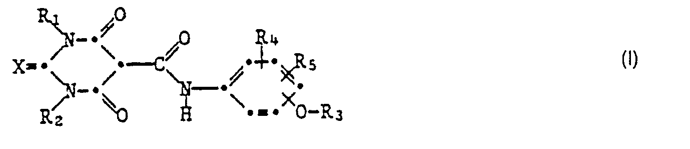 sowie deren tautomeren Formen und Salze, worin
X für Sauerstoff oder Schwefel steht;
R₁ C₁-C₆-Alkyl, C₁-C₆-Alkoxy, C₃-C₆-Cycloalkyl oder Allyl bedeutet ;
R₂ für C₁-C₆-Alkyl oder Allyl steht ;
R₃ für ein unsubstituiertes oder substituiertes, über Kohlenstoff gebundenes, heteroaromatisches Fünfringazol steht, ausgewählt aus der Reihe Benzimidazol, Benzoxazol, Benzthiazol, Imidazol, Oxazol, Thiazol, Oxadiazol, Thiadiazol, und Triazol ; und
R₄ und R₅ unabhängig voneinander Wasserstoff, C₁-C₆-Alkyl, C₁-C₆-Haloalkyl, C₁-C₆-Alkoxy oder C₁-C₆-Haloalkoxy bedeuten.

Die Verbindungen der Formel I können beispielsweise in folgenden tautomeren Formen vorliegen :

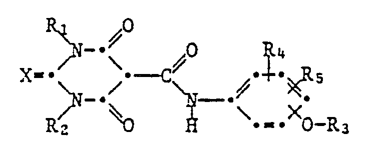

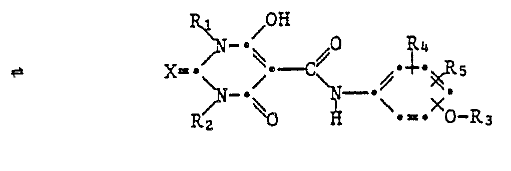

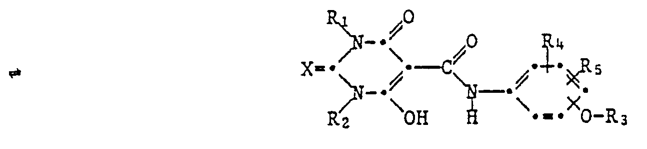

Die Erfindung betrifft alle tautomeren Formen der Verbindungen der Formel I.

5(Phenylcarbamoyl)barbitursäure derivate, deren Phenylteil halogeniert, methyliert oder trifluormethyliert ist, werden in der Deutschen Offenlegungschrift DE-2,719,777 als Insektizide, insbesondere zur Kontrolle pflanzenschädigender Insekten, offenbart.

Das Fünfringazol (R₃) ist somit stets über ein Kohlenstoffatom an das phenolische O-Atom des Aminophenol-Teils des Moleküls gebunden und kann seinerseits unsubstituiert oder substituiert vorliegen. Verbindungen der Formel I, worin R₃ beispielsweise eine der nachfolgend angegebenen Bedeutungen hat, bilden je nach Definition von R₃ eine der folgenden erfindungsgemässen bevorzugten Gruppen a bis f bildet :

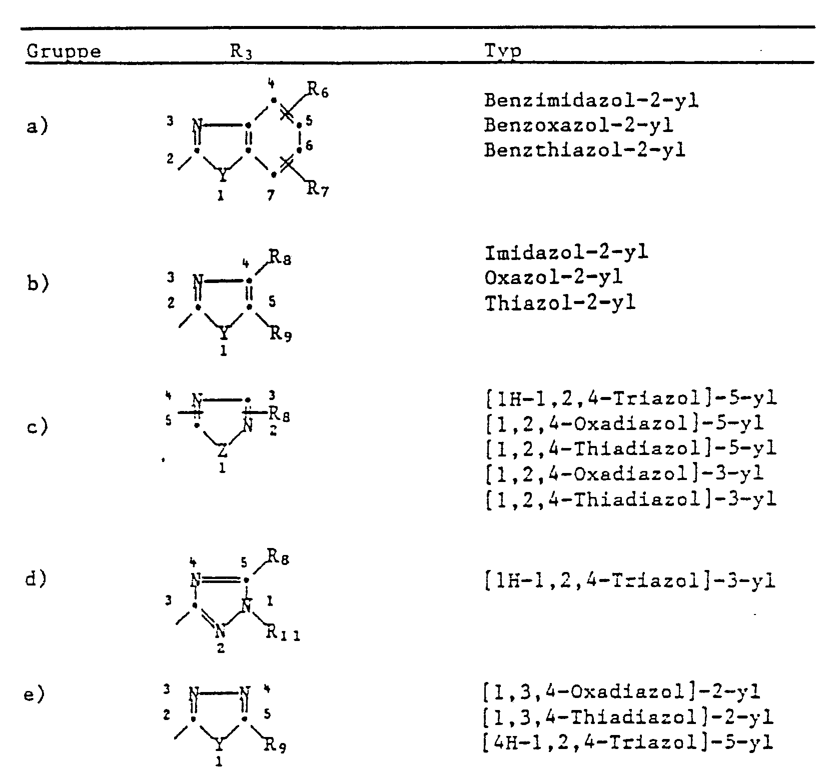

oder 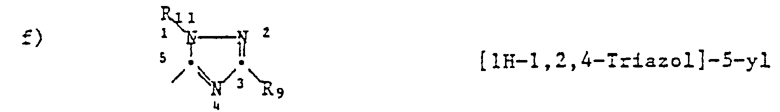 wobei
Y für Sauerstoff, Schwefel oder NR₁₀ steht;
Z für Sauerstoff, Schwefel oder NH steht;
R₆ und R₇ unabhängig voneinander Wasserstoff, C₁-C₄-Alkyl, C₁-C₄-Haloalkyl, C₁-C₄-Alkoxy, C₁-C₄-Haloalkoxy, C₁-C₄-Alkylthio, Halogen, Nitro oder Cyano bedeuten ;
R₈ und Rg unabhängig voneinander für Wasserstoff, C₁-C₆-Alkyl, C₁-C₆-Haloalkyl, C₁-C₆-Alkoxy, C₁-C₆-Alkylthio, Halogen, Nitro oder unabhängig voneinander für unsubstituiertes oder durch Halogen oder C₁-C₃-Alkyl substituiertes C₃-C₇-Cycloalkyl stehen ;
R₁₀ Wasserstoff oder C₁-C₆-Alkyl bedeutet ; und
R₁₁ für C₁-C₆-Alkyl steht.

Innerhalb der Gruppen b bis f sind auch diejenigen Verbindungen der Formel I interessant, worin Y, Z, R₁₀ und R₁₁ wie oben definiert sind und R₈ und Rg unabhängig voneinander jedoch für unsubstituiertes oder durch Halogen oder C₁-C₃-Alkyl substituiertes C3-CrCycloalkyl, vorzugsweise Cyclopropyl oder Cyclohexyl stehen.

Der Substituent R₃ hat somit u.a. folgende Bedeutungen : 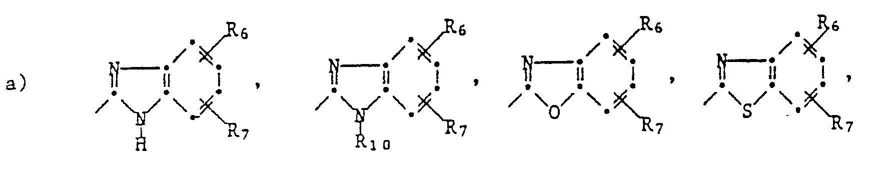 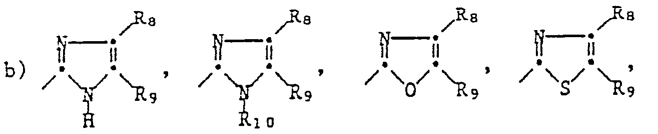 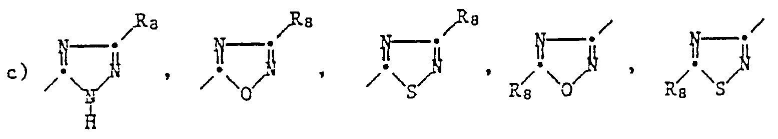 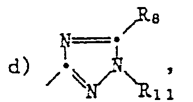 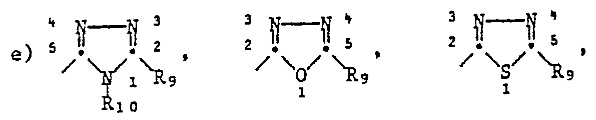 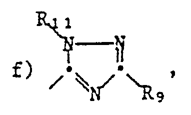 wobei die Substituenten R₆ bis R₁₁ wie vorstehend definiert sind.

Bevorzugt sind alle Gruppen von Verbindungen der Formel I, die formal durch Kombination mit dem Molekülfragment 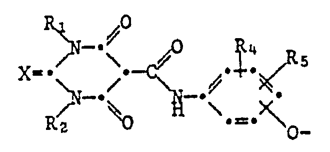 worin X, R₁, R₂, R₄ und R₅ die unter Formel I angegebenen Bedeutungen haben, mit einem unter a bis f genannten Azole R₃ gebildet werden. Jede dieser Gruppen ist Bestandteil der vorliegenden Erfindung.

Bevorzugt innerhalb aller genannten Untergruppen sind jene Verbindungen der Formel I, worin R₁ für Methyl oder Methoxy steht, R₂ Methyl bedeutet ; R₄ und R₅ unabhängig voneinander für Wasserstoff, Methyl, CF₃, OCH₃, OCHF₂ oder OCF₃ stehen.

Innerhalb der Gruppe la sind diejenigen Verbindungen der Formel I zusätzlich bevorzugt, worin R₆ und R₇ unabhängig voneinander für Wasserstoff, Methyl, CF₃, Methoxy, Halomethoxy, Fluor, Chlor oder Brom stehen und die übrigen Substituenten wie oben definiert sind.

Innerhalb der Gruppen Ib bis If sind diejenigen Verbindungen der Formel I zusätzlich bevorzugt, worin R₈ und Rg unabhängig voneinander für Wasserstoff, Methyl, Ethyl, C₃-C₇-Cycloalkyl, CF₃, C₂F₅, C₃F₇, CCl₃, CHCI₂, CH₂CI, SCH₃ oder Halogen stehen ; R₁₀ Wasserstoff oder Methyl bedeutet und R₁₁ für C₁-C₄-Alkyl, insbesondere Methyl steht.

Unter dem Begriff Alkyl selbst oder als Bestandteil eines anderen Substituenten sind im Rahmen der vorliegenden Erfindung je nach Anzahl der angegebenen Kohlenstoffatome folgende Gruppen zu verstehen z.B. : Methyl, Ethyl, Butyl, Pentyl oder Hexyl, sowie ihre isomeren, wie z.B. Isopropyl, Isobutyl, sek.-Butyl, tert.-Butyl, Isopentyl. Ein durch Halogen substituiertes Alkyl (Haloalkyl) steht allein oder als Bestandteil von Haloalkoxy für einen einfach bis perhalogenierten Alkylsubstituenten, wie z.B. CHCI₂, CH₂Cl, CCI₃, CF₃, C₂F₅, CH₂CH₂CI, C₂Cl₅, CHFCHCI₂, CF₂H, vorzugsweise für CF₃. Unter Halogen soll hier und im folgenden Fluor, Chlor, Brom oder Jod, vorzugsweise Fluor, Chlor oder Brom, vor allem aber Chlor verstanden werden. Cycloalkyl steht für Cyclopropyl, Cyclobutyl, Cyclopentyl oder Cyclohexyl.

Bevorzugte Einzelsubstanzen sind :
1,3-Dimethyl-5-[4-(6-brom-benzthiazol-2-yloxy)phenylcarbamoyl]-barbitursäure (Nr. 1.8) ;
1,3-Dimethyl-5-[4-(6-chlor-benzthiazol-2-yloxy)phenylcarbamoyl]-barbitursäure (Nr. 1.5) ;
1,3-Dimethyl-5-[4-(6-fluor-(benzthiazol-2-yloxy)phenylcarbamoyl]-barbitursäure (Nr. 1.38) ;
1,3-Dimethyl-5-[4-(3-dichlormethyl-1,2,4-thiadiazol-5-yloxy)phenylcarbamoyl]barbitursäure (Nr. 3.1) ;
1,3-Dimethyl-5-[4-(5-tert-butyl-1,3,4-oxadiazol-2-yloxy)phenylcarbamoyl]barbitursäure (Nr. 4.1) ;
1,3-Dimethyl-5-[4-(5-tert.-butyl-1,3,4-thiadiazol-2-yloxy)phenylcarbamoyl]barbitursäure (Nr. 4.2) ;
1-Methyl-3-methoxy-5-[4-(5-tert.-butyl-1,3,4-thiadiazol-2-yloxy)phenylcarbamoyl]barbitursäure (Nr. 4.55) ;
1,3-Dimethyl-5-[4-(5-isopropyl-1,3,4-oxadiazol-2-yloxy)phenylcarbamoyl]barbitursäure (Nr. 4.10) ;
1,3-Dimethyl-5-[4-(1-isopropyl-3-trifluormethyl-1H-1,2,4-triazol-5-yloxy)phenylcarbamoyl]barbitursäure (Nr. 5.2) ;
1,3-Dimethyl-5-[2,6-dimethyl-4-(1-methyl-3-heptafluorpropyl-1H-1,2,4-triazol-5-yloxy)phenylcarbamoyl]barbitursäure (Nr. 5.28) ;
1,3-Dimethyl-5-[4-(1-isopropyl-3-pentafluorethyl-1 H-1,2,4-triazol-5-yloxy)phenylcarbamoyl]barbitursäure (Nr. 5.6) ;
1,3-Dimethyl-5-[4-( 1-isopropyl-3-trifluormethyl-1 H-1,2,4-triazol-5-yloxy)-2,6-dimethyl-phenylcarbamoyl]barbitursäure (Nr. 5.5) ;
1,3-Dimethyl-5-[3-(1-methyl-3-trifluormethyl-1H-1,2,4-triazol-5-yloxy)phenylcarbamoyl]barbitursäure (Nr. 5.16) ; 1,3-Dimethyl-5-[4-(6-trifluormethyl-benzthiazol-2-yloxy)phenylcarbamoyl]barbitursäure (Nr. 1.39) ;
1,3-Dimethyl-5-[3-methoxy-4-(5-tert.-butyl-1,3,4-thiadiazol-2-yloxy)phenylcarbamoyl]barbitursäure (Nr. 4.54) ; 1,3-Dimethyl-5-[3-methoxy-4-(6-chlor-benzthiazol-2-yloxy)phenylcarbamoyl]barbitursäure (Nr. 1.11) ;
1,3-Dimethyl-5-[4-(5,6-dichlor-benzthiazol-2-yloxy)phenylcarbamoyl]barbitursäure (Nr. 1.9) ;
1,3-Dimethyl-5-[4-(6,7-dichlor-benzthiazol-2-yloxy)phenylcarbamoyl]barbitursäure (Nr. 1.9) ;
1,3-Dimethyl-5-[4-(benzthiazol-2-yloxy)phenylcarbamoyl]barbitursäure (Nr. 1.32) ;
1,3-Dimethyl-5-[4-(5-chlor-6-fluor-benzthiazol-2-yloxy)phenylcarbamoyl]barbitursäure (Nr. 1.43) ;
1,3-Dimethyl-5-[4-(7-chlor-6-fluor-benzthiazol-2-yloxy)phenylcarbamoyl]barbitursäure (Nr. 1.43) ;
1,3-Dimethyl-5-[4-(6-trifluormethoxy-benzthiazol-2-yloxy)phenylcarbamoyl]barbitursäure (Nr. 1.41) ;
1,3-Dimethyl-5-[2-isopropyl-4-(5-cyclopropyl-1,3,4-oxadiazol-2-yloxy)phenylcarbamoyl]barbitursäure (Nr. 4.59) ; 1,3-Dimethyl-5-[4-(5-cyclohexyl-1,3,4-thiadiazol-2-yloxy)phenylcarbamoyl]barbitursäure (Nr. 4.66) ;
1,3-Dimethyl-5-[4-(1-methyl-3-pentafluorethyl-1H-1,2,4-triazo)-5-yloxy)phenylcarbamoyl]barbitursäure (Nr. 5.37) ; 1,3-Dimethyl-5-[4-(6-methoxy-benzthiazol-2-yloxy)phenylcarbamoyl]barbitursäure (Nr. 1.40) ;
1,3-Dimethyl-5-(4-(5-cyclopropyl-1,3,4-thiadiazol-2-yloxy)phenylcarbamoyl]barbitursäure(Nr. 4.69) ;
1,3-Dimethyl-5-[4-(6-chlor-7-fluor-benzthiazol-2-yloxy)phenylcarbamoyl]barbitursäure (Nr. 1.78) ;
1-Methyl-3-methoxy-5-[4-(6-chlor-7-fluor-benzthiazol-2-yloxy)phenylcarbamoyl]barbitursäure (Nr. 1.79) ;
1,3-Dimethyl-5-[3-methoxy-4-(6-chlor-7-fluorbenzthiazol-2-yloxy)phenylcarbamoyl]barbitursäure (Nr. 1.80).

Zu den in Frage kommenden Salzen der Verbindungen der Formel I zählen beispielsweise die Metall-, Ammonium- oder Aminsalze, wobei Natrium-, Kalium-, Aluminium-, Ammonium- oder Alkylaminsalze, besonders Triäthylaminsalze, bevorzugt sind.

Die erfindungsgemässen neuen Verbindungen der Formel I besitzen überraschenderweise ein sehr günstiges Wirkungsspektrum gegen im Tierorganismus, vor allem in Warmblütern und Säugetieren, parasitierende Helminthen. Sie sind vor allem gegen Nematoden, bei erhöhter Dosis auch gegen Cestoden und Trematoden mit gutem Erfolg anwendbar. Dabei zeichnen sie sich vor allem dadurch aus, dass sie auch gegen benzimidazol-resistente, insbesondere gegen thiabendazolresistente Species voll wirksam sind, wobei unter « Thiabendazol » der Wirkstoff 2-[4-Thiazolyl]-benzimidazol zu verstehen ist.

Die Verbindungen der Formel werden hergestellt, indem man zur Reaktion bringt :
a) einen Ester der Formel II 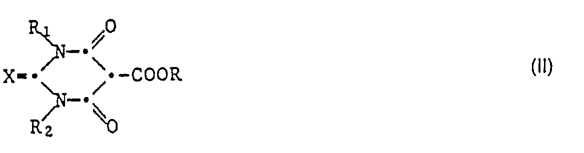 mit einem Anilinderivat der Formel III 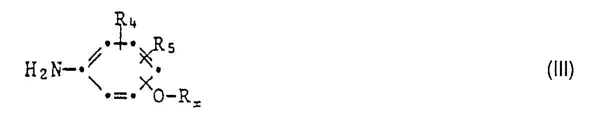 worin R eine Niederalkyl- oder eine gegebenenfalls durch Nitro substituierte Phenylgruppe darstellt, oder
b) eine substituierte Barbitursäure der Formel IV 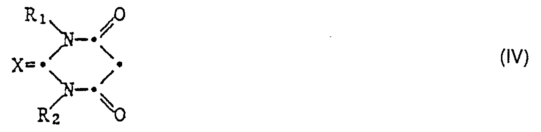 mit einem substituierten Phenylisocyanat der Formel V 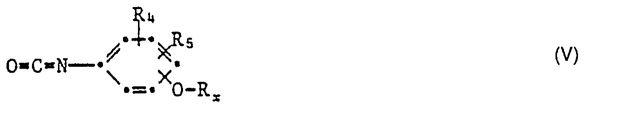 oder
c) eine substituierte Barbitursäure der Formel IV mit einem substituierten Benzoylazid der Formel VI 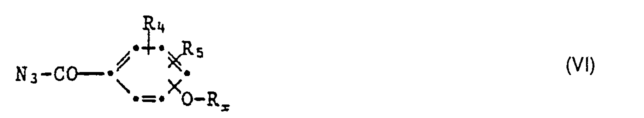 umsetzt, wobei die Substituenten X, R₁, R₂, R₄ und R₅ in den Formeln II bis VI die unter Formel I angegebenen Bedeutungen haben und Rₓ für Wasserstoff oder einen unter R₃ angegebenen Rest steht und man in den Fällen in denen Rₓ für Wasserstoff steht, entweder auf der Stufe der Ausgangsprodukte eines der Hydroxy-Derivate (Rₓ = H) der Formeln III, V oder VI mit einer Verbindung der Formel XX 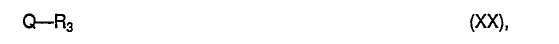 worin Q für eine übliche Abgangsgruppe steht, verethert und dann zum Endprodukt weiterreagieren lässt oder die Hydroxy-Derivate der Formeln III, V oder VI zuerst mit einem der Verbindungen II oder IV zu einem Hydroxy-Derivat (Rₓ = H) der Formel I' umsetzt, worin X, Rᵢ, R₂, R₄ und R₅ wie unter Formel I definiert sind und Rₓ für Wasserstoff steht und dann erst das Hydroxy-Derivat der Formel I' mit der Verbindung der Formel XX verethert.

Man kann die besagte Veretherung somit entweder auf der Stufe der Ausgangsprodukte oder auf der unmittelbaren Vorstufe I' durchführen.

Q steht in Formel XX entweder für eine der üblichen Abgangsgruppen, z. B. Halogen, insbesondere Chlor, Brom oder Jod ; für eine Sulfonyloxygruppe, insbesondere Benzolsulfonyloxy, Paratosyloxy oder Niederalkylsulfonyloxy bevorzugt Mesyloxy ; oder für eine Acyloxygruppe wie Trifluoracetyloxy. Q steht auch für eine Hydroxygruppe oder gemäss « Synthesis » 1979, p. 561-569, für den Rest

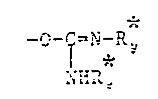

worin Ry^{*} und R_{z}^{*} Organylreste, insbesondere Niederalkyl oder gegebenenfalls substituierte Phenylreste repräsentieren.

Die Herstellungsvarianten (a) und (c) werden bei Reaktionstemperaturen zwischen 50° und 250 °C, vorzugsweise 70° bis 220 °C, durchgeführt. Variante (b) erfordert Reaktionstemperaturen zwischen 0° und 220 °C, insbesondere 0° bis 200 °C. Die Veretherung findet bei Reaktionstemperaturen von 50° bis 150 °C, vorzugsweise 80° bis 120°C, in reaktionsinerten Lösungs- oder Verdünnungsmitteln statt. Die Reaktionen (a), (b), und (c) können bei normalen oder erhöhtem Druck und in Abwesenheit oder vorzugsweise in Gegenwart reaktionsinerter Lösungs- oder Verdünnungsmittel durchgeführt werden, wobei in manchen Fällen vorteilhafterweise mit einer Base gearbeitet wird.

Die Herstellung der erfindunsgemässen Salze von Verbindungen der Formel I erfolgt durch übliche Neutralisation der freien Säure mit einer Base, insbesondere einer physiologisch verträglichen Base. Vorzugsweise genannt seien Alkalisalze wie Natrium-, Kalium- oder Lithiumsalze sowie Ammoniumsalze und Trialkylaminsalze wie z. B. das bevorzugte Triethylaminsalz. Die Neutralisation wird in einem reaktionsinerten polaren Lösungsmittel, z. B. einem Alkanol, Ester oder einer etherartigen Verbindung, durchgeführt.

Für die Herstellung der erfindungsgemässen Wirksubstanzen geeignete Lösungs- oder Verdünnungsmittel sind z. B. Ether und etherartige Verbindungen wie Dialkylether (Diethylether, Diisopropylether, tert.-Butylmethylether usw.), Anisol, Dioxan, Tetrahydrofuran ; aliphatische und aromatische Kohlenwasserstoffe wie Benzol, Toluol, Petrolether ; halogenierte Kohlenwasserstoffe wie Chlorbenzol, Methylenchlorid, Chloroform, Ethylenchlorid, Tetrachlorkohlenstoff, Tetrachlorethylen ; Nitrile wie Acetonitril, Propionitril ; N,N-dialkylierte Amide wie Dimethylformamid ; Dimethylsulfoxid ; Ketone wie Aceton, Diethyiketon, Methylethylketon sowie insbesondere für Veretherungsreaktion Wasser und Alkohole wie z. B. Methanol, Ethanol, Isopropanol oder Butanol ; und ganz allgemein Gemische solcher Lösungsmittel untereinander.

Als Basen kommen organische und anorganischen Basen in Betracht ; z. B. vorzugsweise tertiäre Amine wie Trialkylamine (Trimethylamin, Triethylamin, Tripropylamin usw.), Pyridin und Pyridinbasen (z. B. 4-Dimethylaminopyridin, 4-Pyrrolidylaminopyridin usw.), Picoline und Lutidine sowie Oxide, Hydroxide, Carbonate und Hydrogencarbonate von Alkali- und Erdalkalimetallen (z. B. CaO, BaO, NaOH, KOH, Ca(OH)₂, KHC0₃, NaHC0₃, Ca(HC0₃)₂, K₂C0₃, Na₂C0₃ usw.), femer Acetate wie z. B. CH₃COONa oder CHₛCOOK. Darüber hinaus eignen sich als Basen auch Alkalialkoholate wie z. B. Natriumethylat, Natriumpropylat, Kalium-tert.-butylat oder Natriummethylat. Die Base wird für die Verfahrensvarianten (a), (b) und (c) vorteilhafterweise in bezug auf die Reaktanden in 10 bis 100 % der äquimolaren Menge und in 200 % der äquimolaren Menge für die Veretherungsreaktion zugesetzt.

In manchen Fällen kann es von Vorteil sein, wenn die Reaktion unter Schutzgasatmosphäre durchgeführt wird. Geeignete Schutzgase sind z. B. Stickstoff, Helium, Argon oder Kohlendioxid.

Verbindungen der Formel 111, worin Rₓ die für R₃ unter Formel I angegebenen Definitionen hat, können aus den zugrundeliegenden Hydroxyanilinen der Formel III, worin Rₓ für Wasserstoff steht, durch Veretherung mit Verbindungen der Formel XX hergestellt werden. Diese Veretherungsreaktion erfolgt wie weiter oben beschrieben. Es können jedoch auch statt der Hydroxyaniline, die entsprechenden Nitrophenole eingesetzt werden, dann muss die Nitrogruppe jedoch in einer Folgereaktion zur Aminogruppe reduziert werden. Diese Verbindungen (Rₓ = Rₛ) werden hier und im folgenden als Zwischenprodukte der Formel III' bezeichnet. Sie stellen besonders geeignete Zwischenprodukte zur Herstellung der wertvollen Wirkstoffe der Formel I dar. Aufgrund ihrer strukturellen Beschaffenheit lassen sie sich (vgl. Herstellungsvariante a) in einfacher Weise in die Verbindungen der Formel I überführen. Es handelt sich hierbei im Einzelnen somit um Verbindungen der Formel III', 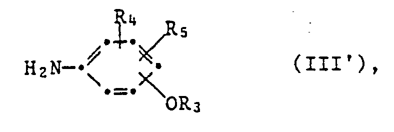 worin R₄, R₅ und R₃ die unter Formel I angegebenen Bedeutungen haben. Dabei kann man die Verbindungen der Formel III' analog zu den Verbindungen der Formel I, je nach den für welches der eingangs genannten Fünfringazole der Substituent R₃ steht, in sechs Gruppen a) bis f) einteilen, wobei jede dieser Gruppierungen eine bevorzugte Ausführungsform darstellt. Besonders bevorzugt sind jedoch diejenigen Verbindungen der Formel III' die zu den in den Tabellen 1 bis 5 genannten Endprodukten führen und diejenigen, die explicite in Tabelle 6 genannt sind.

Die in den Herstellungsvarianten (a), (b) und (c) genannten Ausgangsstoffe sind bekannt oder können in analoger Weise wie die bekannten Substanzen hergestellt werden.

Das beschriebene Herstellungsverfahren ist einschliesslich aller Varianten (a), (b), (c) und den Veretherungsreaktionen ein Bestandteil vorliegender Erfindung.

Die erfindungsgemässen Wirkstoffe der Formel I können in unterschiedlichen tautomeren Formen vorliegen, nämlich in der Keto- oder Enolform oder in einem Gemisch aus Keto- und Enolform. Diese Erfindung betrifft sowohl die einzelnen Tautomeren als auch deren Gemische, aber auch die Salze jeder dieser Formen und deren Herstellung.

Die Erfindung schliesst auch die erfindungsgemäßen Substanzen und Mittel zur Verwendung in einem Verfahren zum prophylaktischen Schutz von Tieren gegen parasitäre Helminthen ein, das dadurch gekennzeichnet ist, dass man die Wirkstoffe der Formel I bzw. die Wirkstofformulierungen als Zusatz zum Futter oder zu den Tränken oder auch in fester oder flüssiger Form oral, durch Injektion oder mittels der Pour-on-Methode den Tieren appliziert.

Bei dieser Verwendung im Verfahren zur Helminthenbekämpfung bzw. bei den erfindungsgemässen Helminthenbekämpfungsmitteln können die Wirkstoffe der Formel I in allen tautomeren Formen, deren Mischungen oder in Form ihrer Salze eingesetzt werden.

Unter den bei Warmblütern vorkommenden Endoparasiten verursachen namentlich die Helminthen grosse Schäden. So können von diesen Parasiten befallene Tiere nicht nur ein verlangsamtes Wachstum, sondern erhebliche physiologische Beeinträchtingungen aufweisen, die sogar zur Mortalität führen können. Daher ist es von grosser Bedeutung, therapeutische Mittel zu entwickeln, die sich zur Bekämpfung von Helminthen und deren Entwicklungsstadien sowie zur Vorbeugung gegen den Befall durch diese Parasiten eignen. Besonders gefährliche Wurmkrankheiten sind solche, die durch im Magen-Darmtrakt und anderen Organen parasitierende Nematoden, Cestoden und Trematoden hervorgerufen werden und vor allem bei Wiederkäuern, wie Schafen, Rindern und Ziegen sowie Pferden, Schweinen, Rotwild, Hunden, Katzen und Geflügel auftreten.

Die durch Helminthiasen verursachten Schäden können bei chronischem und vor allem bei epidemischem Auftreten der Wurmerkrankungen in Viehherden beträchtlich sein. Sie äussern sich unter anderem in Produktivitäts-Verminderungen, geschwächter Widerstandskraft und erhöhter Mortalität. Bekämpfung und Vorbeugung von Helminthiasen gelten deshalb als vordringliche Aufgabe, um derartige vor allem volkswirtschaftlich ins Gewicht fallende Schäden zu vermeiden oder zu mindern.

In der vorliegenden Beschreibung werden unter dem Begriff « Helminthen » insbesondere parasitische Würmer verstanden, die zu den Phyla Plathelminthes (Cestoden, Trematoden) und Nemathelminthes (Nematoden und Verwandte) gehören, also Bandwürmer, Saugwürmer und Rundwürmer des Gastrointestinal-Traktes und anderer Organe (z. B. Leber, Lunge, Niere, Lymphgefässe, Blut etc.). Es sind zwar eine Reihe von Stoffen mit anthelmintischer Wirkung bekannt, die für die Bekämpfung der verschiedenen Helminthen species vorgeschlagen wurden. Diese vermögen jedoch nicht voll zu befriedigen, sei es, dass bei verträglicher Dosierung eine Ausschöpfung ihres Wirkungsspektrums nicht möglich ist, oder dass sie in therapeutisch wirksamen Dosen unerwünschte Nebenwirkungen oder Eigenschaften zeigen. In diesem Zusammenhang spielt auch die heute vermehrt auftretende Resistenz gegen bestimmte Stoffklassen eine immer bedeutendere Rolle. Das beispielsweise in der Literatur beschriebene « Albendazol » (British Pat. No. 1464326 ; Am. J. Vet. Res. 38, 1425-1426 (1977) ; Am. J. Vet. Res. 37, 1515-1516 (1976) ; Am. J. Vet. Res. 38, 807-808 (1977) ; Am. J. Vet. Res. 38, 1247-1248 (1977)) besitzt zwar ein begrenztes anthelmintisches Wirkungsspektrum bei Wiederkäuem, seine Wirkung gegen Benzimidazol-resistente Nematoden und adulte Leberegel ist jedoch unzureichend, da vor allem die pathologisch wichtigen unreifen Wanderformen der letzteren bei den für das Wirtstier verträglichen Dosierungen nicht angegriffen werden.

Ueberraschenderweise wurde nun festgestellt, dass die Wirkstoffe der Formel I nicht nur - wie bereits erwähnt - eine intensive anthelmintische Wirksamkeit mit breitem Wirkungsspektrum gegen Nematoden, Cestoden und Trematoden, sondern darüber hinaus zusätzlich eine günstige Warmblütertoxizität besitzen.

Die erfindungsgemässen neuen Wirkstoffe der Formel sind beispielsweise zur Bekämpfung parasitärer Nematoden der Ordnungen (nach K.I. Skrajabin)
Rhabditida
Ascaridida
Spirurida
Trichocephalida
oder zur Bekämpfung von Cestoden der Ordnungen (nach Wardle & McLeod)
Cyclophyllidae
Pseudophyllidae
oder zur Bekämpfung von Trematoden der Ordnung
Digenea

bei Haus- und Nutztieren wie Rindern, Schafen, Ziegen, Pferden, Schweinen, Katzen, Hunden und Geflügel geeignet. Sie können den Tieren sowohl als Einzeldosis als auch wiederholt verabreicht werden, wobei die einzelnen Gaben je nach Tierart vorzugsweise zwischen 1 und 500 mg pro kg Körpergewicht betragen. Durch eine protrahierte Verabreichung wird in manchen Fällen eine bessere Wirkung erzielt oder man kann mit geringeren Gesamtdosen auskommen.

Die erfindungsgemässen Mittel werden hergestellt, indem die Wirkstoffe der Formel 1 mit flüssigen und/oder festen Formulierungshilfsstoffen durch schrittweises Vermischen und/oder Vermahlen derart in Kontakt gebracht werden, sodass eine anwendungskonforme optimale Entfaltung der anthelmintischen Aktivität der Formulierung erzielt wird.

Die Formulierungsschritte können durch Kneten, Granulieren (Granulate) und gegebenenfalls Pressen (Pellets) ergänzt werden.

Als Formulierungshilfsstoffe dienen beispielsweise feste Trägerstoffe, Lösungsmittel und gegebenenfalss oberflächenaktive Stoffe (Tenside).

Zur Bereitung der erfindungsgemässen Mittel werden folgende Formulierungshilfsstoffe verwendet:

Feste Trägerstoffe wie z. B. Kaolin, Talkum, Bentonit, Kochsalz, Calciumphosphat, Kohlenhydrate, Cellulosepulver, Baumwollsaatmehl, Polyäthylenglykoläther, gegebenenfalls Bindemittel wie z. B. Gelatine, lösliche Cellulosederivate, gewünschtenfalls unter Zusatz von oberflächenaktiven Stoffen wie ionischen oder nicht-ionischen Dispersionsmitteln ; ferner natürliche Gesteinsmehle wie Calcit, Montmorillonit oder Attapulgit. Zur Verbesserung der physikalischen Eigenschaften können auch hochdisperse Kieselsäure oder hochdisperse saugfähige Polymerisate zugesetzt werden. Als gekömte, adsorptive Granulatträger kommen poröse Typen, wie z. B. Bimsstein, Ziegelbruch, Sepiolit oder Bentonit, als nicht sorptive Trägermaterialien z. B. Calcit oder Sand in Frage. Darüber hinaus kann eine Vielzahl von vorgranulierten Materialien anorganischer oder organischer Natur wie insbesondere Dolomit oder zerkleinertes Pflanzenmaterial verwendet werden.

Als Lösungsmittel kommen in Frage : Aromatische Kohlenwasserstoffe, bevorzugt die Fraktionen Cₑ bis C₁₂, wie z. B. Xylolgemische oder substituierte Naphthaline, Phthalsäureester wie Dibutyl- oder Dioctylphthalat ; aliphatische Kohlenwasserstoffe wie z. B. Cyclohexan oder Paraffine, Alkohole und Glykole sowie deren Ether und Ester, wie z. B. Ethanol, Ethylenglykol, Ethylenglykolmonomethyl- oder Ethylether, Ketone wie z. B. Cyclohexanon, stark polare Lösungsmittel wie z. B. N-Methyl-2-pyrrolidon, Dimethylsulfoxid oder Dimethylfo;- mamid, sowie gegebenenfalls epoxydierte Pflanzenöle wie z. B. epoxydiertes Kokosnussöl oder Sojaöl und Wasser.

Als oberflächenaktive Verbindungen kommen je nach Art des zu formulierenden Wirkstoffes der Formel I nicht-ionogene, kation- und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen, als auch wasserlösliche synthetische oberflächenaktive Verbindungen sein.

Als Seifen seien die Alkali-, Erdalkali- oder gegebenenfalls substituierten Ammoniumsalze von höheren Fettsäuren (CIO-C22), wie z. B. die Na- oder K-Salze der Oel- oder Stearinsäure, oder von natürlichen Fettsäuregemischen, die z. B. aus Kokosnuss- oder Talgöl gewonnen werden können.

Häufig werden sog. synthetische Tenside verwendet, insbesondere Fettsulfonate. Fettsulfate. sulfonierte Benzimidazolderivate oder Alkylsulfonate.

Die Fettsulfonate oder -sulfate liegen in der Regel als Alkali-, Erdalkali- oder gegebenenfalls substituierte Ammoniumsalze vor und weisen einen Alkylrest mit 8 bis 22 C-Atomen auf, wobei Alkyl auch den Alkylteil von Acylresten einschliesst, z. B. das Na- oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Ethylenoxid-Addukten. Die sulfonierten Benzimidazolderivate enthalten vorzugsweise 2 Sulfonsäuregruppen und einen Fettsäurerest mit 8-22 C-Atomen. Alkylarylsulfonate sind z. B. die Na-, Ca- oder Triethanolaminsalze der Dodecylbenzolsulfonsäure, der Dibutylnaphthalinsulfonsäure oder eines Naphthalinsulfonsäure-Formaldehydkondensationsproduktes.

Ferner können auch entsprechende Phosphate, wie z. B. Salze des Phosphorsäureesters eines p-Nonylphenol-(4-14)-Ethylenoxyd-Adduktes oder Phospholipide, als Formulierungshilfsstoffe Anwendung finden.

Als nicht-ionische Tenside kommen in erster Linie Polyglykoletherderivate von aliphatischen oder cycloaliphatischen Alkoholen, gesättigten oder ungesättigten Fettsäuren und Alkylphenolen in Frage, die 3 bis 30 Glykolethergruppen und 8 bis 20 Kohlenstoffatome im (aliphatischen) Kohlenwasserstoffrest und 6 bis 18 Kohlenstoffatome im Alkylrest der Alkylphenole enthalten können.

Weitere geeignete nicht-ionische Tenside sind die wasserlöslichen, 20 bis 250 Ethylenglykolethergruppen und 10 bis 100 Propylenglykolethergruppen enthaltenden Polyethylenoxidaddukte an Polypropylenglykol, Ethylendiaminopolypropylenglykol und Alkylpolypropylenglykol mit 1 bis 10 Kohlenstoffatomen in der Alkylkette. Die genannten Verbindungen enthalten üblicherweise pro Propylenglykol-Einheit 1 bis 5 Ethylenglykoleinheiten.

Als Beispiele nicht-ionischer Tenside seien Nonylphenolpolyethoxyethanole, Ricinusölpolyglykolether, Polypropylen-Polyethylenoxydaddukte, Tributylphenoxypolyethoxyethanol, Polyethylenglykol und Octylphenoxypolyethoxyethanol erwähnt.

Ferner kommen auch Fettsäureester von Polyoxyethylensorbitan wie das Polyoxyethylensorbitan-trioleat in Betracht.

Bei den kationischen Tensiden handelt es sich vor allem um quartäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl- oder niedrige Hydroxyalkylreste aufweisen. Die Salze liegen vorzugsweise als Halogenide, Methylsulfate oder Ethylsulfate vor, z. B. das Stearyltrimethylammoniumchlorid oder das Benzyldi(2-chlorethyl)ethylammoniumbromid.

Die in der Formulierungstechnik gebräuchlichen Tenside sind u. a. in folgenden Publikationen beschrieben :

« Mc Cutcheon's Detergents and Emulsifiers Annual » MC Publishing Corp., Ridgewood New Jersey, 1981 ; Helmut Stache « Tensid-Taschenbuch » Carl Hanser-Verlag München/Wien 1981.

Als Bindemittel für Tabletten und Boli kommen chemisch abgewandelte, in Wasser oder Alkohol lösliche, polymere Naturstoffe in Frage, wie Stärke-, Cellulose- oder Proteinderivate (z. B. Methylcellulose, Carboxymethylcellulose, Ethylhydroxyethylcellulose, Proteine wie Zein, Gelatine und dergleichen) sowie synthetische Polymere wie z. B. Polyvinylalkohol, Polyvinylpyrrolidon etc. Ferner sind in Tabletten Füllstoffe, (z. B. Stärke, mikrokristalline Cellulose, Zucker, Milchzucker etc), Gleitmittel und Sprengmittel enthalten.

Liegen die anthelmintischen Mittel in Form von Futterkonzentraten vor, so dienen als Trägerstoffe z. B. Leistungsfutter, Futtergetreide oder Proteinkonzentrate. Solche Futterkonzentrate oder -mittel können ausser den Wirkstoffen noch Zusatzstoffe, Vitamine, Antibiotika, Chemotherapeutika, oder andere Pestizide, vomehmlich Bakteriostaika, Fungistatika, Coccidiostatika, oder auch Hormonpräparate, Stoffe mit anaboler Wirkung oder das Wachstum begünstigende, die Fleischqualität von Schlachttieren beeinflussende oder in anderer Weise für den Organismus nützliche Stoffe enthalten. Werden die Mittel oder die darin enthaltenen Wirkstoffe der Formel I direkt dem Futter oder den Viehtränken zugesetzt, so enthält das Fertigfutter oder die Fertigtränke die Wirkstoffe vorzugsweise in einer Konzentration von etwa 0,0005 bis 0,02 Gewichtsprozent (5-200 ppm).

Die Applikation der erfindungsgemässen Mittel an die zu behandelnden Tiere kann peroral, parenteral, subcutan oder topikal durchgeführt werden, wobei die Mittel in Form von Lösungen, Emulsionen, Suspensionen (Drenchs), Pulvern, Tabletten, Boli und Kapseln vorliegen.

Die erfindungsgemässen anthelmintischen Mittel enthalten in der Regel 0,1 bis 99 Gew.%, insbesondere 0,1 bis 95 Gew.-%, Wirkstoff der Formel und 99,9 bis 1 Gew.-%, insbesondere 99,9 bis 5 Gew.-%, eines festen oder flüssigen Zusatzstoffes, darunter 0 bis 25 Gew.-%, insbesondere 0,1 bis 25 Gew.-%, eines Tensides.

Während als Handelsware eher konzentrierte Mittel bevorzugt werden, verwendet der Endverbraucher in der Regel verdünnte Mittel.

Solche Mittel können noch weitere Zusätze wie Stabilisatoren, Entschäumer, Viskositätsregulatoren, Bindemittel, Haftmittel sowie andere Wirkstoffe zur Erzielung spezieller Effekte enthalten.

Derartige vom Endverbraucher verwendete anthelmintische Mittel sind ebenfalls ein Bestandteil der vorliegenden Erfindung.

Die nachfolgenden Beispiele dienen zur näheren Erläuterung der Erfindung, ohne dieselbe einzuschränken.

### Herstellungsbeispiele

### Beispiel 1 : Herstellung von

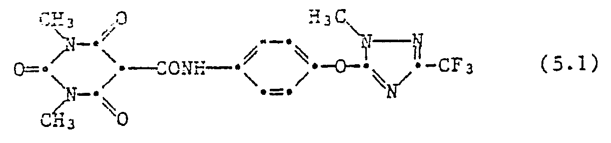

1,3-Dimethyl-5-[4-(1-methyl-3-trifluormethyl-1H-1,2,4-triazol-5-yloxy)phenylcarbamoyl]barbitursäure

### a) Herstellung des Ausgangsproduktes

### 1-Methyl-3-triffuormethyl-5-(4-aminophenoxy)-1H-1,2,4-triazol

10,9 g p-Aminophenol in 120 ml Dimethylsulfoxid werden mit 8 g pulverisierter KOH versetzt und das Gemisch 0,5 Stunden gerührt. Anschliessend werden 22,9g 1-Methyl-3-trifluormethyl-5-methylsulfonyl-1H-1,2,4-triazol zugesetzt, die Reaktionsmischung weitere 2 Stunden gerührt und danach auf 1 Liter Eiswasser gegossen. Das ausgefallene Produkt wird abfiltriert mit Wasser gewaschen und getrocknet. Ausbeute : 19,6 g (76 % der Theorie [d. Th.]). Fp. 111-112°C.

### b) Herstellung des Endproduktes

22,8 g 1,3-Dimethylbarbitursäure-5-carbonsäureethylester und 25,8 g 1-Methyl-3-trifluormethyl-5-(4-amino- phenoxy)-1H-1,2,4-triazol werden in 200 ml Toluol 4 Stunden unter Rückfluss erhitzt. Das beim Abkühlen auf Raumtemperatur ausfallende Rohprodukt wird abfiltriert, mit Diethylether gewaschen und getrocknet. Ausbeute : 39,2 g (89 % der Theorie [d. Th.]). Fp. 202-204 °C.

### Beispiel 2 : Herstellung von

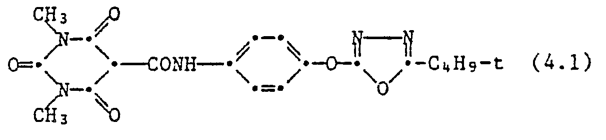

1,3-Dimethyl-5-[4-(5-tert-butyl-1,3,4-oxadiazol-2-yloxy)phenylcarbamoyl]barbitursäure

### a) Herstellung des Ausgangsproduktes

### 5-tert.-Butyl-2-(4-aminophenoxy)-1,3,4-oxadiazol

10,9 g 4-Aminophenol in 100 ml Dimethylsulfoxid werden mit 7,3 g pulverisiertem KOH versetzt und das Gemisch 15 Minuten gerührt. Dann werden 20,4 g 5-tert.-Butyl-2-methylsulfonyl-1,3,4-oxadiazol zugegeben, die Reaktionsmischung 12 Stunden bei Raumtemperatur gerührt, anschliessend mit Eiswasser versetzt, mit Diethylether extrahiert und die vereinigten Extrakte getrocknet und eingedampft. Der ölige Rückstand wird mit Petrolether versetzt, wobei das Produkt kristallisiert. Nach dem Abfiltrieren und Trocknen erhält man 16,5 g (71 % d. Th.). Fp. 80-81 °C.

### b) Herstellung des Endproduktes

22,8 g 1,3-Dimethylbarbitursäure-5-carbonsäureethylester und 23,3 g 5-tert.-Butyl-2-(4-aminophenoxy)-1,3,4-oxadiazol werden 4 Stunden in 250 ml Toluol unter Rückfluss erhitzt. Das beim Abkühlen ausfallende Produkt wird abfiltriert, mit Diethylether gewaschen und getrocknet. Ausbeute : 37,7 g (91 % d. Th.). Fp. 200-203 °C.

c) Die Herstellung von 5-tert.-Butyl-1,3,4-oxadiazol und weiterer 5-substituierten 1,3,4-Oxadiazole ist in der Literatur beschrieben (vgl. Deutsche Offenlegungsschrift DE-31 45 422 A1).

### Beispiel 3 : Herstellung von

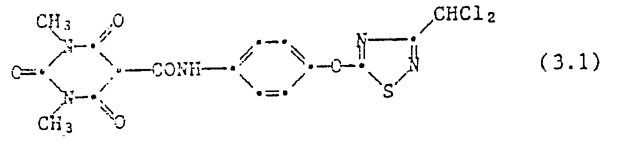

### 1,3-Dimethyl-5-[4-(3-dichlormethyl-1,2,4-thiadiazol-5-yloxy)phenylcarbamoyl]barbitursäure

### a) Herstellung des Ausgangsproduktes

### 3-Dichlormethyl-5-(4-aminophenoxy)-1,2,4-thiadiazol

5,5 g 4-Aminophenol und 5,6 g pulverisiertes Kaliumhydroxid werden in 150 ml Dimethylsulfoxid 30 Minuten bei Raumtemperatur gerührt. Dann werden 10,0 g 5-Chlor-3-dichlormethyl-1,2,4-thiadiazol in 50 ml Dimethylsulfoxid zugetropft, wobei die Temperatur auf 40 °C ansteigt. Nach zweistündigem Rühren wird das Reaktionsgemisch auf Eiswasser gegossen und das Produkt mit Essigsäureethylester extrahiert. Die vereinigten Extrakte werden getrocknet und eingeengt. Das ölige Rohprodukt wird mit Silicagel gereinigt. Ausbeute : 8,5 g der Titelsubstanz in Form eines gelben Oels, das in der nächsten Stufe eingesetzt wird.

### b) Herstellung des Endproduktes

Eine Lösung von 12 g 1,3-Dimethylbarbitursäure-5-carbonsäureethylester und 15g 3-Dichlormethyl-5-(4-aminophenoxy)-1,2,4-thiadiazol in 300 ml Toluol wird 2 Stunden unter Rückfluss erhitzt. Anschliessend wird die gelbe Reaktionslösung bis zur Trockene eingedampft und der Rückstand in Dichlormethan gerührt, wobei die Titelsubstanz auskristallisiert. Diese wird abfiltriert und getrocknet. Man erhält 19 g blassgelber Kristalle. Fp. 195-198 °C.

### Beispiel 4 : Herstellung von

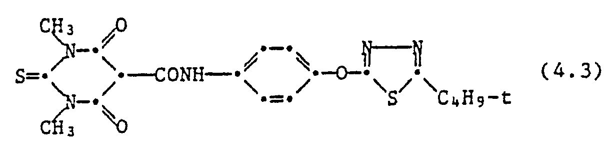

### 1,3-Dimethyl-5-[4-(5-tert.-butyl-1,3,4-thiadiazol-2-yloxy)phenylcarbamoyl]-2-thiobarbitursäure

1,7 g 1,3-Dimethylbarbitursäure-5-carbonsäureethylester und 1,75 g 4-(5-tert.-Butyl-1,3,4-thiadiazol-2-ylo- xy)anilin werden in 30 ml Toluol 30 Stunden unter Rückfluss erhitzt. Nach dem Abkühlen auf Raumtemperatur wird das Gemisch mit 40 ml Hexan verdünnt, der gebildete Niederschlag abfiltriert, mit Isopropanol gewaschen und getrocknet. Ausbeute : 2,5 g (82 % d. Th.). Fp. 174-176 °C.

### Beispiel 5 : Herstellung von

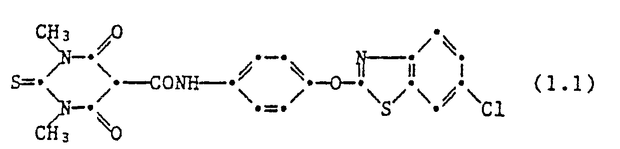

### 1,3-Dimethyl-5-j4-(6-chlor-benzthiazol-2-yloxy)-phenylcarbamoyl]-2-thiobarbitursäure

2,5 g 1,3-Dimethylthiobarbitursäure-5-carbonsäuremethylester und 2,8 g 4-(5-Chlor-benzthiazol-2-ylo- xy)anilin werden mit 30 ml Ethanol und 3 ml Dimethylformamid gemischt und das Gemisch 7 Stunden unter Rückfluss erhitzt. Beim Abkühlen auf Raumtemperatur bildet sich ein Niederschlag, der abfiltriert, mit Ethanol gewaschen und getrocknet wird. Ausbeute : 3,7 g (75 % d. Th.). Fp. 213-215 °C.

### Beispiel 6 : Herstellung von

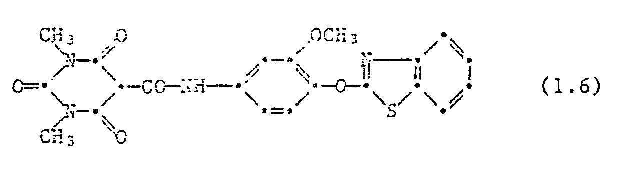

### 1,3-Dimethyl-5-[3-methoxy-4-(benzthiazol-2-yloxy)phenylcarbamoyl]-barbitursäure

### a) Herstellung des Ausgangsproduktes

### 1,3-Dimethyl-5-(4-hydroxy-3-methoxy-phenylcarbamoyl)barbitursäure.

6,85 g 1,3-Dimethyl-5-ethoxycarbonylbarbitursäure und 4,2 g 4-Hydroxy-3-methoxyanilin werden in 60 ml Toluol suspendiert und 3 Stunden unter Rückfluss erhitzt, wobei Ethanol entweicht. Beim Abkühlen auf Raumtemperatur bildet sich ein Niederschlag, der abfiltriert, mit Ethanol gewaschen und getrocknet wird. Ausbeute: 8,3 g (86 % d. Th.). Fp. 252-253 °C.

### b) Herstellung des Endproduktes

3,2 g 1,3-Dimethyl-5-(4-hydroxy-3-methoxyphenylcarbamoyl)barbitursäure werden in 25 ml Toluol und 15 ml Dimethylsulfoxid mit 1,3 g 85 % igem KOH versetzt. Das Gemisch wird mittels eines Wasserabscheiders bei Rückflusstemperatur entwässert. Nach dem Abdestillieren des Toluols werden 1,7 g 2-Chlor-benzthiazol zugegeben und die Badtemperatur langsam auf 40° bis 60 °C erhöht. Das Gemisch wird noch 10 Stunden bei dieser Temperatur gehalten, dann auf Raumtemperatur abgekühlt und mit verdünnter, wässriger HCI neutralisiert. Der gebildete Niederschlag wird abfiltriert, mit Wasser und Ethanol gewaschen und getrocknet. Fp. 210-211 °C.

### Beispiel 7 : Herstellung von

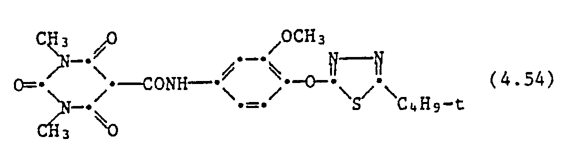

### 1,3-Dimethyl-5-[3-methoxy-4-(5-tert-butyl-1,3,4-thiadiazol-2-yloxy)-phenylcarbamoyl]barbitursäure

1,7 g 1,3-Dimethyl-2-barbitursäure und 2,7 g 3-Methoxy-4-(5-tert.-butyl-1,3,4-thiadiazol-2-yloxy)phenylisocyanat werden in 10 ml Xylol suspendiert und tropfenweise mit 0,2 g Triethylamin versetzt. Die Temperatur steigt dabei auf 45° bis 50 °C. Nach weiterer Zugabe von 10 ml Xylol wird das Gemisch 18 Stunden bei dieser Temperatur gerührt. Dann wird ca. 1/3 des Xylols abdestilliert und nach Abkühlen auf Raumtemperatur das Reaktionsgemisch mit 50 ml Hexan verdünnt, wobei ein kristalliner Niederschlag ausfällt. Dieser wird abfiltriert, mit Wasser, dann mit Isopropanol gewaschen und getrocknet. Ausbeute : 3,6 g (82 % d. Th.). Fp. 178-179 °C.

Analog zu den beschriebenen Arbeitsweisen sind auch die nachfolgend genannten Verbindungen der Formel I und Zwischenprodukte der Formel III' erhältlich.

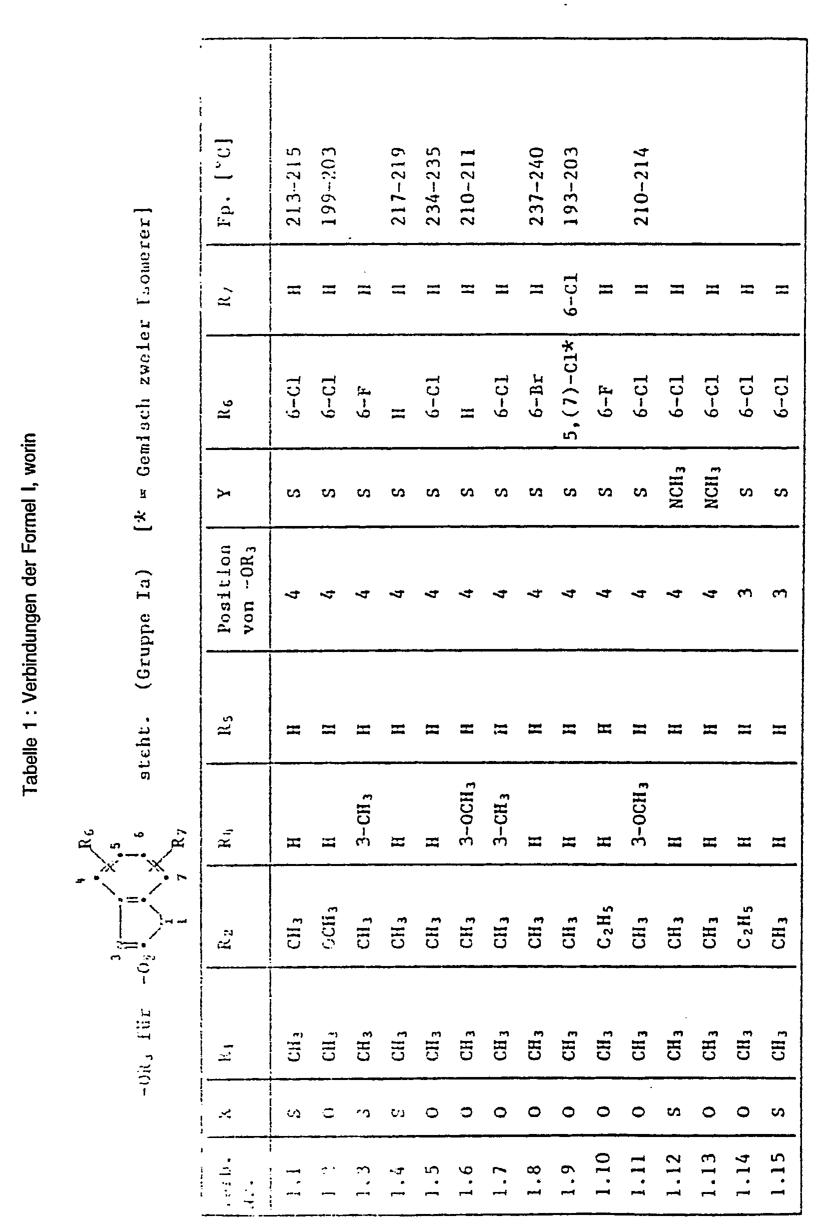

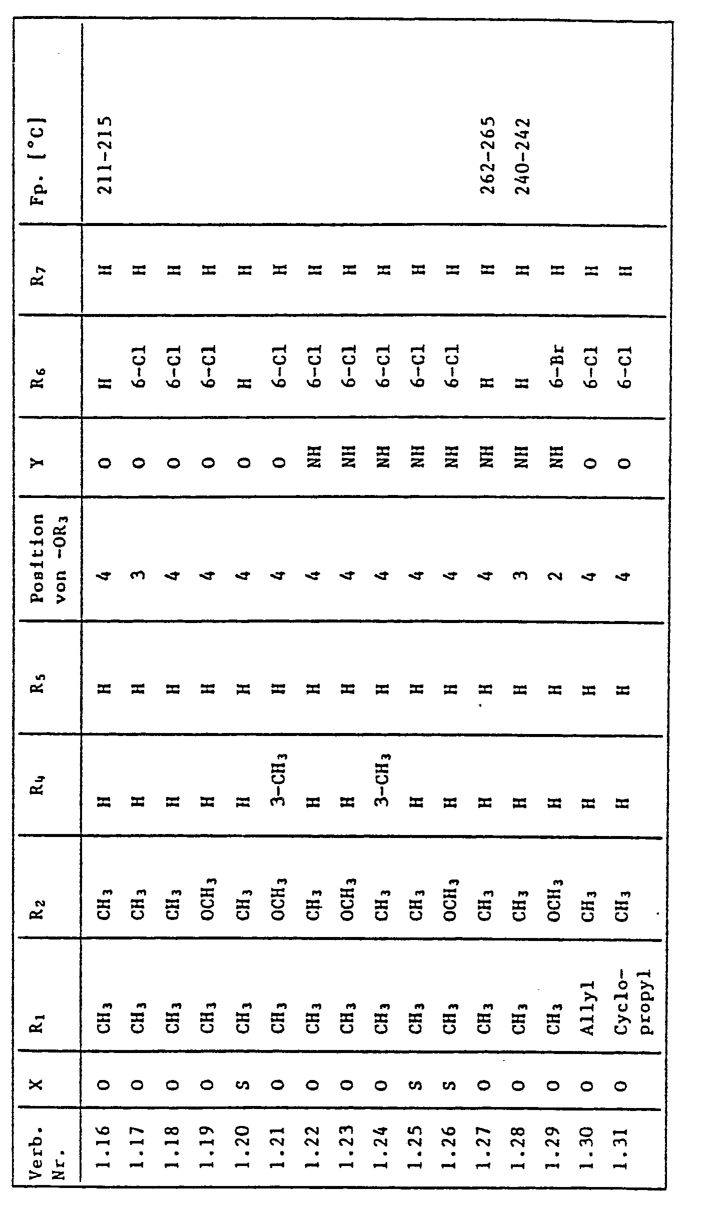

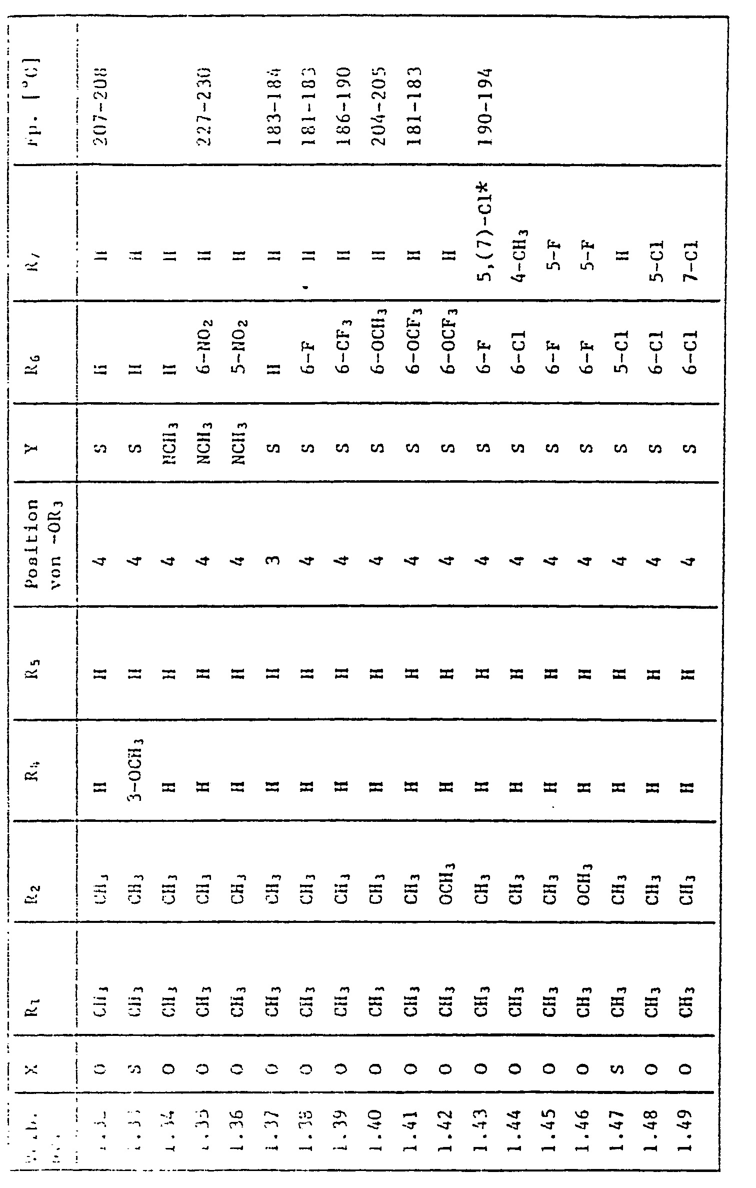

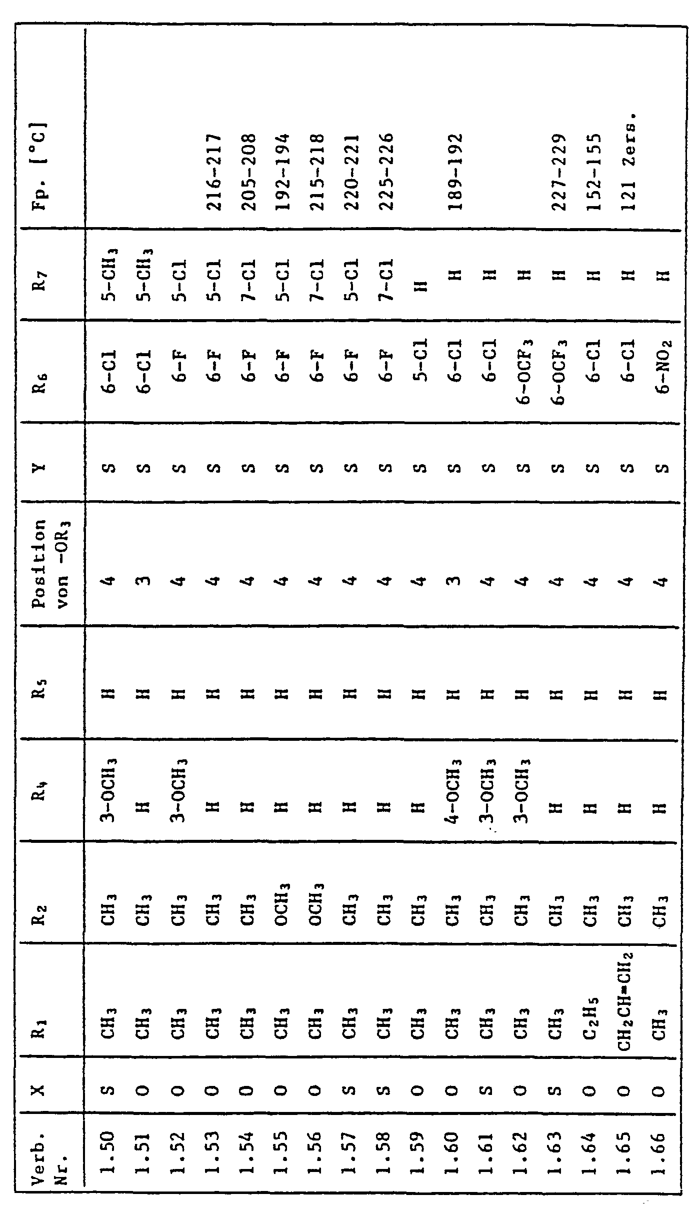

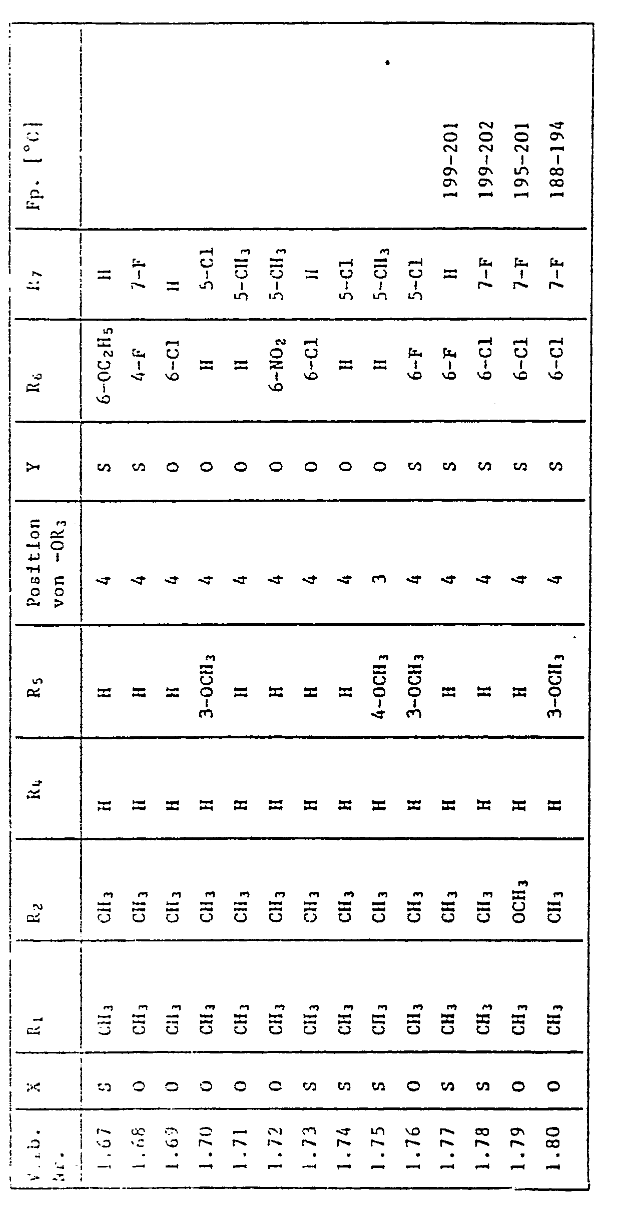

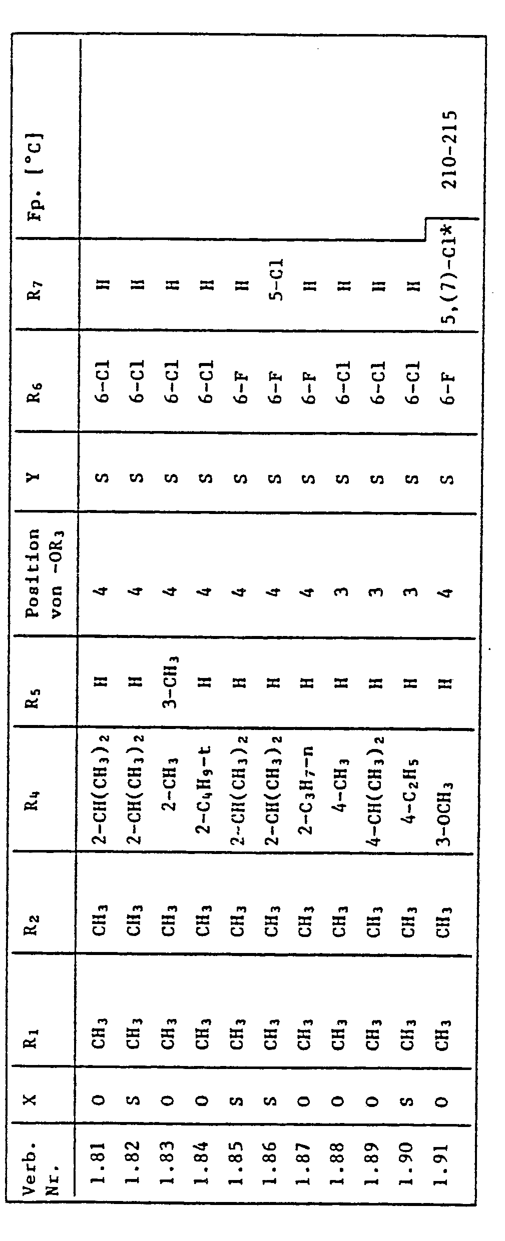

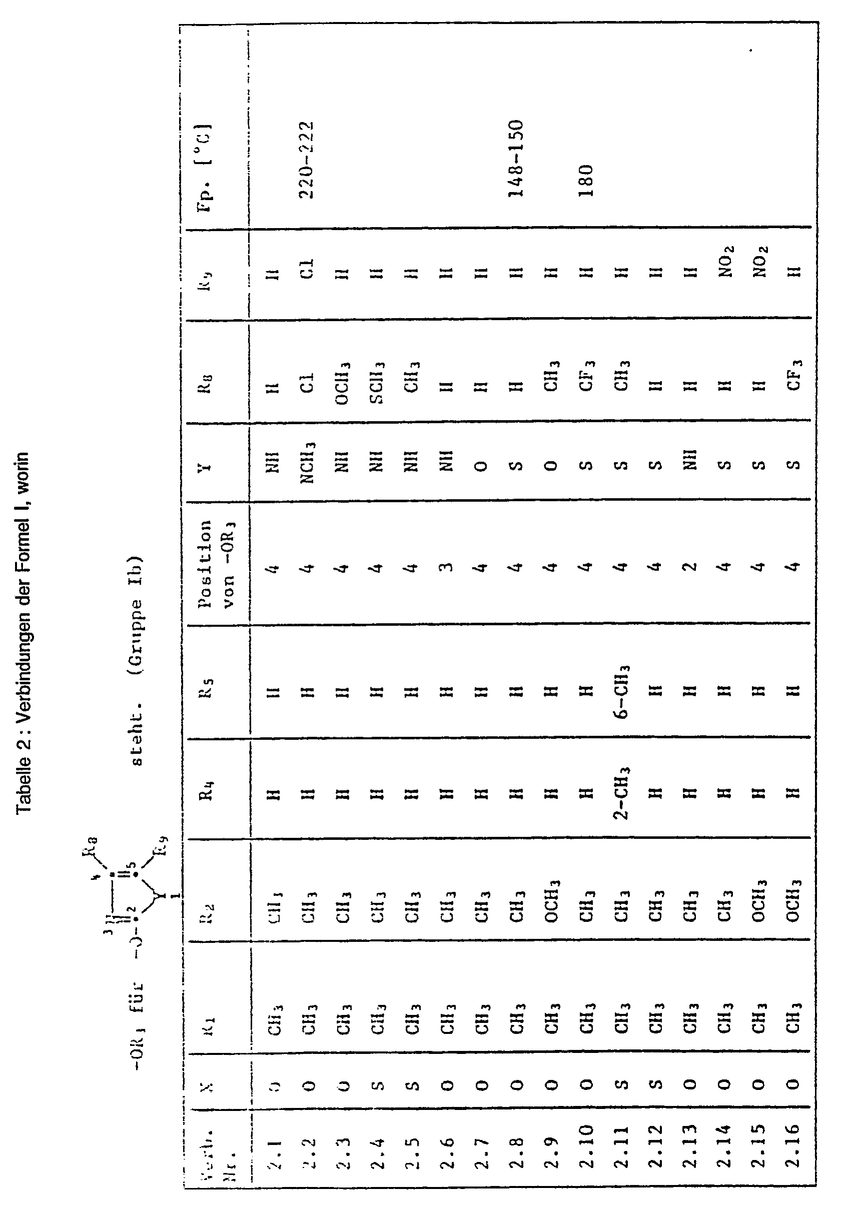

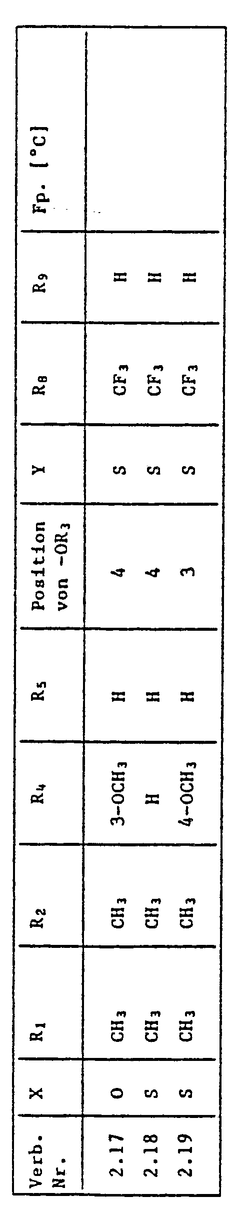

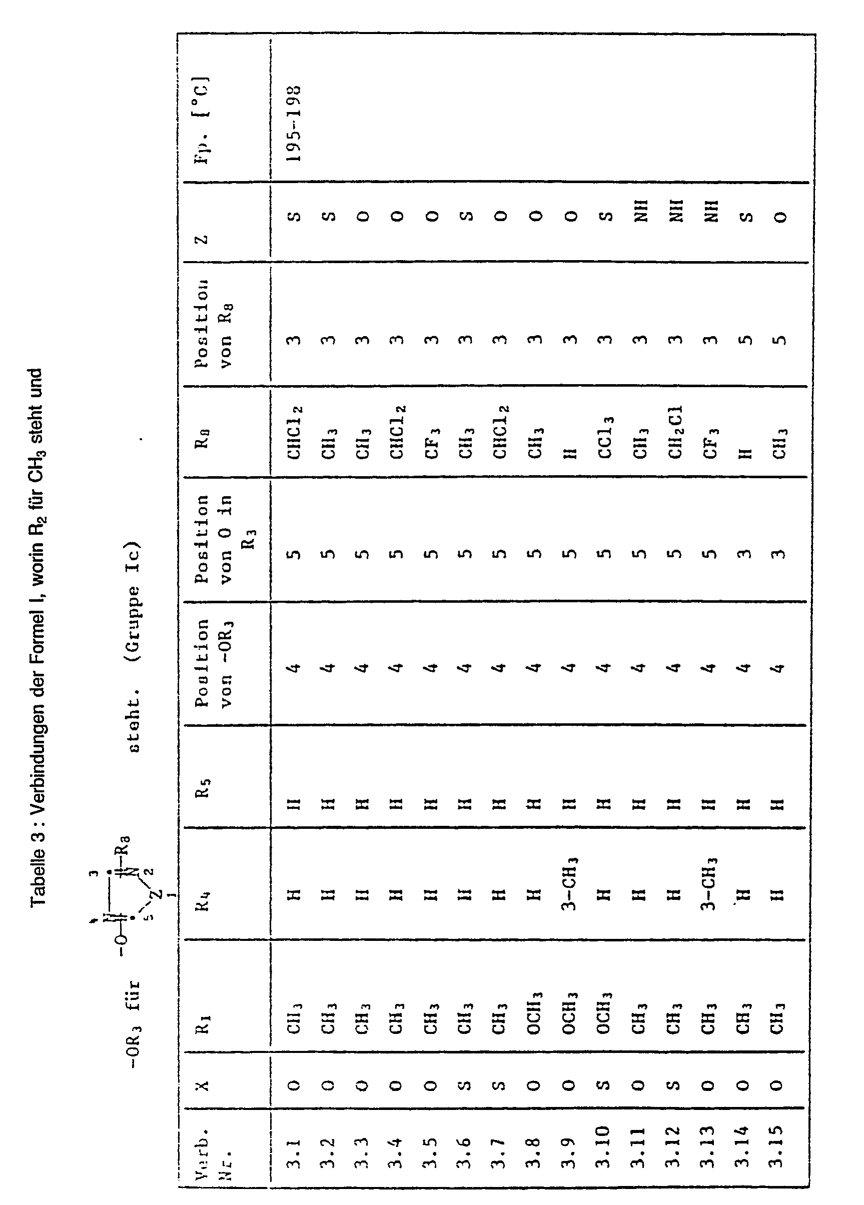

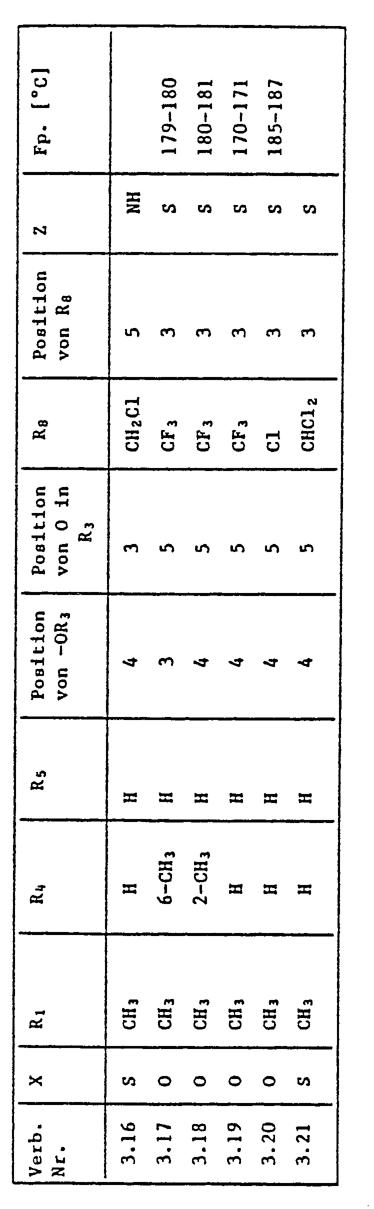

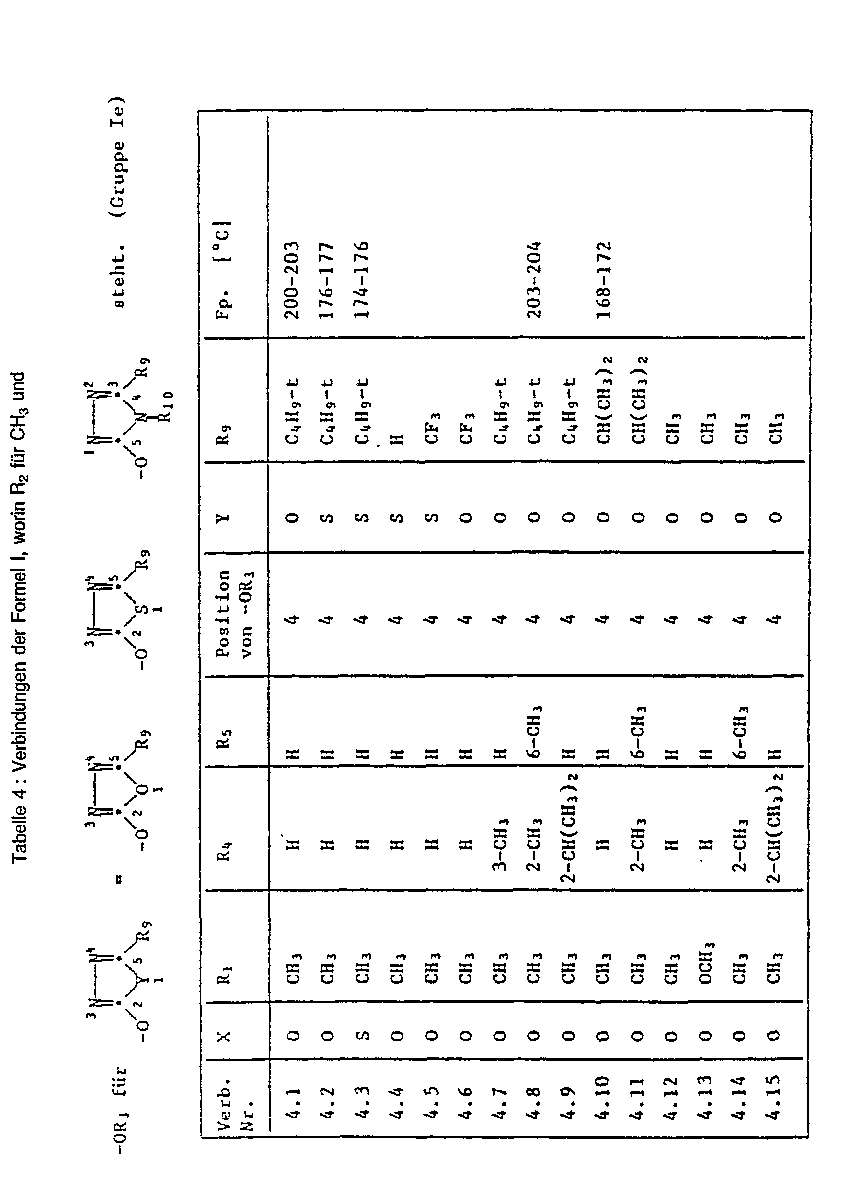

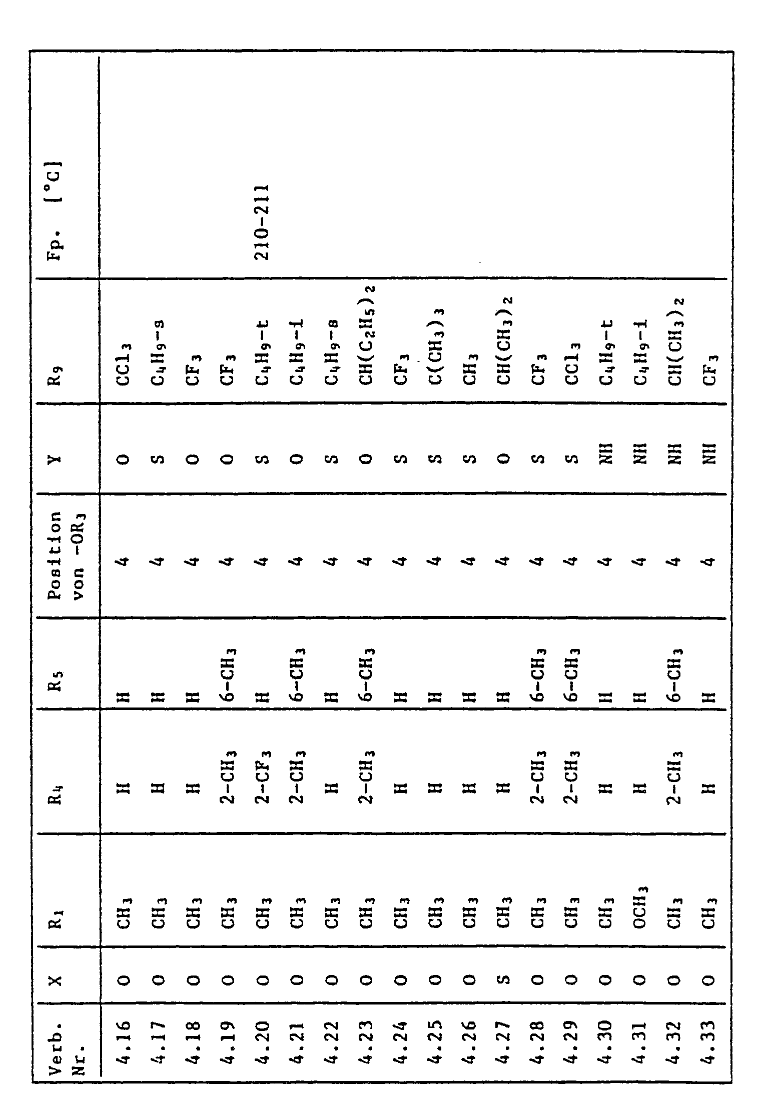

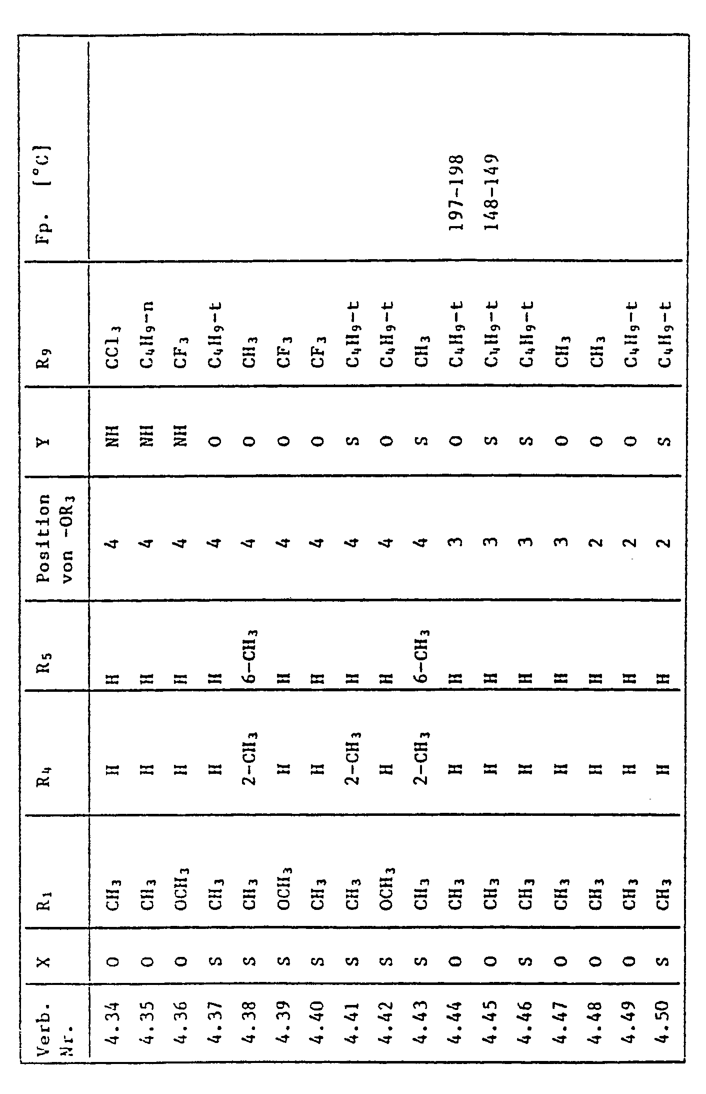

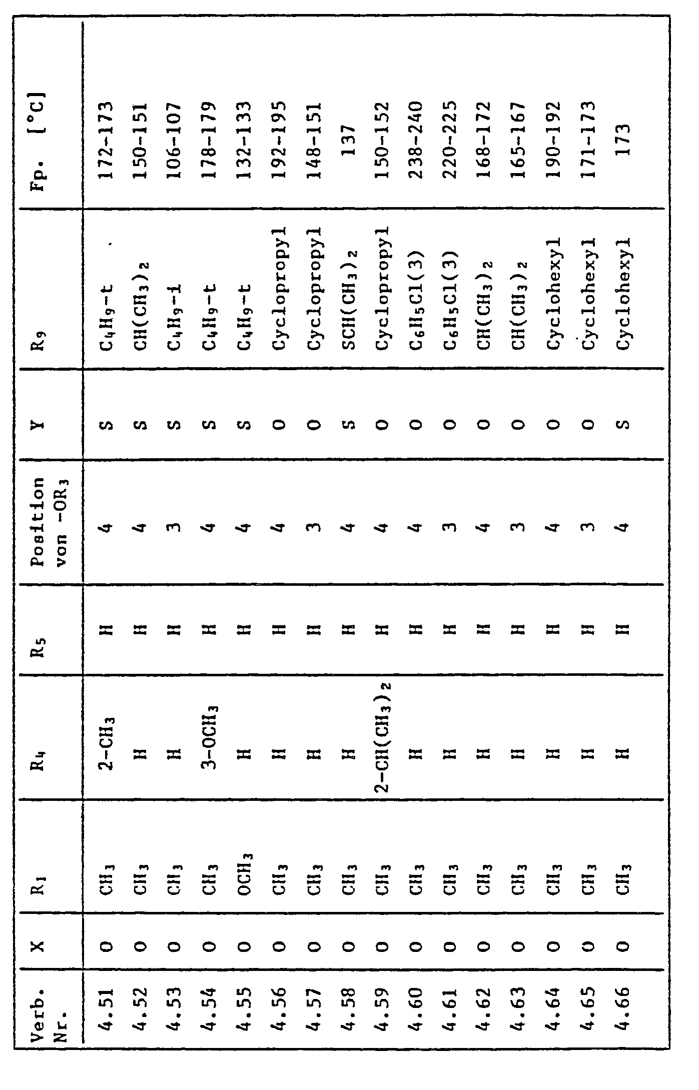

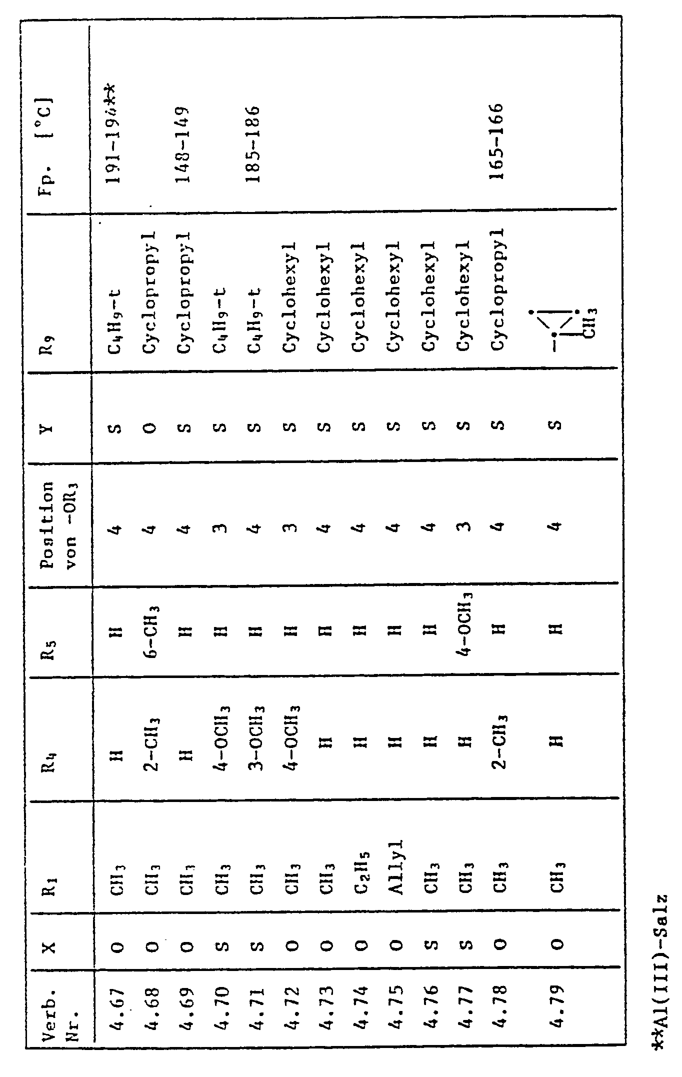

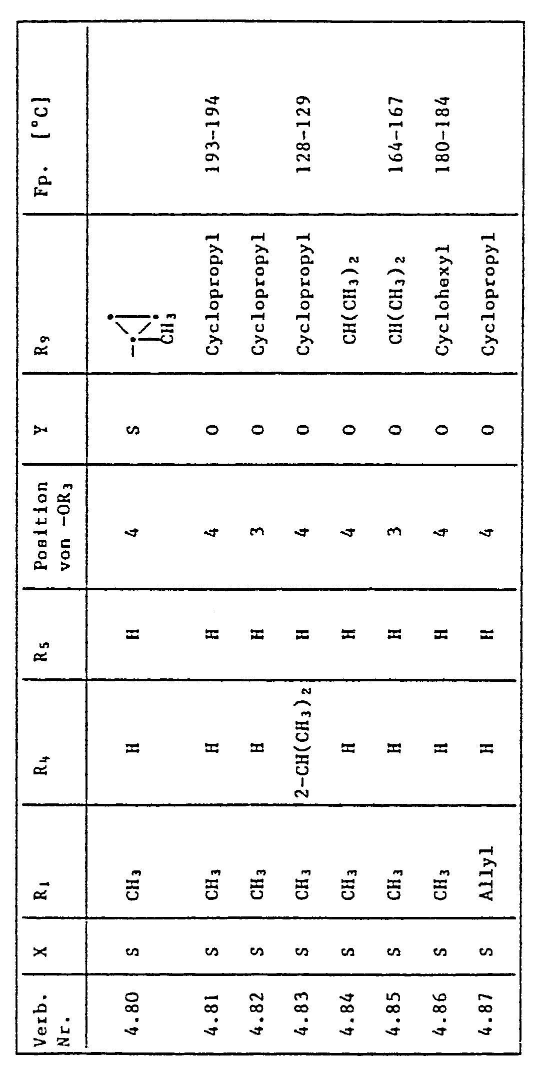

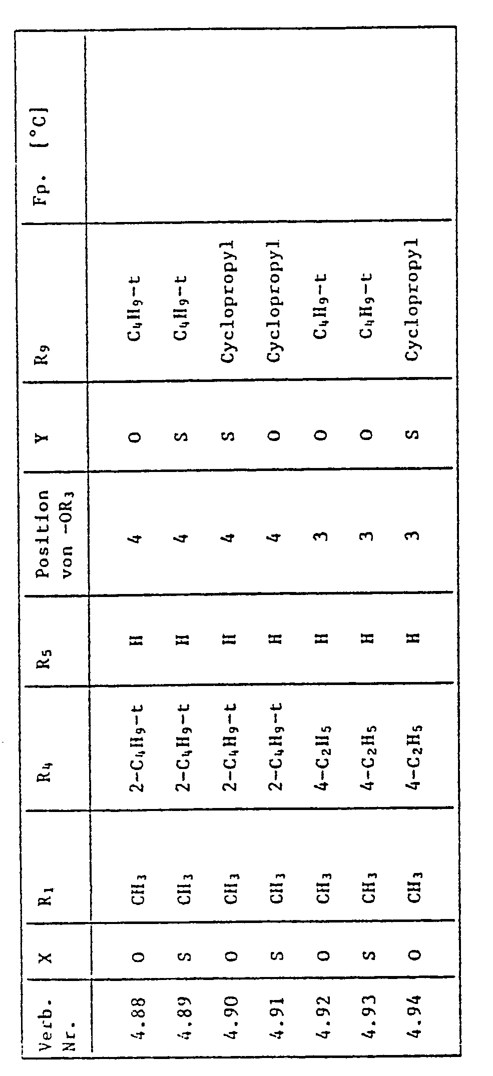

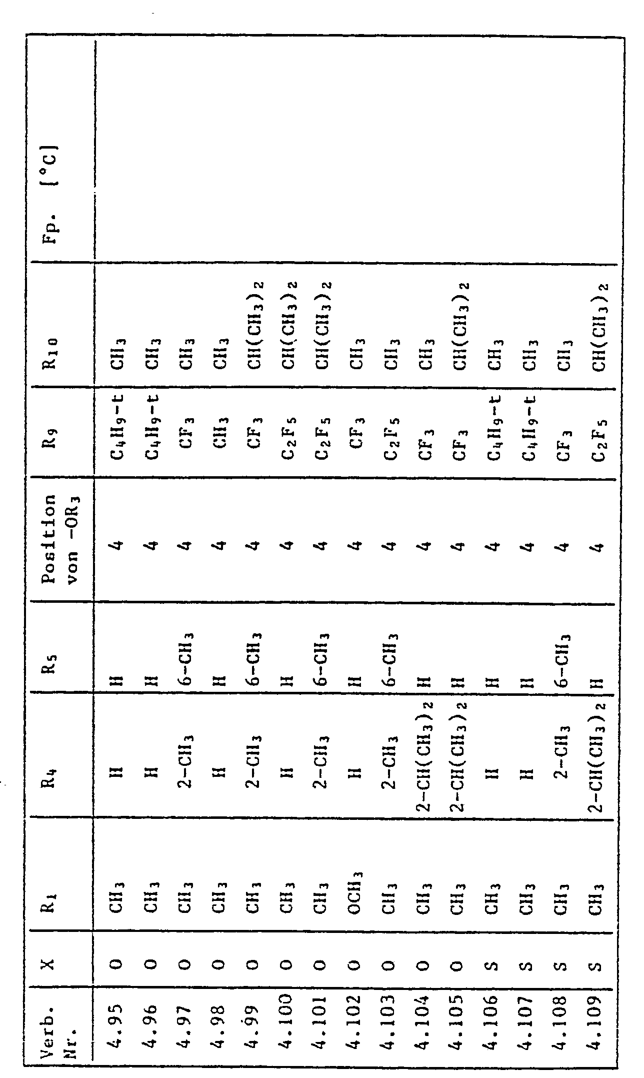

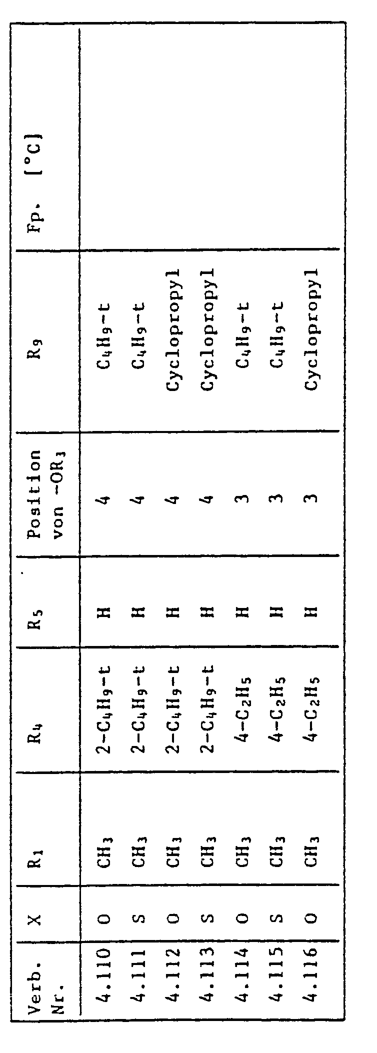

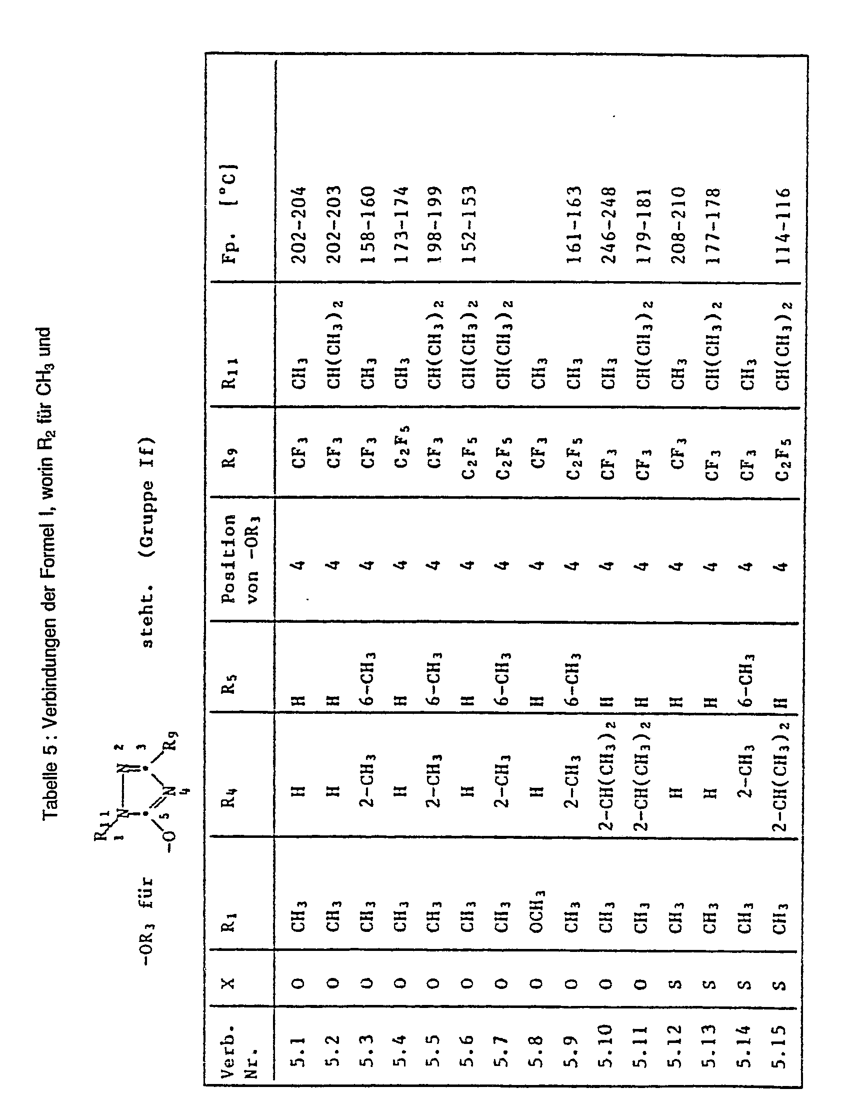

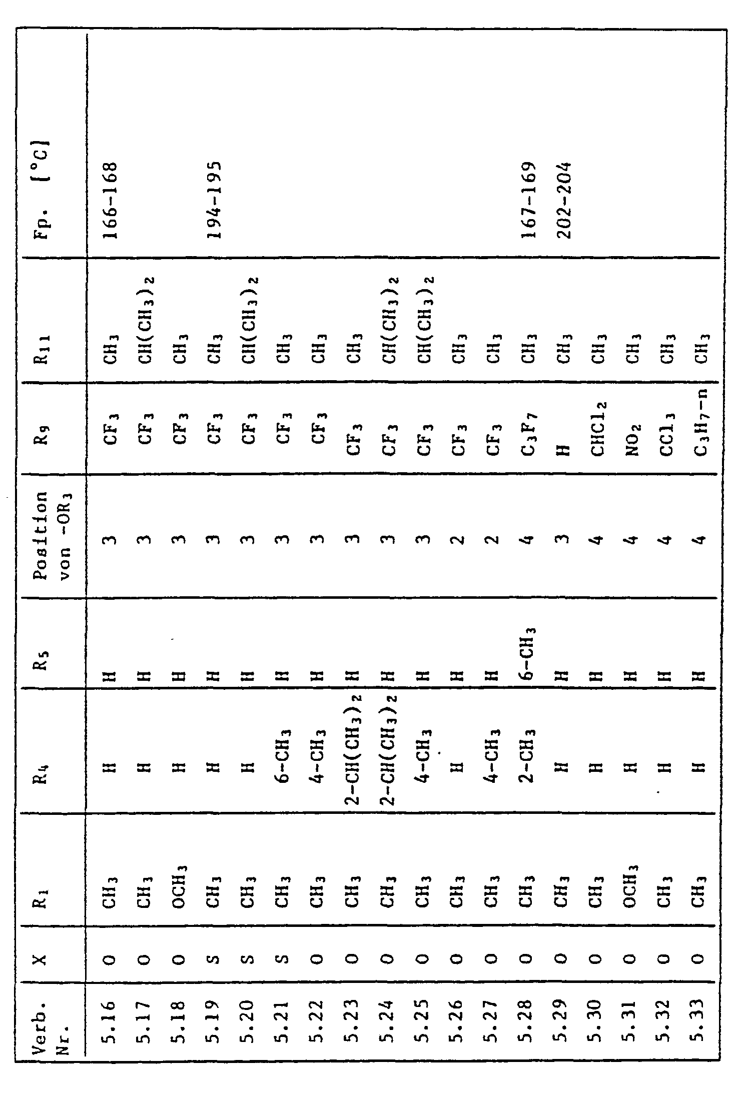

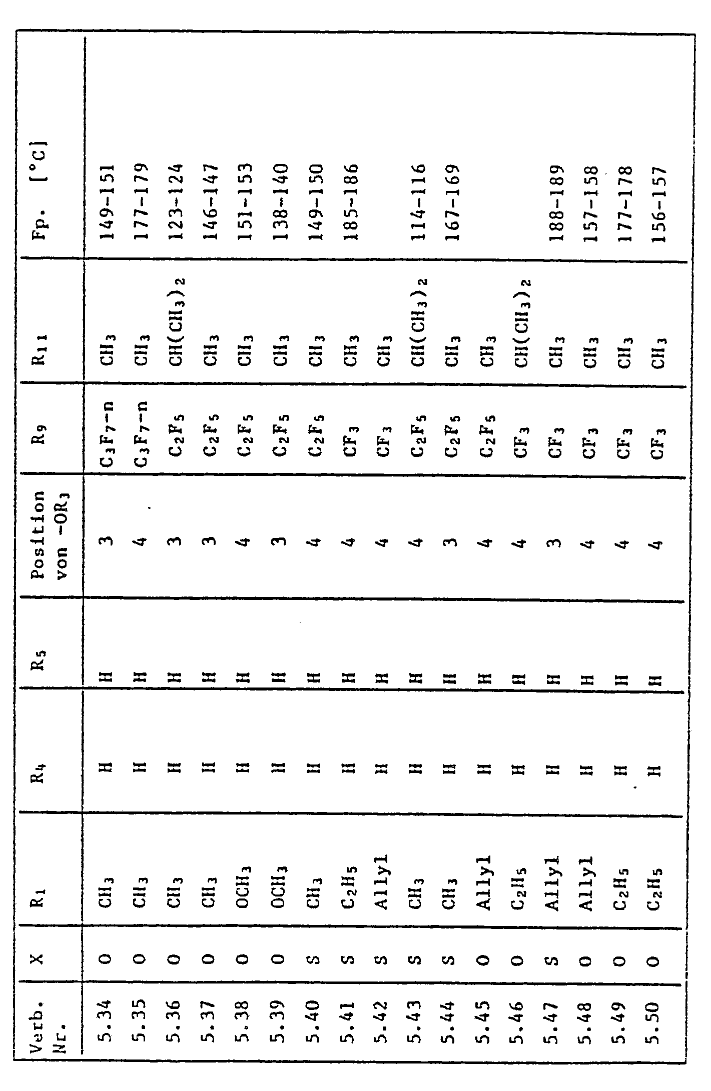

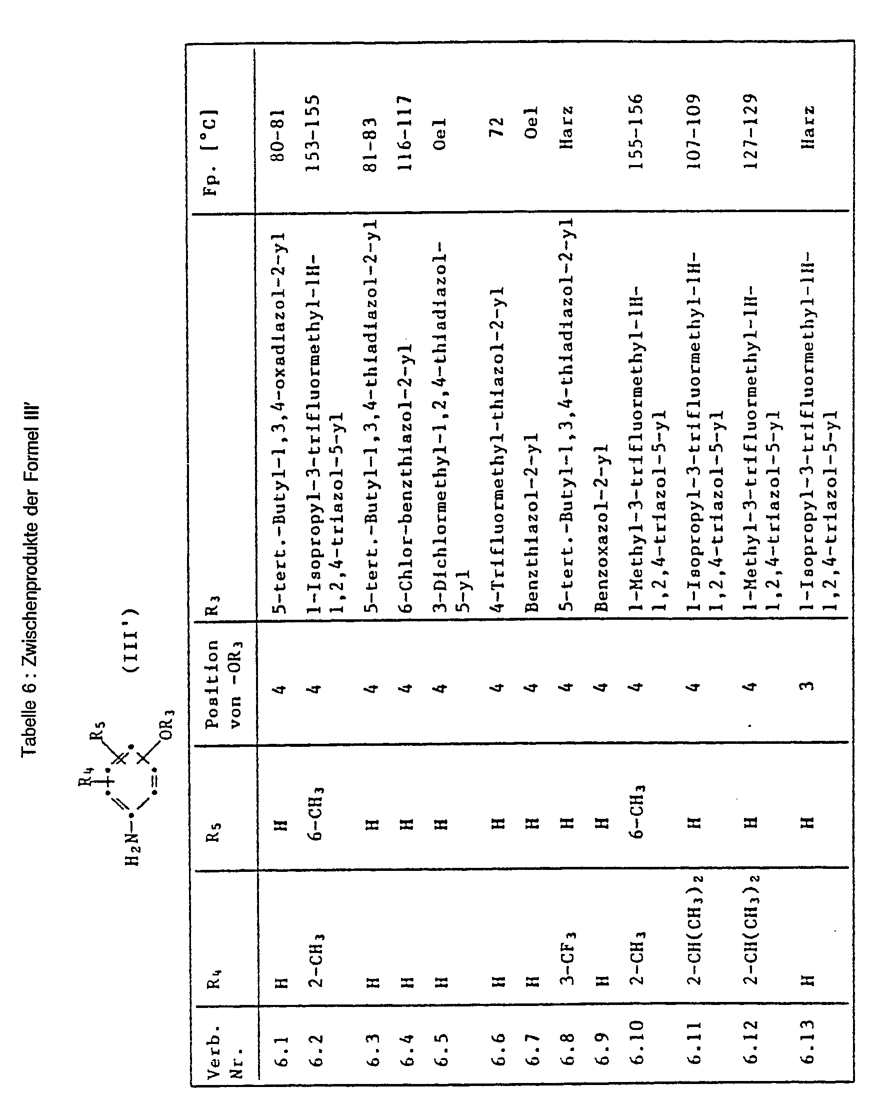

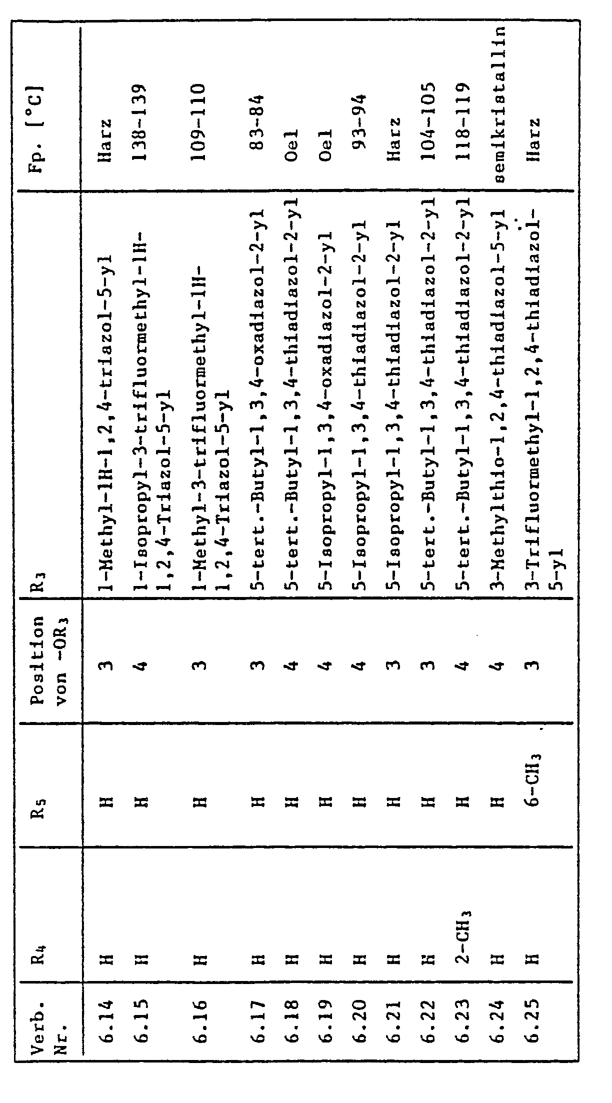

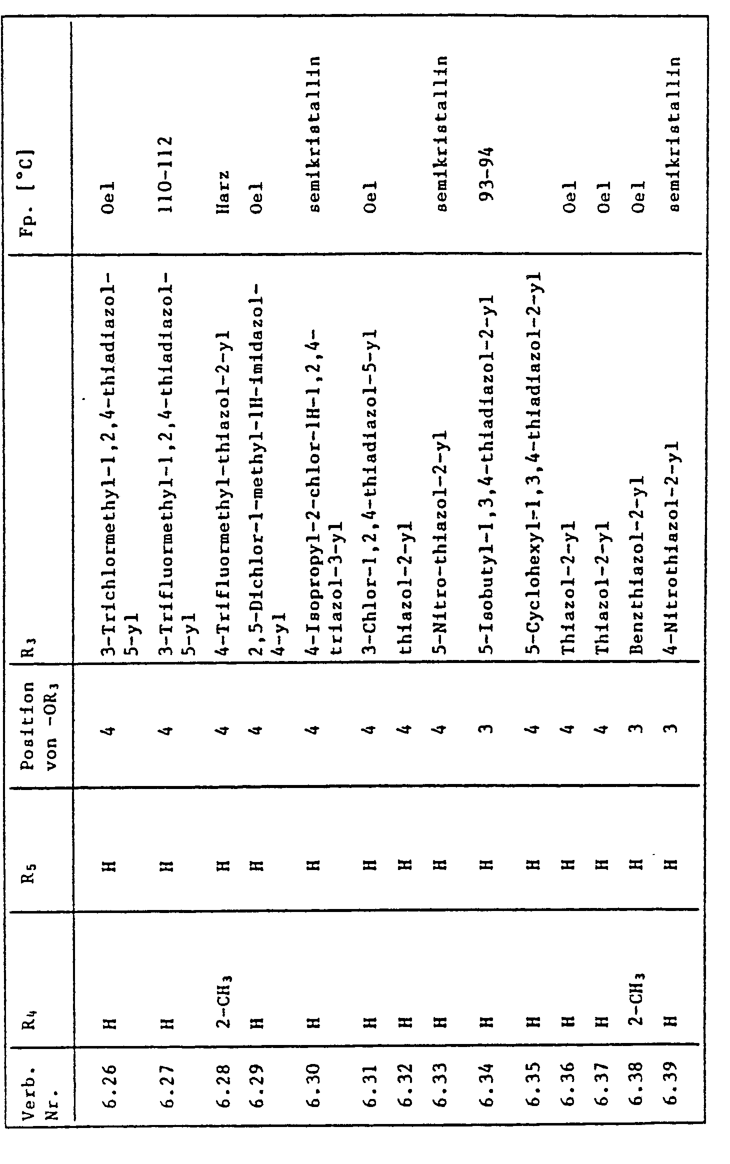

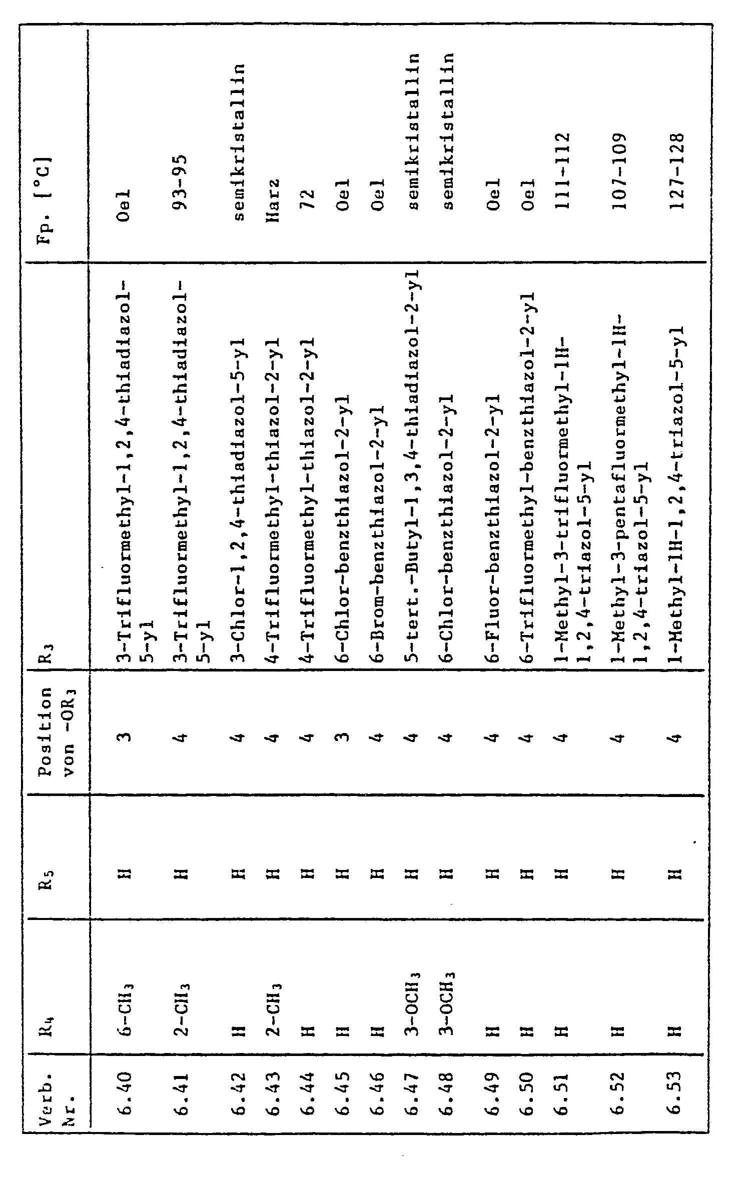

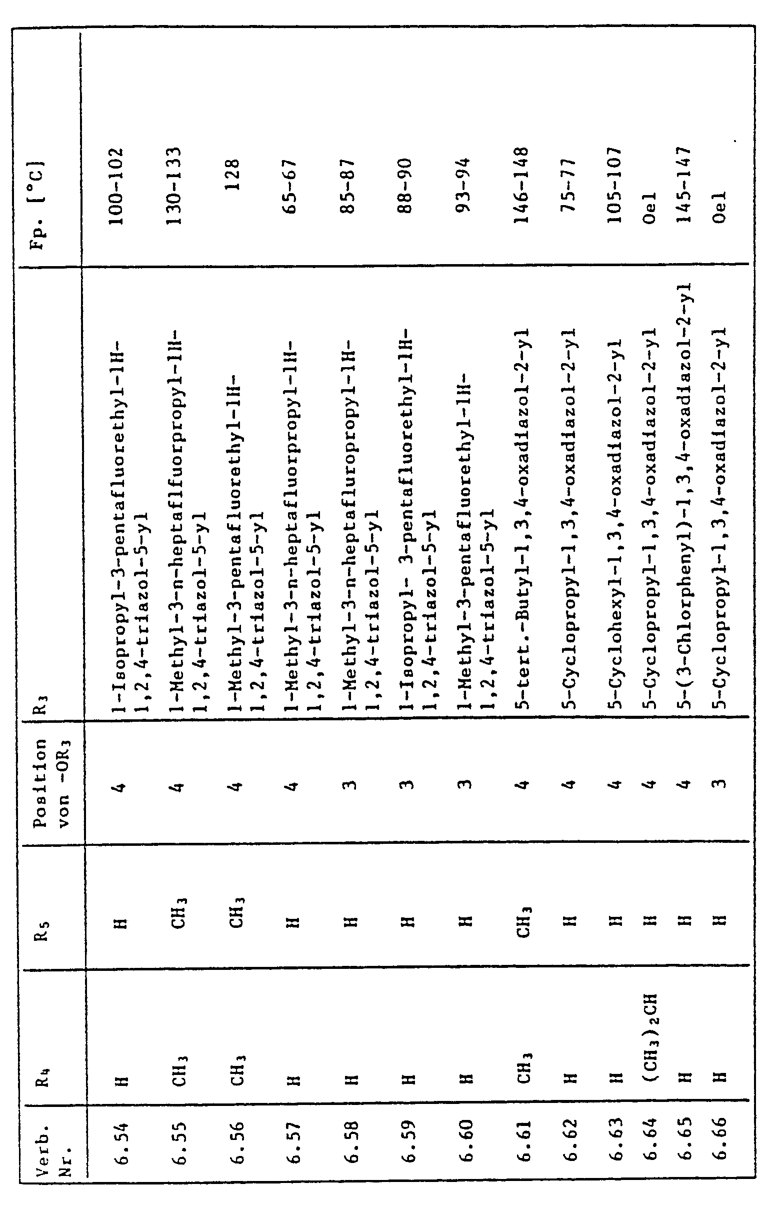

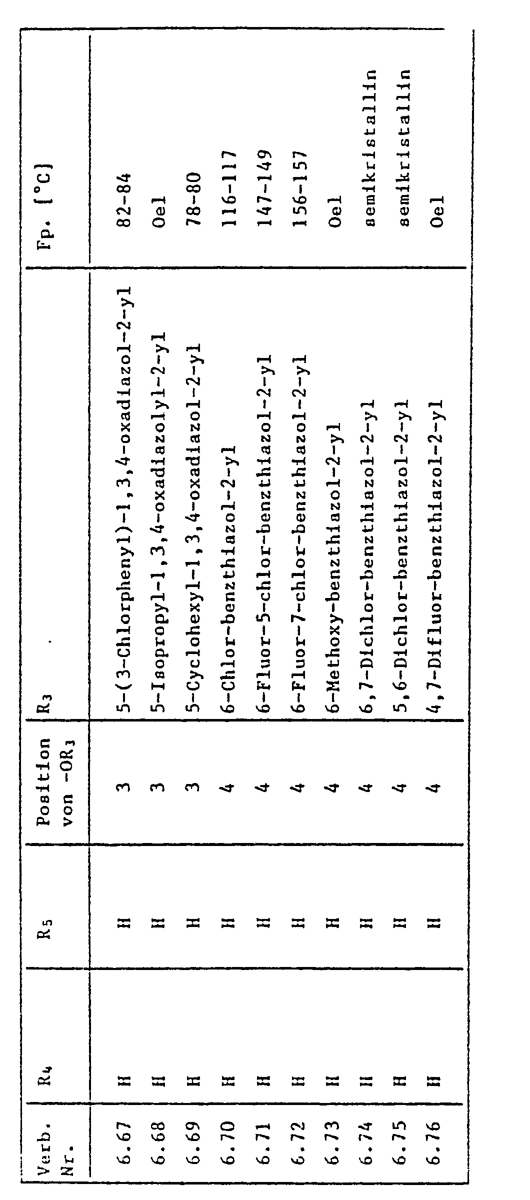

Formulierungsbeispiele (% = Gewichtsprozent)

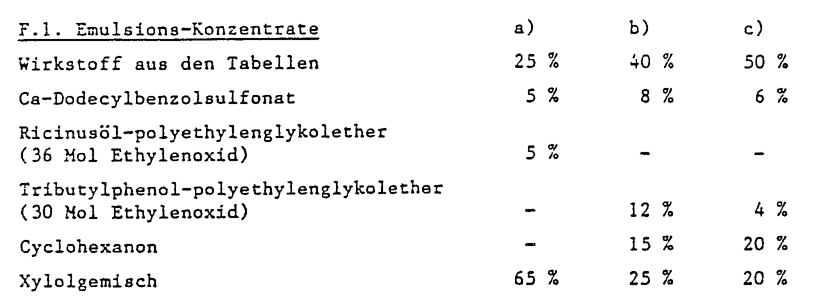

Aus solchen Konzentraten können durch Verdünnen mit Wasser Emulsionen jeder gewünchsten Konzentration hergestellt werden.

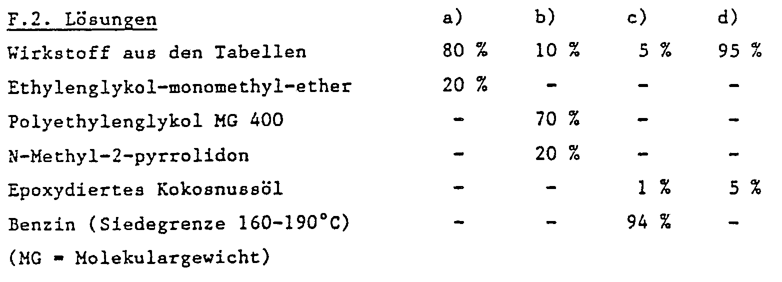

Die Lösungen sind zur Anwendung in Form kleinster Tropfen geeignet.

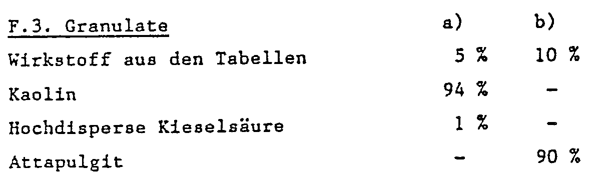

Der Wirkstoff wird in Methylenchlorid gelöst, auf den Träger aufgesprüht und das Lösungsmittel anschliessend im Vakuum eingedampft. Solche Granulate können dem Viehfutter beigemischt werden.

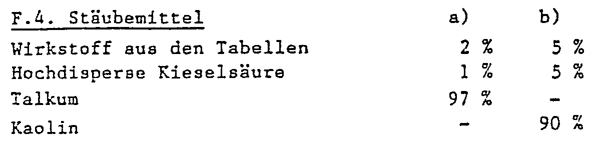

Durch inniges Vermischen auf Trägerstoffe mit dem Wirkstoff erhält man gebrauchsfertige Stäubemittel.

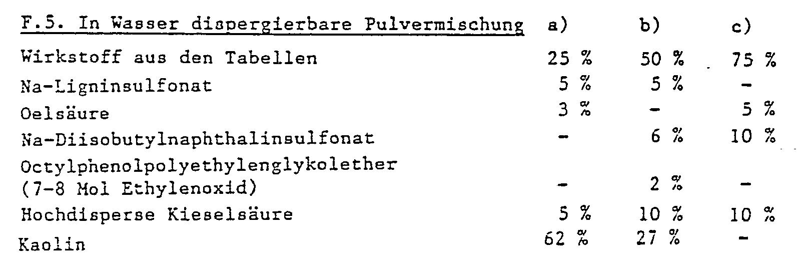

Der Wirkstoff wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

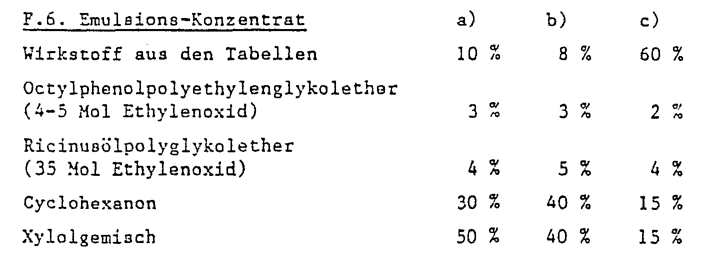

Aus diesem Konzentrat können durch Verdünnen mit Wasser Emulsionen jeder gewünschten Konzentration hergestellt werden.

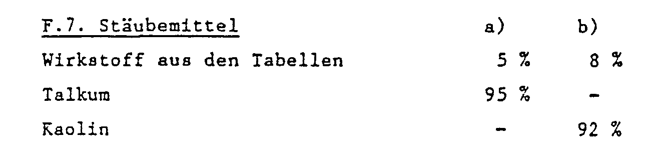

Man erhält anwendungsfertige Stäubemittel, indem der Wirkstoff mit dem Träger vermischt auf einer geeigneten Mühle vermahlen wird.

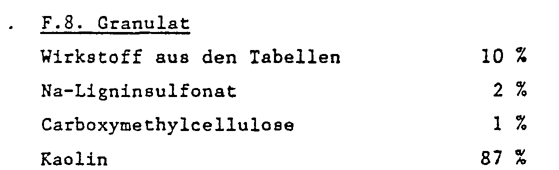

Der Wirkstoff wird mit den Zusatzstoffen vermischt, vermahlen und mit Wasser angefeuchtet. Dieses Gemisch wird extrudiert und anschliessend im Luftstrom getrocknet.

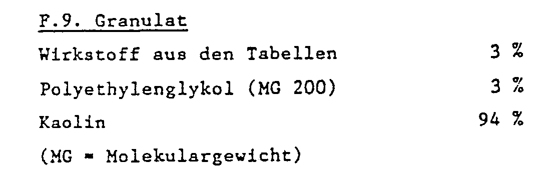

Der fein gemahlene Wirkstoff wird in einem Mischer auf das mit Polyethylenglykol angefeuchtete Kaolin gleichmässig aufgetragen. Auf diese Weise erhält man staubfreie Umhüllungs-Granulate. ₑ

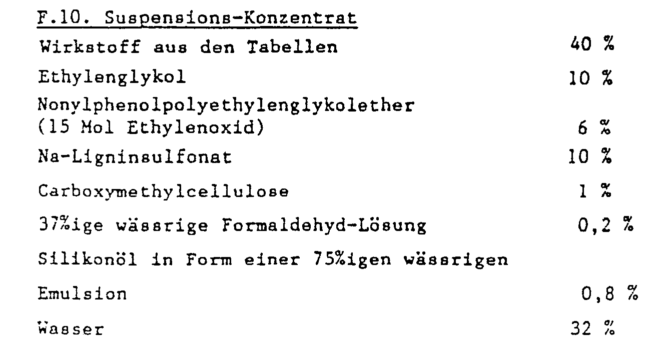

Der fein gemahlene Wirkstoff wird mit den Zusatzstoffen innig vermischt. Man erhält so ein Suspension-Konzentrat, aus welchem durch Verdünnen mit Wasser Suspensionen jeder gewünschten Konzentration hergestellt werden können.

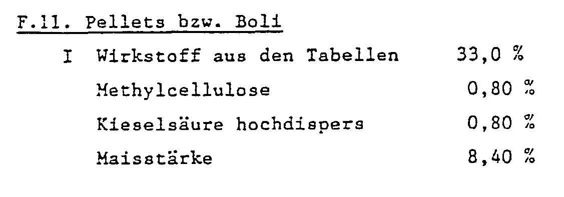

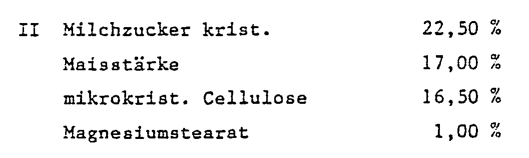

I Methylcellulose in Wasser einrühren und quellen lassen ; Kieselsäure in die Quellung einrühren und homogen suspendieren. Wirkstoff und Maisstärke mischen. In diese Mischung die wässrige Suspension einarbeiten und zu einem Teig kneten. Diese Masse durch ein Sieb 12 M granulieren und trocknen.
II Alle 4 Hilfsstoffe gut mischen.
III Phasen I und II mischen und zu Pellets oder Boli verpressen.

### Biologisches Beispiel

Die anthelmintische Wirksamkeit wird anhand folgender Versuche demonstriert:

B.1. Versuch an mit Nematoden wie Haemonchus contortus und Trichostrongylus colubriformis infizierten Schafen

Der Wirkstoff wird in Form einer Suspension mit einer Magensonde oder durch Pansen-Injektion Schafen verabreicht, die vorher mit Nematoden wie Haemonchus contortus und Trichostrongylus colubriformis künstlich infiziert wurden. Pro Versuch resp. pro Dosis werden 1 bis 3 Tiere verwendet. Jedes Schaf wird mit nur einer einzigen Dosis behandelt.

Eine erste Evaluierung erfolgt dadurch, dass die Anzahl der vor und nach der Behandlung im Kot der Schafe ausgeschiedenen Wurmeier verglichen werden.

Sieben bis zehn Tage nach der Behandlung werden die Schafe getötet und seziert. Die Auswertung erfolgt durch Auszählung der nach der Behandlung im Darm zurückbleibenden Würmer. Gleichzeitig und gleichartig infizierte aber unbehandelte Schafe dienen als Kontrolle resp. Vergleich.

Schafe, die mit einer Suspension eines Wirkstoffes aus der Tabelle 1 behandelt werden, zeigen im Vergleich zu unbehandelten, aber infizierten Vergleichsgruppen bei einer Dosis von 20 mg/kg Körpergewicht einen um mindestens 90 % reduzierten Nematodenbefall. Darüber hinaus zeigen die Verbindungen Nr. 1.1, 1.4, 1.5, 1.6, 1.8, 1.9,1.11,1.32,1.33,1.38,1.39,1.41,1.43,1.53,1.54,1.55, 1.57, 1.63, 1.78, 1.79, 1.80, 3.1, 4.1, 4.2, 4.3, 4.10, 4.20, 4.54, 4.55, 4.59, 4.66, 5.1, 5.2, 5.3, 5.4, 5.5, 5.6, 5.16, 5.28, 5.29 und 5.37, bei einer Dosis von 10 mg/kg eine mindestens 95 %ige Wirksamkeit.

## Patentansprüche (Patentansprüche für folgende(n) Vertragsstaat(en) : DE, FR, CH, Ll, IT, NL, BE, SE, LU)

1. Verbindungen der Formel I 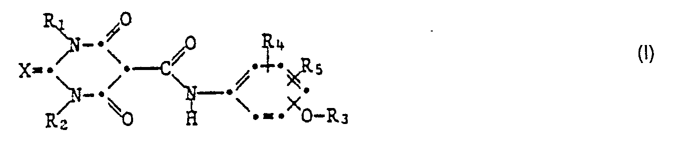 sowie deren tautomeren Formen und Salze, worin
X für Sauerstoff oder Schwefel steht;
R, C₁-C₆-Alkyl, C₁-C₆-Alkoxy, C₃-C₆-Cycloalkyl oder Allyl bedeutet ;
R₂ für C₁-C₆-Alkyl oder Allyl steht ;
R₃ für ein unsubstituiertes oder substituiertes, über Kohlenstoff gebundenes, heteroaromatisches Fünfringazol steht, ausgewählt aus der Reihe Benzimidazol, Benzoxazol, Benzthiazol, Imidazol, Oxazol, Thiazol, Oxadiazol, Thiadiazol und Triazol ;
und R₄ und R₅ unabhängig voneinander Wasserstoff, C₁-C₆-Alkyl, C₁-C₆-Haloalkyl, C₁-C₆-Alkoxy oder C₁-C₆-Haloalkoxy bedeuten.

2. Verbindungen der Formel I nach Anspruch 1, worin X für Sauerstoff oder Schwefel steht ; R₁ C₁-C₆-Alkyl, C₁-C₆-Alkoxy, C₃-C₆-Cycloalkyl oder Allyl bedeutet ; R₂ für C₁-C₆-Alkyl oder Allyl steht ; R₄ und R₅ unabhängig voneinander für Wasserstoff, C₁-C₆-Alkyl, C₁-C₆-Alkoxy oder C₁-C₆-Haloalkoxy stehen; R₃ eine der Gruppen ausgewählt aus der Reihe 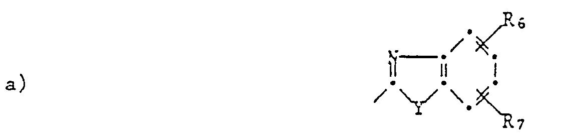 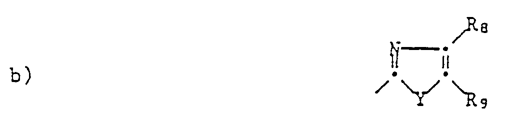 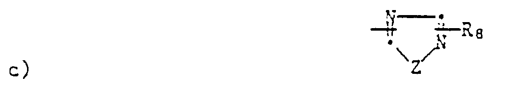 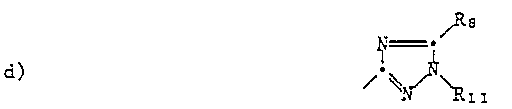 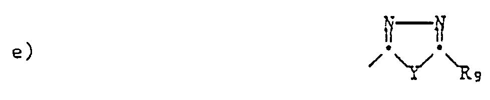 und 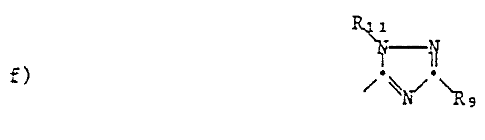 repräsentiert, wobei
Y für Sauerstoff, Schwefel oder NR₁₀ steht;
Z für Sauerstoff, Schwefel oder NH steht;
R₆ und R₇ unabhängig voneinander Wasserstoff, C₁-C₄-Alkyl, C₁-C₄-Haloalkyl, C₁-C₄-Alkoxy, C₁-C₄-Haloalkoxy, C₁-C₄-Alkylthio, Halogen, Nitro oder Cyano bedeuten ;
R₈ und Rg unabhängig voneinander für Wasserstoff, C₁-C₆-Alkyl, C₁-C₆-Haloalkyl, C₁-C₆-Alkoxy, C₁-C₆-Alkylthio, Halogen, Nitro oder unabhängig voneinander für unsubstituiertes oder duch Halogen oder C₁-C₃-Alkyl substituiertes C₃-C₇-Cycloalkyl stehen ;
R₁₀ Wasserstoff oder C₁-C₆-Alkyl bedeutet ; und
R₁₁ für C₁-C₆-Alkyl steht.

3. Verbindungen der Formel I nach Anspruch 2, worin X für Sauerstoff oder Schwefel steht; R₁ C₁-C₆-Alkyl, C₁-C₆-Alkoxy, C₃-C₆-Cycloalkyl oder Allyl bedeutet ; R₂ für C₁-C₆-Alkyl oder Allyl steht; R₄ und Rₛ unabhängig voneinander für Wasserstoff, C₁-C₆-Alkyl, C₁-C₆-Alkoxy oder C₁-C₆-Haloalkoxy stehen ; R₃ eine der Gruppen ausgewählt aus der Reihe 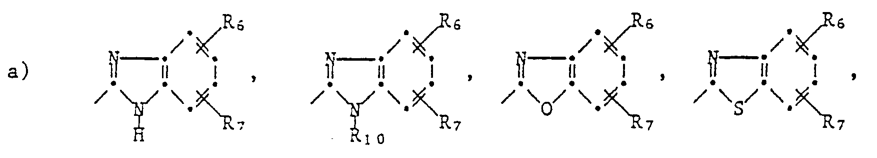 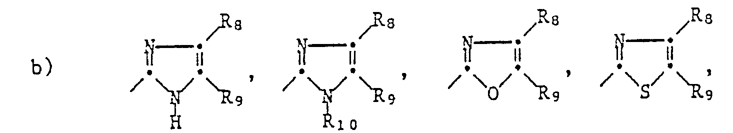 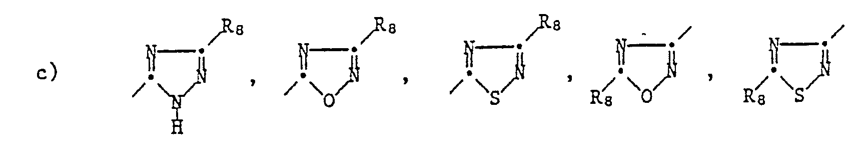 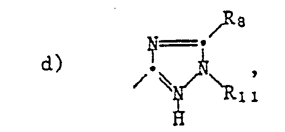 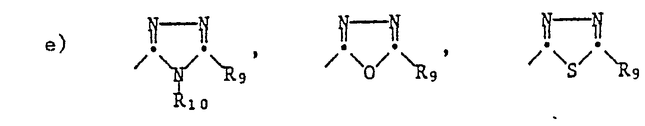 und 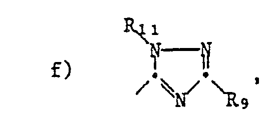 repräsentiert, wobei
R₆ und R₇ unabhängig voneinander Wasserstoff, C₁-C₄-Alkyl, C₁-C₄-Haloalkyl, C₁-C₄-Alkoxy, C₁-C₄-Haloalkoxy, C₁-C₄-Alkylthio, Halogen, Nitro oder Cyano bedeuten ;
R₈ und Rg unabhängig voneinander für Wasserstoff, C₁-C₆-Alkyl, C₁-C₆-Haloalkyl, C₁-C₆-Alkoxy, C₁-C₆-Alkylthio, Halogen oder Nitro stehen ;
R₁₀ Wasserstoff oder C₁-C₆-Alkyl bedeutet ; und
R₁₁ für C₁-C₆-Alkyl steht.

4. Verbindungen der Formel 1 nach Anspruch 2, worin X für Sauerstoff oder Schwefel steht; R₁ C₁-C₆-Alkyl, C₁-C₆-Alkoxy, C₃-C₆-Cycloalkyl oder Allyl bedeutet; R₂ für C₁-C₆-Alkyl oder Ally steht; R₄ und R₅ unabhängig voneinander für Wasserstoff, C₁-C₆-Alkyl, C₁-C₆-Alkoxy oder C₁-C₆-Haloalkoxy stehen ; R₃ für die Gruppe 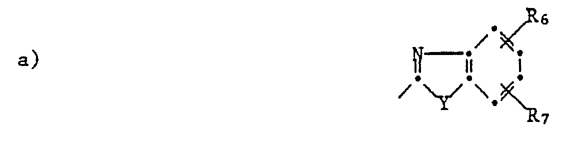 steht, wobei R₆ und R₇ unabhängig voneinander für Wasserstoff, C₁-C₄-Alkyl, C₁-C₄-Haloalkyl, C₁-C₄-Alkoxy, C₁-C₄-Haloalkoxy, C₁-C₄-Alkylthio, Halogen, Nitro oder Cyano stehen; und Y Sauerstoff, Schwefel oder NR₁₀ bedeutet; wobei R₁₀ für Wasserstoff oder C₁-C₆-Alkyl steht.

5. Verbindungen der Formel I nach Anspruch 2, worin X für Sauerstoff oder Schwefel steht; R₁ C₁-C₆-Alkyl, C₁-C₆-Alkoxy, C₃-C₆-Cycloalkyl oder Allyl bedeutet ; R₂ für C₁-C₆-Alkyl oder Allyl steht; R₄ und R₅ unabhängig voneinander für Wasserstoff, C₁-C₆-Alkyl, C₁-C₆-Alkoxy oder C₁-C₆-Haloalkoxy stehen; R₃ für die Gruppe 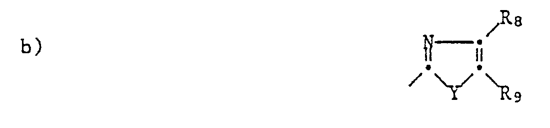 steht; wobei R₈ und Rg unabhängig voneinander für Wasserstoff, C₁-C₆-Alkyl, C₁-C₆-Haloalkyl, C₁-C₆-Alkoxy, C₁-C₆-Alkylthio, Halogen oder Nitro stehen; und Y Sauerstoff, Schwefel oder NR₁₀ bedeutet, wobei R₁₀ für Wasserstoff oder C₁-C₆-Alkyl steht.

6. Verbindungen der Formel I nach Anspruch 2, worin X für Sauerstoff oder Schwefel steht ; R₁ C₁-C₆-Alkyl, Cₗ-C₆-Alkoxy, C₃-C₆-Cycloalkyl oder Allyl bedeutet ; R₂ für C₁-C₆-Alkyl oder Allyl steht; R₄ und R₅ unabhängig voneinander für Wasserstoff, C₁-C₆-Alkyl, C₁-C₆-Alkoxy oder C₁-C₆-Haioalkoxy stehen ; R₃ für die Gruppe 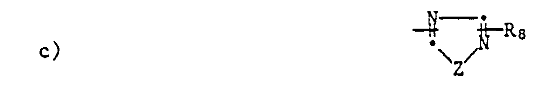 steht; worin R₈ für Wasserstoff, C₁-C₆-Alkyl, C₁-C₆-Haloalkyl, C₁-C₆-Alkoxy, C₁-C₆-Alkylthio, Halogen oder Nitro steht ; und Z Sauerstoff, Schwefel oder NR repräsentiert.

7. Verbindungen der Formel I nach Anspruch 2, worin X für Sauerstoff oder Schwefel steht; R₁ C₁-C₆-Alkyl, C₁-C₆-Alkoxy, C₃-Cₛ-Cycloalkyl oder Allyl bedeutet; R₂ für C₁-C₆-Alkyl oder Allyl steht; R₄ und R₅ unabhängig voneinander für Wasserstoff, C₁-C₆-Alkyl, C₁-C₆-Alkoxy oder C₁-C₆-Haloalkoxy stehen ; R₃ für die Gruppe 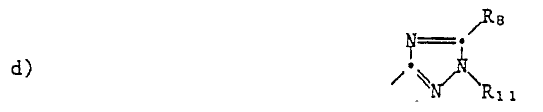 steht; worin R₈ für Wasserstoff, C₁-C₆-Alkyl, C₁-C₆-Haloalkyl, C₁-C₆-Alkoxy, C₁-C₆-Alkylthio, Halogen oder Nitro steht; und R₁₁ für C₁-C₆-Alkyl steht.

8. Verbindungen der Formel I nach Anspruch 2, worin X für Sauerstoff oder Schwefel steht; R₁ C₁-C₆-Alkyl, C₁-C₆-Alkoxy, C₃-C₆-Cycloalkyl oder Allyl bedeutet; R₂ für C₁-C₆-Alkyl oder Allyl steht; R₄ und R₅ unabhängig voneinander für Wasserstoff, C₁-C₆-Alkyl, C₁-C₆-Alkoxy oder C₁-C₆-Haloalkoxy stehen ; R₃ für die Gruppe 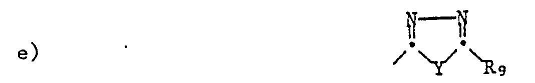 steht; worin Rg Wasserstoff, C₁-C₆-Alkyl, C₁-C₆-Haloalkyl, C₁-C₆-Alkoxy, C₁-C₆-Alkylthio, Halogen oder Nitro bedeutet; Y für Sauerstoff, Schwefel oder NR₁₀ steht; und R₁₀ Wasserstoff oder C₁-C₆-Alkyl bedeutet.

9. Verbindungen der Formel I nach Anspruch 1, dadurch gekennzeichnet, dass X für Sauerstoff oder Schwefel steht; R₁ C₁-C₆-Alkyl, C₁-C₆-Alkoxy, C₃-C₆-Cycloalkyl oder Allyl bedeutet; R₂ für C₁-C₆-Alkyl oder Allyl steht ; R₄ und R₅ unabhängig voneinander für Wasserstoff, C₁-C₆-Alkyl, C₁-C₆-Alkoxy oder C₁-C₆-Haloalkoxy stehen ; R₃ eine der Gruppen ausgewählt aus der Reihe 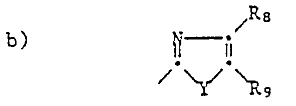 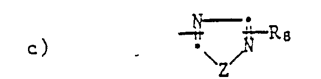 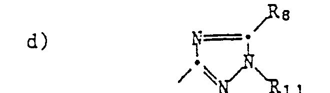 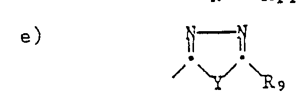 und 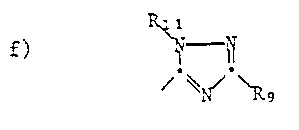 repräsentiert; R₈ und Rg unabhängig voneinander für Wasserstoff, C₁-C₆-Alkyl, C₁-C₆-Haloalkyl, C₁-C₆-Alkoxy, C₁-C₆-Alkylthio, Halogen, Nitro oder unabhängig voneinander für unsubstituiertes oder durch Halogen oder C₁-C₃-Alkyl substituiertes C₃-C₇-Cycloalkyl stehen ; Y für Sauerstoff, Schwefel oder NR,ₒ steht; Z für Sauerstoff, Schwefel oder NH steht; R₁₀ Wasserstoff oder C₁-C₆-Alkyl bedeutet; und R₁₁ für C₁-C₆-Alkyl steht.

10. Verbindungen der Formel I nach Anspruch 2, worin X für Sauerstoff oder Schwefel steht ; R₁ C₁-C₆-Alkyl, C₁-C₆-Alkoxy, C₃-C₆-Cycloalkyl oder Allyl bedeutet ; R₂ für C₁-C₆-Alkyl oder Allyl steht; R₄ und R₅ unabhängig voneinander für Wasserstoff, C₁-C₆-Alkyl, C₁-C₆-Alkoxy oder C₁-C₆-Haloalkoxy stehen ; R₃ für die Gruppe 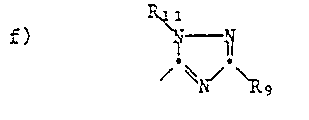 steht; worin Rg Wasserstoff, C₁-C₆-Alkyl, C₁-C₆-Haloalkyl, C₁-C₆-Alkoxy, C₁-C₆-Alkyithio, Halogen oder Nitro bedeutet ; und R₁₁ für C₁-C₆-Alkyl steht.

11. Verbindungen der Formel I nach Anspruch 2, worin R₃ für die Gruppe 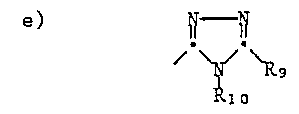 steht, worin Rg Wasserstoff, C₁-C₆-Alkyl, C₁-C₆-Haloalkyl, C₁-C₆-Alkoxy, C₁-C₆-Alkylthio, Halogen oder Nitro bedeutet ; und R₁₀ für Wasserstoff oder C₁-C₆-Alkyl steht.

12. Verbindungen der Formel I nach einem der Ansprüche 1 bis 11, worin X für Sauerstoff oder Schwefel steht, R₁ für Methyl oder Methoxy steht; R₂ für Methyl steht; R₄ und R₅ unabhängig voneinander für Wasserstoff, Methyl, CF₃, OCH₃, OCHF₂ oder OCF₃ stehen; und die übrigen Substituenten wie im rückbezogenen Anspruch definiert sind.

13. Verbindungen der Formel I nach Anspruch 4, worin R₆ und R₇ unabhängig voneinander für Wasserstoff, Methyl, CF₃, Methoxy, Halomethoxy, Fluor, Chlor oder Brom stehen und die übrigen Substituenten wie in einem der Ansprüche 1 bis 12 definiert sind.

14. Verbindungen der Formel I nach einem der Ansprüche 5 bis 8 und 10 bis 11, worin R₈ und Rg unabhängig voneinander für Wasserstoff, Methyl, Ethyl, C₃-CᵣCycloalkyl, CF₃, C₂F₅, C₃F₇, CCI₃, CHCI₂, CH₂CI, SCH₃ oder Halogen stehen ; R₁₀ Wasserstoff oder Methyl bedeutet ; R₁₁ für C₁-C₄-Alkyl steht ; und die übrigen Substituenten wie in den Ansprüchen 5 bis 8 definiert sind.

15. Eine Verbindung der Formel nach einem der Ansprüche 1 bis 3, ausgewählt aus der Reihe
1,3-Dimethyl-5-[4-(6-brom-benzthiazol-2-yloxy)phenylcarbamoyl]-barbitursäure ;
1,3-Dimethy(-5-[4-(6-chlor benzthiazol-2-yloxy)phenylcarbamoyl]-barbitursäure ;
1,3-Dimethyl-5-[4-(6-fluor-(benzthiazol-2-yloxy)phenylcarbamoyl]-barbitursäure ;
1,3-Dimethyl-5-[4-(3-dichlormethyl-1,2,4-thiadiazol-5-yloxy)phenylcarbamoyl]barbitursäure ;
1,3-Dimethyl-5-[4-(5-tert-butyl-1,3,4-oxadiazol-2-yloxy)phenylcarbamoyl]barbitursäure ;
1,3-Dimefhyl-5-[4-(5-tert-butyl-1,3,4-thiadiazol-2-yloxy)phenylcarbamoyl]barbitursäure ;
1-Methyl-3-methoxy-5-[4-(5-tert-butyl-1,3,4-thiadiazol-2 yloxy)-phenylcarbamoyl]barbitursäure ;
1,3-Dimethyl-5-[4-(5-isopropyl-1,3,4-oxadiazol-2-yloxy)phenylcarbamoyl]barbitursäure ;
1,3-Dimethyl-5-[4-(1-isopropyl-3-trifluormethyl-1H-1,2,4-triazol-5-yloxy)phenylcarbamoyl]barbitursäure ;
1,3-Dimethyl-5-[2,6-dimethy)-4-(1-methy)-3-heptaf)uorpropy)-1H-1,2,4-triazo)-5-y)oxy)phenylcarbamoyl]barbitursäure ;
1,3-Dimethyl-5-[4-(1-isopropyl-3-pentafluorethyl-1H-1,2,4-triazol-5-yloxy)phenylcarbamoyl]barbitursäure ;
1,3-Dimethyl-5-[4-(1-isopropyl-3-trifluormethyl-1H-1,2,4-triazol-5-yloxy)-2,6-dimethyl-phenylcarbamoyl]barbitursäure ;
1,3-Dimethyl-5-[3-(1-methyl-3-trifluormethyl-1 H-1,2,4-triazol-5-yloxy)phenylcarbamoyl]barbitursäure ;
1,3-Dimethyl-5-[4-(6-trifluormethyl-benzthiazol-2-yloxy)phenylcarbamoyl]barbitursäure ;
1,3-Dimethyl-5-[3-methoxy-4-(5-tert-butyl-1,3,4-thiadiazol-2-yloxy)phenylcarbamoyl]barbitursäure ;
1,3-Dimethyl-5-[3-methoxy-4-(6-chlor-benzthiazol-2-yloxy)phenylcarbamoyl]barbitursäure ;
1,3-Dimethyl-5-[4-(5,6-dichlor-benzthiazol-2-yloxy)phenylcarbamoyl]barbitursäure ;
1,3-Dimethyl-5-[4-(6,7-dichlor-benzthiazol-2-yloxy)phenylcarbamoyl]-barbitursäure ;
1,3-Dimethyl-5-[4-benzthiazol-2-yloxy)phenylcarbamoyl]barbitursäure ;
1,3-Dimethyl-5-[4-(5-chlor-6-fluor-benzthiazol-2-yloxy)phenylcarbamoyl]barbitursäure ;
1,3-Dimethyl-5-[4-(7-chlor-6-fluor-benzthiazol-2-yloxy)phenylcarbamoyl]barbitursäure ;
1,3-Dimethyl-5-[4-(6-trifluormethoxy-benzthiazol-2-yloxy)phenylcarbamoyl]barbitursäure ;
1,3-Dimethyl-5-[2-isopropyl-4-(5-cyclopropyl-1,3,4-oxadiazol-2-yloxy)phenylcarbamoyl]barbitursäure ;
1,3-Dimethyl-5-[4-(5-cyclohexyl-1,3,4-thiadiazol-2-yloxy)phenylcarbamoyl]barbitursäure ;
1,3-Dimethyl-5-[4-(1-methyl-3-pentaftuorethy)-1H-1,2,4-triazol-5-yloxy)phenylcarbamoyl]barbitursäure ;
1,3-Dimethyl-5-[4-(6-methoxy-benzthiazol-2-yloxy)phenylcarbamoyl]-barbitursäure ;
1,3-Dimethyl-5-[4-(5-cyclopropyl-1,3,4-thiadiazol-2-yloxy)phenylcarbamoyl]barbitursäure ;
1,3-Dimethyl-5-[4-(6-chlor-7-fluor-benzthiazol-2-yloxy)phenylcarbamoyl]barbitursäure ;
1-Methyl-3-methoxy-5-[4-(6-chlor-7-fluor-benzthiazol-2-yloxy)phenylcarbamoyl]barbitursäure und 1,3-Dimethyl-5-[3-methoxy-4(6-chlor-7-fluorbenzthiazol-2-yloxy)phenylcarbamoyl]barbitursäure.

16. Verfahren zur Herstellung einer Verbindung der Formel I nach einem der Ansprüche 1 bis 15, dadurch gekennzeichnet, dass man
a) bei Temperaturen zwischen 50° und 250 °C einen Ester der Formel II 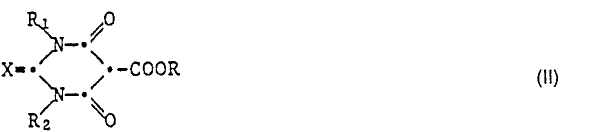 mit einem Anilinderivat der Formel III 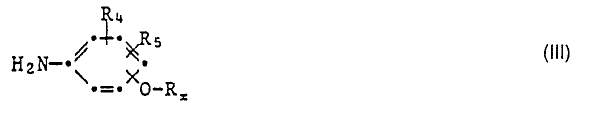 worin R eine Niederalkyl- oder eine gegebenenfalls durch Nitro substituierte Phenylgruppe darstellt, oder
b) bei Temperaturen zwischen 0° und 220 °C eine substituierte Barbitursäure der Formel IV 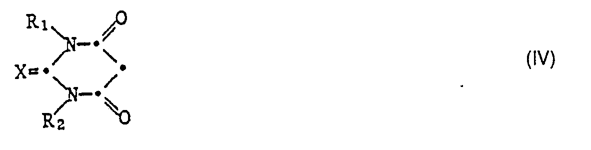 mit einem substituierten Phenylisocyanat der Formel V 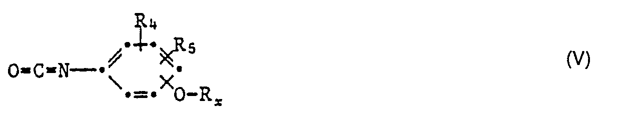 oder
c) bei Temperaturen zwischen 50° und 250 °C eine substituierte Barbitursäure der Formel IV mit einem substituierten Benzoylazid der Formel VI 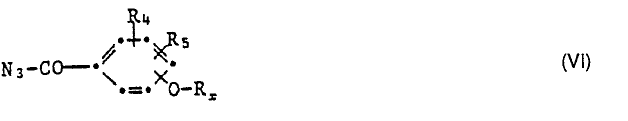 umsetzt, wobei die Substituenten X, R₁, R₂, R₄ und R₅ in den Formeln II bis VI die unter Formel I angegebenen Bedeutungen haben und Rₓ für Wasserstoff oder einen unter R₃ angegebenen Rest steht und man in den Fällen in denen Rₓ für Wasserstoff steht, entweder auf der Stufe der Ausgangsprodukte eines der Hydroxy-Derivate (Rₓ = H) der Formeln III, V oder VI mit einer Verbindung der Formel XX 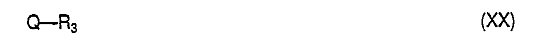 worin Q für eine übliche Abgangsgruppe steht, bei Temperaturen von 50° bis 150°C verethert und dann zum Endprodukt weiterreagieren lässt oder die Hydroxy-Derivate der Formeln III, V oder VI zuerst mit einer der Verbindungen II oder IV zu einem Hydroxy-Derivat (Rₓ = H) der Formel I' umsetzt, worin X, R₁, R₂, R₄ und R₅ wie unter Formel I definiert sind und Rₓ für Wasserstoff steht und dann erst das Hydroxy-Derivat der Formel I' mit der Verbindung der Formel XX verethert, wobei die Reaktionen (a), (b) und (c) bei normalem oder erhöhtem Druck und in Ab- oder Anwesenheit reaktionsinerter Lösungs- oder Verdünnungsmittel durchgeführt werden.

17. Verfahren nach Anspruch 16, dadurch gekennzeichnet, dass die Verfahren (a), (b) und (c) in Gegenwart reaktionsinerter Lösungs- oder Verdünnungsmittel durchgeführt werden.

18. Verfahren nach den Ansprüchen 16 und 17, dadurch gekennzeichnet, dass die Verfahrensvarianten (a) und (c) bei 70° bis 220 °C, Verfahrensvariante (b) bei 0° bis 200 °C und die Veretherung bei 80° bis 120 °C durchgeführt werden.

19. Verfahren nach den Ansprüchen 16 bis 18, dadurch gekennzeichnet, dass die Verfahren (a), (b) und (c) in Gegenwart einer Base durchgeführt werden.

20. Anthelmintisches Mittel, dadurch gekennzeichnet, dass es neben üblichen Formulierungshilfsstoffen als aktive Komponente mindestens eine Verbindung der Formel nach einem der Ansprüche 1 bis 15 enthält.

21. Mittel nach Anspruch 20, dadurch gekennzeichnet, dass es einen Wirkstoffgehalt von 0,1 bis 99,0 Gew.- % und einen Gehalt an Träger- und weiteren Hilfsstoffen von 99,9 bis 1 Gew.-% besitzt.

22. Verbindung nach einem der Ansprüche 1. bis 15 zur Verwendung in einem Verfahren zur Bekämpfung parasitärer Helminthen beim Nutztier.

23. Mittel nach einem der Ansprüche 20 oder 21 zur Verwendung in einem Verfahren zur Bekämpfung von parasitären Helminthen beim Nutztier.

24. Verwendung einer Verbindung der Formel I nach einem der Ansprüche 1 bis 15 zur Herstellung von Anthelmintika.

## Patentansprüche (Patentansprüche für folgende(n) Vertragsstaat(en) : AT)

1. Anthelmintisches Mittel, dadurch gekennzeichnet, dass es neben üblichen Formulierungshilfsstoffen als aktive Komponente mindestens eine Verbindung der Formel I, 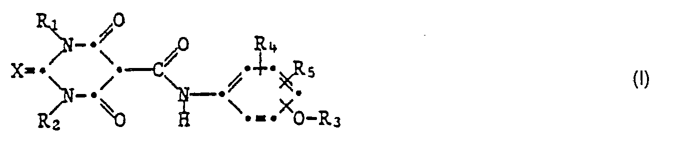 oder eines seiner Tautomeren, gegebenenfalls als Salz, enthält, worin
X für Sauerstoff oder Schwefel steht;
R₁ C₁-C₆-Alkyl, C₁-C₆-Alkoxy, C₃-C₆-Cycloalkyl oder Allyl bedeutet ;
R₂ für C₁-C₆-Alkyl oder Allyl steht ;
R₃ für ein unsubstituiertes oder substituiertes, über Kohlenstoff gebundenes, heteroaromatisches Fünfringazol steht, ausgewählt aus der Reihe Benzimidazol, Benzoxazol, Benzthiazol, Imidazol, Oxazol, Thiazol, Oxadiazol, Thiadiazol und Triazol ; und
R₄ und R₅ unabhängig voneinander Wasserstoff, C₁-C₆-Alkyl, C₁-C₆-Haloalkyl, C₁-C₆-Alkoxy oder C₁-C₆-Haloalkoxy bedeuten.

2. Mittel nach Anspruch 1, dadurch gekennzeichnet, dass es einen Wirkstoffgehalt von 0,1 bis 99,0 Gew.-% und einen Gehalt an Träger- und weiteren Hilfsstoffen von 99,9 bis 1 Gew.-% besitzt.

3. Mittel nach einem der Ansprüche 1 oder 2, dadurch gekennzeichnet, dass es als Wirkstoff eine Verbindung der Formel I enthält, worin X für Sauerstoff oder Schwefel steht ; R₁ C₁-C₆-Alkyl, C₁-C₆-Alkoxy, C₃-C₆-Cycloalkyl oder Allyl bedeutet ; R₂ für C₁-C₆-Alkyl oder Allyl steht ; R₄ und R₅ unabhängig voneinander für Wasserstoff, C₁-C₆-Alkyl, C₁-C₆-Alkoxy oder C₁-C₆-Haloalkoxy stehen ; R₃ eine der Gruppen ausgewählt aus der Reihe 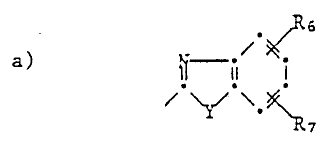 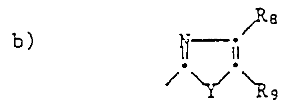 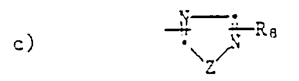 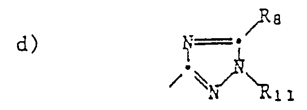 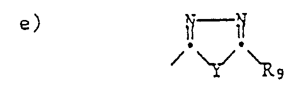 und 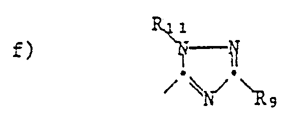 repräsentiert, wobei
Y für Sauerstoff, Schwefel oder NR₁₀ steht ;
Z für Sauerstoff, Schwefel oder NH steht;
R₆ und R₇ unabhängig voneinander Wasserstoff, C₁-C₄-Alkyl, C₁-C₄-Haloalkyl, C₁-C₄-Alkoxy, C₁-C₄-Haloalkoxy, C₁-C₄-Alkylthio, Halogen, Nitro oder Cyano bedeuten ;
R₈ und Rg unabhängig voneinander für Wasserstoff, C₁-C₆-Alkyl, C₁-C₆-Haloalkyl, C₁-C₆-Alkoxy, C₁-C₆-Alkylthio, Halogen, Nitro oder unabhängig voneinander für unsubstituiertes oder durch Halogen oder C₁-C₃-Alkyl substituiertes C₃-CᵣCycloalkyl stehen ;
R₁₀ Wasserstoff oder C₁-C₆-Alkyl bedeutet ; und
R₁₁ für C₁-C₆-Alkyl steht.

4. Mittel nach Anspruch 3, enthaltend als Wirkstoff eine der Verbindungen der Formel I, worin X für Sauerstoff oder Schwefel steht ; R₁ C₁-C₆-Alkyl, C₁-C₆-Alkoxy, C₃-C₆-Cycloalkyl oder Allyl bedeutet ; R₂ für C₁-C₆-Alkyl oder Allyl steht ; R₄ und R₅ unabhängig voneinander für Wasserstoff, C₁-C₆-Alkyl, C₁-C₆-Alkoxy oder C₁-C₆-Haloalkoxy stehen ; R₃ eine der Gruppen ausgewählt aus der Reihe 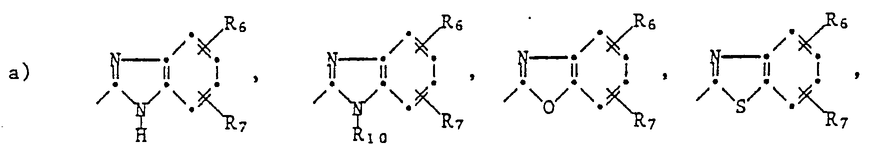 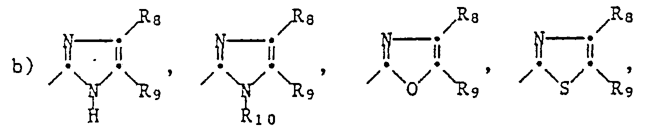 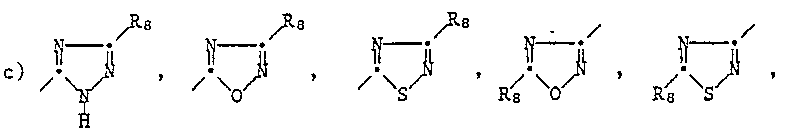 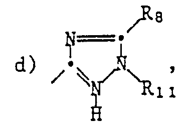 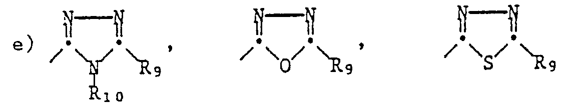 und 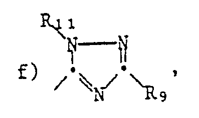 repräsentiert, wobei
R₆ und R₇ unabhängig voneinander Wasserstoff, C₁-C₄-Alkyl, C₁-C₄-Haloalkyl, C₁-C₄-Alkoxy, C₁-C₄-Haloalkoxy, C₁-C₄-Alkylthio, Halogen, Nitro oder Cyano bedeuten ;
R₈ und Rg unabhängig voneinander für Wasserstoff, C₁-C₆-Alkyl, C₁-C₆-Haloalkyl, C₁-C₆-Alkoxy, C₁-C₆-Alkylthio, Halogen oder Nitro stehen ;
R₁₀ Wasserstoff oder C₁-C₆-Alkyl bedeutet ; und
R₁₁ für C₁-C₆-Alkyl steht.

5. Mittel nach Anspruch 3, enthaltend als Wirkstoff eine der Verbindungen der Formel 1, worin X für Sauerstoff oder Schwefel steht ; R₁ C₁-C₆-Alkyl, C₁-C₆-Alkoxy, C₃-Cₛ-Cycloalkyl oder Allyl bedeutet ; R₂ für C₁-C₆-Alkyl oder Allyl steht ; R₄ und R₅ unabhängig voneinander für Wasserstoff, C₁-C₆-Alkyl, C₁-C₆-Alkoxy oder C₁-C₆-Haloalkoxy stehen ; R₃ für die Gruppe 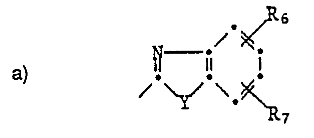 steht, wobei R₆ und R₇ unabhängig voneinander für Wasserstoff, C₁-C₄-Alkyl, C₁-C₄-Haloalkyl, C₁-C₄-Alkoxy, C₁-C₄-Haloalkoxy, C₁-C₄-Alkylthio, Halogen, Nitro oder Cyano stehen ; und Y Sauerstoff, Schwefel oder NR₁₀ bedeutet ; wobei R₁₀ für Wasserstoff oder C₁-C₆-Alkyl steht.

6. Mittel nach Anspruch 3, enthaltend als Wirkstoff eine der Verbindungen der Formel 1, worin X für Sauerstoff oder Schwefel steht; R₁ C₁-C₆-Alkyl, C₁-C₆-Alkoxy, C₃-C₆-Cycloalkyl oder Allyl bedeutet ; R₂ für C₁-C₆-Alkyl oder Allyl steht; R₄ und Rₛ unabhängig voneinander für Wasserstoff, C₁-C₆-Alkyl, C₁-C₆-Alkoxy oder C₁-C₆-Haloalkoxy stehen ; R₃ für die Gruppe 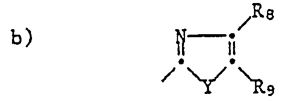 steht; wobei R₈ und Rg unabhängig voneinander für Wasserstoff, C₁-C₆-Alkyl, C₁-C₆-Haloalkyl, C₁-C₆-Alkoxy, C₁-C₆-Alkylthio, Halogen oder Nitro stehen ; und Y Sauerstoff, Schwefel oder NR₁₀ bedeutet, wobei R₁₀ für Wasserstoff oder C₁-C₆-Alkyl steht.

7. Mittel nach Anspruch 3, enthaltend als Wirkstoff eine der Verbindungen der Formel I, worin X für Sauerstoff oder Schwefel steht ; R₁ C₁-C₆-Alkyl, C₁-C₆-Alkoxy, C₃-C₆-Cycloalkyl oder Allyl bedeutet ; R₂ für C₁-C₆-Alkyl oder Allyl steht ; R₄ und R₅ unabhängig voneinander für Wasserstoff, C₁-C₆-Alkyl, C₁-C₆-Alkoxy oder C₁-C₆-Haloalkoxy stehen ; R₃ für die Gruppe 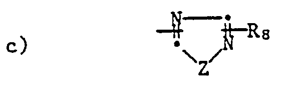 steht; worin R₈ für Wasserstoff, C₁-C₆-Alkyl, C₁-C₆-Haloalkyl, C₁-C₆-Alkoxy, C₁-C₆-Alkylthio, Halogen oder Nitro steht ; und Z Sauerstoff, Schwefel oder NH repräsentiert.

8. Mittel nach Anspruch 3, enthaltend als Wirkstoff eine der Verbindungen der Formel I, worin X für Sauerstoff oder Schwefel steht ; R₁ C₁-C₆-Alkyl, C₁-C₆-Alkoxy, C₃-C₆-Cycloalkyl oder Allyl bedeutet ; R₂ für C₁-C₆-Alkyl oder Allyl steht ; R₄ und R₅ unabhängig voneinander für Wasserstoff, C₁-C₆-Alkyl C₁-C₆-Alkoxy oder C₁-C₆-Haloalkoxy stehen ; R3 für die Gruppe 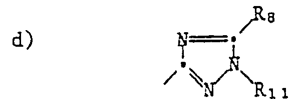 steht; worin R₈ für Wasserstoff, C₁-C₆-Alkyl, C₁-C₆-Haloalkyl, C₁-C₆-Alkoxy, C₁-C₆-Alkylthio, Halogen oder Nitro steht ; und R₁₁ für C₁-C₆-Alkyl steht.

9. Mittel nach Anspruch 3, enthaltend als Wirkstoff eine der Verbindungen der Formel I, worin X für Sauerstoff oder Schwefel steht ; R₁ C₁-C₆-Alkyl, C₁-C₆-Alkoxy, C₃-C₆-Cycloalkyl oder Allyl bedeutet ; R₂ für C₁-C₆-Alkyl oder Allyl steht ; R₄ und R₅ unabhängig voneinander für Wasserstoff, C₁-C₆-Alkyl, C₁-C₆-Alkoxy oder C₁-C₆-Haloalkoxy stehen ; R₃ für die Gruppe 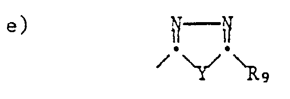 steht; worin Rg Wasserstoff, C₁-C₆-Alkyl, C₁-C₆-Haloalkyl, C₁-C₆-Alkoxy, C₁-C₆-Alkylthio, Halogen oder Nitro bedeutet ; Y für Sauerstoff, Schwefel oder NR₁₀ steht ; und R₁₀ Wasserstoff oder C₁-C₆-Alkyl bedeutet.

10. Mittel nach einem der Ansprüche 1 oder 2, enthaltend als Wirkstoff Verbindungen der Formel I, dadurch gekennzeichnet, dass X für Sauerstoff oder Schwefel steht ; R₁ C₁-C₆-Alkyl, C₁-C₆-Alkoxy, C₃-Cₛ-Cycloalkyl oder Allyl bedeutet ; R₂ für C₁-C₆-Alkyl oder Allyl steht ; R₄ und R₅ unabhängig voneinander für Wasserstoff, C₁-C₆-Alkyl, C₁-C₆-Alkoxy oder C₁-C₆-Haloalkoxy stehen ; R₃ eine der Gruppen ausgewählt aus der Reihe 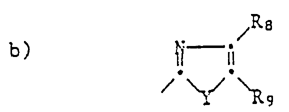 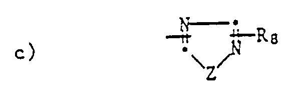 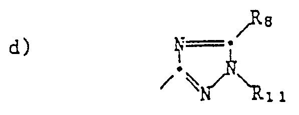 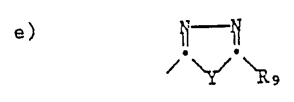 und 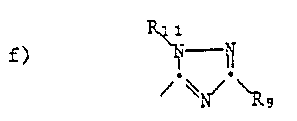 repräsentiert; R₈ und Rg unabhängig voneinander für Wasserstoff, C₁-C₆-Alkyl, C₁-C₆-Haloalkyl, C₁-C₆-Alkoxy, C₁-C₆-Alkylthio, Halogen, Nitro oder unabhängig voneinander für unsubstituiertes oder durch Halogen oder C₁-C₃-Alkyl substituiertes C₃-C₇-Cycloalkyl stehen ; Y für Sauerstoff, Schwefel oder NR₁₀ steht ; Z für Sauerstoff, Schwefel oder NH steht; R₁₀ Wasserstoff oder C₁-C₆-Alkyl bedeutet ; und R₁₁ für C₁-C₆-Alkyl steht.

11. Mittel nach Anspruch 3, enthaltend als Wirkstoff eine der Verbindungen der Formel I, worin R₃ für die Gruppe 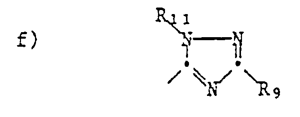 steht ; worin Rg Wasserstoff, C₁-C₆-Alkyl, C₁-C₆-Haloalkyl, C₁-C₆-Alkoxy, C₁-C₆-Alkylthio, Halogen oder Nitro bedeutet ; und R₁₁ für C₁-C₆-Alkyl steht.

12. Mittel nach Anspruch 3, enthaltend als Wirkstoff eine der Verbindungen der Formel I, worin X für Sauerstoff oder Schwefel steht; R₁ C₁-C₆-Alkyl, C₁-C₆-Alkoxy, C₃-Cₛ-Cycloalkyl oder Allyl bedeutet ; R₂ für C₁-C₆-Alkyl oder Allyl steht ; R₄ und R₅ unabhängig voneinander für Wasserstoff, C₁-C₆-Alkyl, C₁-C₆-Alkoxy oder C₁-C₅-Haloalkoxy stehen ; R₃ für die Gruppe 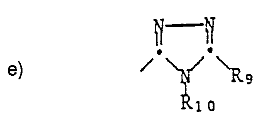 steht, worin Rg Wasserstoff, C₁-C₆-Alkyl, C₁-C₆-Haloalkyl, C₁-C₆-Alkoxy, C₁-C₆-Alkylthio, Halogen oder Nitro bedeutet ; und R₁₀ für Wasserstoff oder C₁-C₆-Alkyl steht.

13. Mittel nach einem der Ansprüche 3 bis 12, enthaltend eine der Verbindungen der Formel I, worin X für Sauerstoff oder Schwefel steht, R₁ für Methyl oder Methoxy steht ; R₂ für Methyl steht ; R₄ und R₅ unabhängig voneinander für Wasserstoff, Methyl, CF₃, OCH₃, OCHF₂ oder OCF₃ stehen ; und die übrigen Substituenten wie im rückbezogenen Anspruch definiert sind.

14. Mittel nach Anspruch 5, enthaltend als Wirkstoff eine der Verbindungen der Formel I, worin R₆ und R₇ unabhängig voneinander für Wasserstoff, Methyl, CF₃, Methoxy, Halomethoxy, Fluor, Chlor oder Brom stehen und die übrigen Substituenten wie in einem der Ansprüche 1 bis 12 definiert sind.

15. Mittel nach einem der Ansprüche 6 bis 9 und 11 bis 13, enthaltend als Wirkstoff eine der Verbindungen der Formel I worin R₈ und Rg unabhängig voneinander für Wasserstoff, Methyl, Ethyl, C₃-C₇-Cycloalkyl, CF₃, C₂F₅, C₃F₇, CCI₃, CHCI₂, CH₂CI, SCH₃ oder Halogen stehen ; R₁₀ Wasserstoff oder Methyl bedeutet ; R₁₁ für C₁-C₄-Alkyl steht ; und die übrigen Substituenten wie in den Ansprüchen 5 bis 8 definiert sind.

16. Mittel nach einem der Ansprüche 1 oder 2, enthaltend als Wirkstoff eine Verbindung der Formel I, ausgewählt aus der Reihe 1,3-Dimethyl-5-[4-(6-brom-benzthiazol-2-yloxy)phenylcarbamoyl]-barbitursäure ;
1,3-Dimethyl-5-j4-(6-chlor-benzthiazol-2-yloxy)phenylcarbamoyl]-barbitursäure ;
1,3-Dimethyl-5-[4-(6-fluor-(benzthiazol-2-yloxy)phenylcarbamoyl]-barbitursäure ;
1,3-Dimethyl-5-[4-(3-dichlormethyl-1,2,4-thiadiazol-5-yloxy)phenylcarbamoyl]barbitursäure ;
1,3-Dimethyl-5-[4-(5-tert.-butyl-1,3,4-oxadiazol-2-yloxy)phenylcarbamoyl]barbitursäure ;
1,3-Dimethyl-5-[4-(5-tert.-butyl-1,3,4-thiadiazol-2-yloxy)phenylcarbamoyl]barbitursäure;
1-Methyl-3-methoxy-5-[4-(5-tert:buty(-1,3,4-thiadiazol-2-yloxy)-phenylcarbamoyl]barbitursäure;
1,3-Dimethyl-5-[4-(5-isopropyl-1,3,4-oxadiazol-2-yloxy)phenylcarbamoyl]barbitursäure;
1,3-Dimethyl-5-[4-(1-isopropyl-3-trifluormethyl-iH-1,2,4-triazol-5-yloxy)phenylcarbamoyl]barbitursäure;
1,3-Dimethyl-5-[2,6-dimethyl-4-(1-methyl-3-heptafluorpropyl-1 H-1,2,4-triazol-5-yloxy)phenylcarbamoyl]barbitursäure ;
1,3-Dimethy)-5-[4-(1-isopropy)-3-pentafiuorethyi-1H-1,2,4-triazoi-5-yioxy)phenyicarbamoy)]barbitursäure;
1,3-Dimethyl-5-[4-(1-isopropyl-3-trifluormethy(-1H-1,2,4-triazol-5-yloxy)-2,6-dimethyl-phenylcarbamoyl]barbitursäure ;
1,3-Dimethyl-5-[3-(1-methyl-3-trifluormethyl-1H-1,2,4-triazol-5-yloxy)phenylcarbamoyl]barbitursäure;
1,3-Dimethyl-5-[4-(6-trifluormethyl-benzthiazol-2-yloxy)phenylcarbamoyl]barbitursäure ;
1,3-Dimethyl-5-[3-methoxy-4-(5-tert-butyl-1,3,4-thiadiazol-2-yloxy)phenylcarbamoyl]barbitursäure ;
1,3-Dimethyl-5-[3-methoxy-4-(6-chlor-benzthiazol-2-yloxy)phenylcarbamoyl]barbitursäure;
1,3-Dimethyl-5-[4-(5,6-dichlor-benzthiazol-2-yloxy)phenylcarbamoyl]barbitursäure;
1,3-Dimethyl-5-[4-(6,7-dichlor-benzthiazol-2-yloxy)phenylcarbamoyl]-barbitursäure ;
1,3-Dimethyl-5-[4-(benzthiazol-2-yloxy)phenylcarbamoyl]barbitursäure ;
1,3-Dimethyl-5-[4-(5-chlor-6-fluor-benzthiazol-2-yloxy)phenylcarbamoyl]barbitursäure;
1,3-Dimethyl-5-[4-(7-chlor-6-fluorbenzthiazol-2-yloxy)phenylcarbamoyl]barbitursäure;
1,3-Dimethyl-5-[4-(6-trifluormethoxy-benzthiazol-2-yloxy)phenylcarbamoyl]barbitursäure;
1,3-Dimethyl-5-[2-isopropyl-4-(5-cyclopropyl-1,3,4-oxadiazol-2-yloxy)phenylcarbamoyl]barbitursäure;
1,3-Dimethyl-5-[4-(5-cyclohexyl-1,3,4-thiadiazol-2-yloxy)phenylcarbamoyl]barbitursäure;
1,3-Dimethyl-5-[4-(1-methyl-3-pentafluorethyl-1 H-1,2,4-triazol-5-yloxy)phenylcarbamoyl]barbitursäure;
1,3-Dimethyl-5-[4-(6-methoxy-benzthiazol-2-yloxy)phenylcarbamoyl]-barbitursäure ;
1,3-Dimethy!-5-[4-(5-cyclopropyl-1,3,4-thiadiazol-2-yloxy)phenylcarbamoyl]barbitursäure;
1,3-Dimethyl-5-[4-(6-chlor-7-fluor-benzthiazol-2-yloxy)phenylcarbamoyl]barbitursäure;
1-Methyl-3-methoxy-5-[4-(6-chlor-7-fluor-benzthiazol-2-yloxy)phenylcarbamoyl]barbitursäure und
1,3-Dimethyl-5-[3-methoxy-4(6-chlor-7-fluorbenzthiazol-2-yloxy)-phenylcarbamoyl]barbitursäure.

17. Verfahren zur Herstellung einer Verbindung der Formel nach einem der Ansprüche 1 bis 16, dadurch gekennzeichnet, dass man
a) bei Temperaturen zwischen 50° und 250 °C einen Ester der Formel II 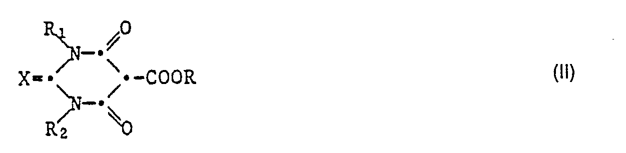 mit einem Anilinderivat der Formel III 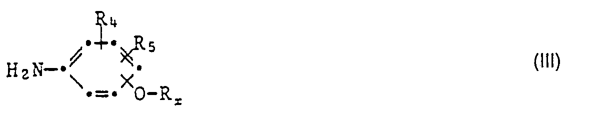 worin R eine Niederalkyl- oder eine gegebenenfalls durch Nitro substituierte Phenylgruppe darstellt, oder
b) bei Temperaturen zwischen 0° und 220 °C eine substituierte Barbitursäure der Formel IV 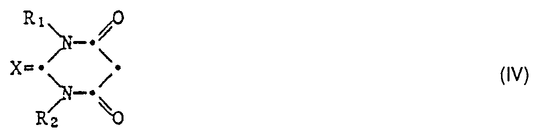 mit einem substituierten Phenylisocyanat der Formel V 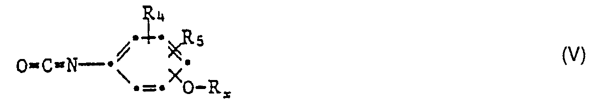 oder
c) bei Temperaturen zwischen 50° und 250 °C eine substituierte Barbitursäure der Formel IV mit einem substituierten Benzoylazid der Formel VI 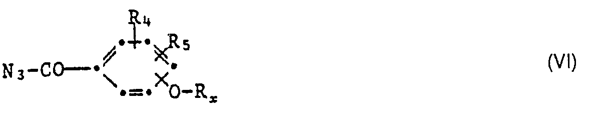 umsetzt, wobei die Substituenten X, R₁, R₂, R₄ und R₅ in den Formeln II bis VI die unter Formel angegebenen Bedeutungen haben und Rₓ für Wasserstoff oder einen unter R₃ angegebenen Rest steht und man in den Fällen in denen Rₓ für Wasserstoff steht, entweder auf der Stufe der Ausgangsprodukte eines der Hydroxy-Derivate (Rₓ = H) der Formeln III, V oder VI mit einer Verbindung der Formel XX 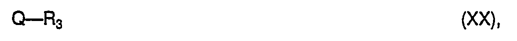 worin Q für eine übliche Abgangsgruppe steht, bei Temperaturen von 50° bis 150 °C verethert und dann zum Endprodukt weiterreagieren lässt oder die Hydroxy-Derivate der Formeln 111, V oder VI zuerst mit einer der Verbindungen II oder IV zu einem Hydroxy-Derivat (Rₓ = H) der Formel I' umsetzt, worin X, R₁, R₂, R₄ und R₅ wie unter Formel I definiert sind und Rₓ für Wasserstoff steht und dann erst das Hydroxy-Derivat der Formel I' mit der Verbindung der Formel XX verethert, wobei die Reaktionen (a), (b) und (c) bei normalem oder erhöhtem Druck und in An- oder Abwesenheit reaktionsinerter Lösungs- oder Verdünnungsmittel durchgeführt werden.

18. Verfahren nach Anspruch 17, dadurch gekennzeichnet, dass die Verfahren (a), (b) und (c) in Gegenwart reaktionsinerter Lösungs- oder Verdünnungsmittel durchgeführt werden.

19. Verfahren nach den Ansprüchen 17 und 18, dadurch gekennzeichnet, dass die Verfahrensvarianten (a) und (c) bei 70° bis 220 °C, Verfahrensvariante (b) bei 0° bis 200 °C und die Veretherung bei 80° bis 120 °C durchgeführt werden.

20. Verfahren nach den Ansprüchen 17 bis 19, dadurch gekennzeichnet, dass die Verfahren (a), (b) und (c) in Gegenwart einer Base durchgeführt werden.

21. Verbindung der Formel I nach einem der Ansprüche 1 bis 16 zur Verwendung in einem Verfahren zur Bekämpfung parasitärer Helminthen beim Nutztier.

22. Mittel nach einem der Ansprüche 1 bis 16 zur Verwendung in einem Verfahren zur Bekämpfung parasitärer Helminthen beim Nutztier.

23. Verwendung einer Verbindung der Formel I nach einem der Ansprüche 1 bis 16 zur Herstellung von Anthelmintika.

## Claims (Claims for the following Contracting State(s) : DE, FR, CH, LI, IT, NL, BE, SE, LU)

1. Compounds of formula I 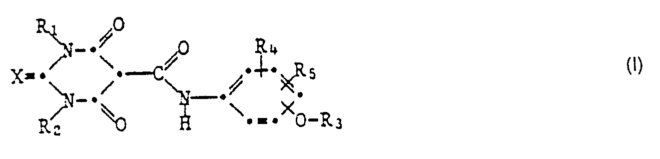 and also the tautomers and salts thereof wherein
X is oxygen or sulfur ;
R₁ is C₁-C₆alkyl, C₁-C₆alkoxy, C₃-C₆cycloalkyl or allyl ;
R₂ is C₁-C₆alkyl or allyl ;
R₃ is an unsubstituted or substituted heteroaromatic five-membered azole ring which is bound through carbon and is selected from the series consisting of benzimadazole, benzoxazole, benzothiazole, imidazole, oxazole, thiazole, oxadiazole, thiadiazole and triazole ; and
R₄ and R₅ are each independently of the other hydrogen, C₁-C₆alkyl, C₁-C₆haloalkyl, C₁-C₆alkoxy or C₁-C₆haloalkoxy.

2. Compounds of formula I according to claim 1, wherein X is oxygen or sulfur; R₁ is C₁-Csalkyl, C₁-C₆alkoxy, C₃-C₆cycloalkyl or allyl ; R₂ is C₁-C₆alkyl or allyl ; R₄ and R₅ are each independently of the other hydrogen, C₁-C₆alkyl, C₁-C₆alkoxy or C₁-C₆haloalkoxy ; R₃ is a radical selected from the series consisting of 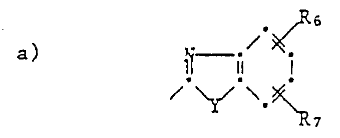 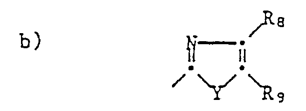 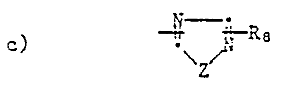 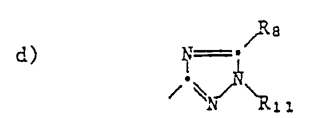 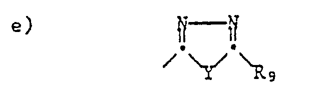 and 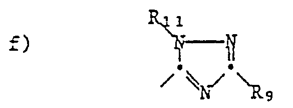 in which formulae
Y is oxygen, sulfur or NR₁₀ ;
Z is oxygen, sulfur or NH ;
R₈ and R₇ are each independently of the other hydrogen, C₁-C₄alkyl, C₁-C₄haloalkyl, C₁-C₄alkoxy, C₁-C₄haloalkoxy, C₁-C₄alkylthio, halogen, nitro or cyano ;
R₈ and Rg are each independently of the other hydrogen, C₁-C₆alkyl, C₁-C₇haloalkyl, C₁-C₆alkoxy, C₁-C₆alkylthio, halogen or nitro or are each independently of the other C₃-C₇ycloalkyl that is unsubstituted or substituted by halogen or by C₁-C₃alkyl ;
R₁₀ is hydrogen or C₁-C₆alkyl ; and
R₁₁ is C₁-C₈alkyl.

3. Compounds of formula I according to claim 2, wherein X is oxygen or sulfur ; R₁ is C₁-C₆alkyl, C₁-C₆alkoxy, C₃-Cₛcycloalkyl or allyl ; R₂ is C₁-C₆alkyl or allyl ; R₄ and R₅ are each independently of the other hydrogen, C₁-C₆alkyl, C₁-C₆alkoxy or C₁-C₆haloalkoxy; and R₃ is a radical selected from the series consisting of 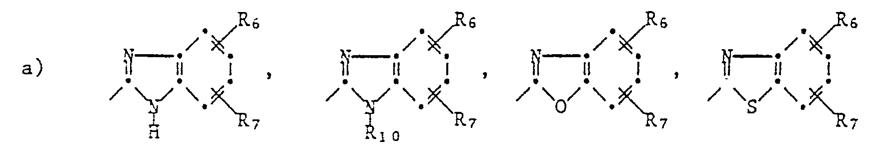 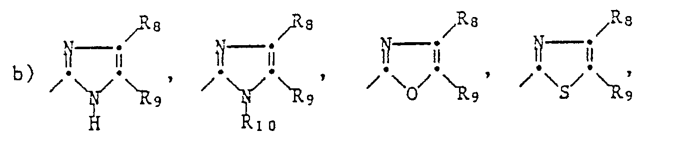 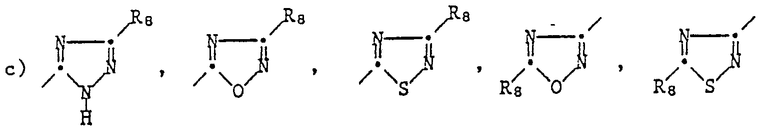 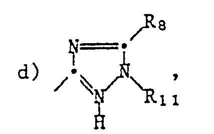 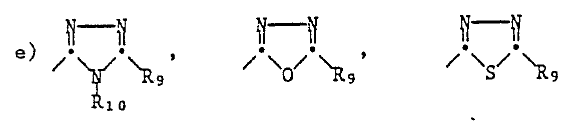 and 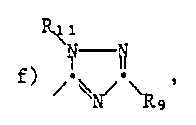 in which formulae
R₆ and R₇ are each independently of the other hydrogen, C₁-C₄alkyl, C₁-C₄haloalkyl, C₁-C₄alkoxy, C₁-C₄haloalkoxy, C₁-C₄alkylthio, halogen, nitro or cyano ;
R₈ and Rg are each independently of the other hydrogen, C₁-C₆alkyl, C₁-C₆haloalkyl, C₁-C₆alkoxy, C₁-C₆alkylthio, halogen or nitro ;
R₁₀ is hydrogen or C₁-C₆alkyl ; and
R₁₁ is C₁-C₆alkyl.

4. Compounds of formula I according to claim 2, wherein X is oxygen or sulfur ; R₁ is C₁-C₆alkyl, C₁-C₆alkoxy, C₃-C₆cycloalkyl or allyl ; R₂ is C₁-C₆alkyl or allyl ; R₄ and R₅ are each independently of the other hydrogen, C₁-C₆alkyl, C₁-C₆alkoxy or C₁-C₆haloalkoxy ; and R₃ is the radical 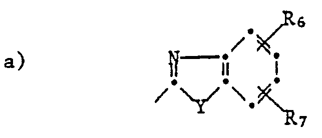 wherein R₆ and R₇ are each independently of the other hydrogen, C₁-C₄alkyl, C₁-C₄haloalkyl, C₁-C₄alkoxy, C₁-C₄haloalkoxy, C₁-C₄alkylthio, halogen, nitro or cyano ; and Y is oxygen, sulfur or NR₁₀ wherein R₁₀ is hydrogen or C₁-C₆alkyl.

5. Compounds of formula I according to claim 2, wherein X is oxygen or sulfur ; R₁ is C₁-Cₛalkyl, C₁-C₆alkoxy, C₃-C₆cycloalkyl or allyl ; R₂ is C₁-C₆alkyl or allyl ; R₄ and R₅ are each independently of the other hydrogen, C₁-C₆alkyl, C₁-C₆alkoxy or C₁-C₆haloalkoxy ; and R₃ is the radical 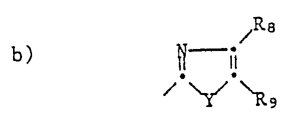 wherein R₈ and Rg are each independently of the other hydrogen, C₁-C₆alkyl, C₁-C₆haloalkyl, C₁-C₆alkoxy, C₁-C₆alkylthio, halogen or nitro ; and Y is oxygen, sulfur or NR₁₀, wherein R₁₀ is hydrogen or C₁-C₆alkyl.

6. Compounds of formula I according to claim 2, wherein X is oxygen or sulfur ; R₁ is C₁-C₆alkyl, C₁-C₆alkoxy, C₃-C₆cycloalkyl or allyl ; R₂ is C₁-C₆alkyl or allyl ; R₄ and R₅ are each independently of the other hydrogen, C₁-C₆alkyl, C₁-C₆alkoxy or C₁-C₆haloalkoxy ; and R₃ is the radical 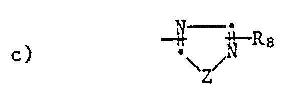 wherein R₈ is hydrogen, C₁-C₆alkyl, C₁-C₆haloalkyl, C₁-C₆alkoxy, C₁-C₆alkylthio, halogen or nitro ; and Z is oxygen sulfur or NH.

7. Compounds of formula I according to claim 2, wherein X is oxygen or sulfur : R₁ is C₁-C₆alkyl, C₁ C₆alkoxy, C₃-C₆cycloalkyl or allyl; R₂ is C₁-C₆alkyl or allyl ; R₄ and R₅ are each independently of the other hydrogen, C₁-C₆alkyl, C₁-C₆alkoxy or C₁-C₆haloalkoxy ; and R₃ is the radical 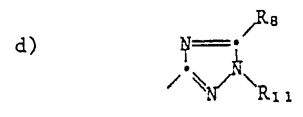 wherein R₈ is hydrogen, C₁-C₈alkyl, C₁-C₆haloalkyl, C₁-C₆alkoxy, C₁-C₆alkylthio, halogen or nitro; and R₁₁ is C₁-C₆alkyl.

8. Compounds of formula I according to claim 2, wherein X is oxygen or sulfur ; R₁ is C₁-C₅alkyl, C₁-C₆alkoxy, C₃-C₆cycloalkyl or allyl ; R₂ is C₁-C₆alkyl or allyl ; R₄ and R₅ are each independently of the other hydrogen, C₁-C₆alkyl, C₁-C₆alkoxy or C₁-C₆haloalkoxy ; and R₃ is the radical 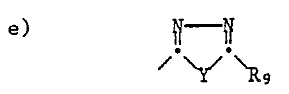 wherein Rg is hydrogen, C₁-C₆alkyl, C₁-C₆haloalkyl, C₁-C₆alkoxy, C₁-C₆alkylthio, halogen or nitro ; and Y is oxygen, sulfur or NR₁₀, wherein R₁₀ is hydrogen or C₁-C₆alkyl.

9. Compounds of formula I according to claim 1, wherein X is oxygen or sulfur; R₁ is C₁-C₆alkyl, C₁-C₆alkoxy, C₃-C₆cycloalkyl or allyl ; R₂ is C₁-C₆alkyl or allyl ; R₄ and R₅ are each independently of the other hydrogen, C₁-C₆alkyl, C₁-Cₛalkoxy or C₁-C₆haloalkoxy ; and R₃ is a radical selected from the series consisting of 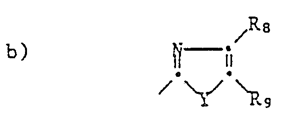 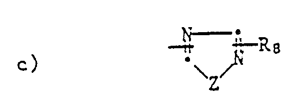 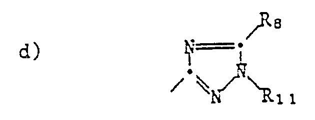 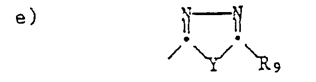 and 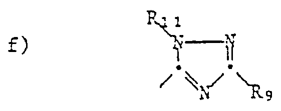 R₈ and Rg are each independently of the other hydrogen, C₁-C₆alkyl, C₁-C₆haloalkyl, C₁-C₆alkoxy, C₁-C₆alkylthio, halogen or nitro or are each independently of the other C₃-C₇cycloalkyl that is unsubstituted or substituted by halogen or by C₁-C₃alkyl ; Y is oxygen, sulfur or NR₁₀ ; Z is oxygen, sulfur or NH ; R₁₀ is hydrogen or C₁-C₆alkyl ; and R₁₁ is C₁-C₆alkyl.

10. Compounds of formula I according to claim 2, wherein X is oxygen or sulfur ; R₁ is C₁-C₆alkyl, C₁-Cₛalkoxy, C₃-C₆cycloalkyl or allyl ; R₂ is C₁-C₆alkyl or allyl ; R₄ and R₅ are each independently of the other hydrogen, C₁-C₆alkyl, C₁-C₆alkoxy or C₁-C₆haloalkoxy ; and R₃ is the radical 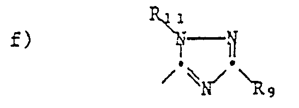 wherein Rg is hydrogen, C₁-C₆alkyl, C₁-C₆haloalkyl, C₁-C₆alkoxy, C₁-C₆alkylthio, halogen or nitro ; and R₁₁ is C₁-C₆alkyl.

11. Compounds of formula I according to claim 2, wherein R₃ is the radical 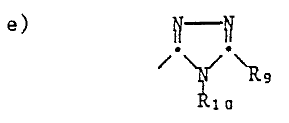 wherein Rg is hydrogen, C₁-C₆alkyl, C₁-C₆haloalkyl, C₁-C₆alkoxy, C₁-C₆alkylthio, halogen or nitro; and R₁₀ is hydrogen or C₁-C₆alkyl.

12. Compounds of formula I according to any one of claims 1 to 11, wherein X is oxygen or sulfur ; R₁ is methyl or methoxy ; R₂ is methyl ; R₄ and R₅ are each independently of the other hydrogen, methyl, CF₃, OCH₃, OCHF₂ or OCF₃ ; and the remaining substituents are as defined in the relevant claim.

13. Compounds of formula I according to claim 4, wherein R₆ and R₇ are each independently of the other hydrogen, methyl, CF₃, methoxy, halomethoxy, fluorine, chlorine or bromine and the remaining substituents are as defined in any one of claims 1 to 12.

14. Compounds of formula I according to any one of claims 5 to 8, 10 and 11, wherein R₈ and Rg are each independently of the other hydrogen, methyl, ethyl, C₃-C₇cycloalkyl, CF₃, C₂Fₛ, C₃F₇, CCI₃, CHCI₂, CH₂CI, SCH₃ or halogen ; R₁₀ is hydrogen or methyl ; R₁₁ is C₁-C₄alkyl ; and the remaining substituents are as defined in claims 5 to 8.

15. A compound of formula I according to any one of claims 1 to 3, selected from the series consisting of 1,3-dimethyl-5-[4-(6-bromobenzothiazol-2-yloxy)phenyl-carbamoyl]barbituric acid ;
1,3-dimethyl-5-[4-(6-chlorobenzothiazol-2-yloxy)phenyl-carbamoyl]barbituric acid ;
1,3-dimethyl-5-[4-(6-fluoro(benzothiazol-2-yloxy)phenylcarbamoyl]barbituric acid ;
1,3-dimethyl-5-[4-(3-dichloromethyl-1,2,4-thiadiazol-5-yloxy)phenylcarbamoyl]barbituric acid ;
1,3-dimethyl-5-[4-(5-tert.-butyl-1 ,3,4-oxadiazol-2-yloxy)phenylcarbamoyl]barbituric acid ;
1,3-dimethyl-5-[4-(5-tert.-butyl-1,3,4-thiadiazol-2-yloxy)phenylcarbamoyl]barbituric acid ;
1-methyl-3-methoxy-5-[4-(5-tert.-butyl-1,3,4-thiadiazol-2-yloxy)phenylcarbamoyl]barbituric acid ;
1,3-dimethyl-5-[4-(5-isopropyl-1,3,4-oxadiazol-2-yloxy)-phenylcarbamoyl]barbituric acid ;
1,3-dimethyl-5-[4-(1-isopropyl-3-trifluoromethyl-1H-1,2,4-triazol-5-yloxy)phenyfcarbamoyl]barbituric acid ;
1,3-dimethyl-5-[2,6-dimethyl-4-(1-methyl-3-heptafluoropropyl-1 H-1,2,4-triazol-5-yloxy)phenylcarbamoyl]barbituric acid ;
1,3-dimethyl-5-[4-(1-isopropyl-3-pentafluoroethyl-1 H-1,2,4-triazol-5-yloxy)phenylcarbamoyl]barbituric acid ;
1,3-dimethyl-5-[4-(1-isopropyl-3-trifluoromethyl-1 H-1,2,4-triazol-5-yloxy)-2,6-dimethylphenylcar- bamoyl]barbituric acid ;
1,3-dimethyl-5-[3-(1-methyl-3-trifluoromethyl-1H-1,2,4-triazol-5-yloxy)phenylcarbamoyl]barbituric acid ;
1,3-dimethyl-5-[4-(6-trifluoromethylbenzothiazol-2-yloxy)phenylcarbamoyl]barbituric acid ;
1,3-dimethyl-5-[3-methoxy-4-(5-tert.-butyl-1 ,3,4-thiadiazol-2-yloxy)phenylcarbamoyl]barbituric acid ;
1,3-dimethyl-5-[3-methoxy-4-(6-chlorobenzothiazol-2-yloxy)phenylcarbamoyl]barbituric acid;
1,3-dimethyl-5-[4-(5,6-dichlorobenzothiazol-2-yloxy)phenylcarbamoyl]barbituric acid ;
1,3-dimethyl-5-[4-(6,7-dichlorobenzothiazol-2-yloxy)phenylcarbamoyl]barbituric acid;
1,3-dimethyl-5-[4-(benzothiazol-2-yloxy)phenylcarbamoyl]barbituric acid ;
1,3-dimethyl-5-[4-(5-chloro-6-fluorobenzothiazol-2-yloxy)phenylcarbamoyl]barbituric acid ;
1,3-dimethyl-5-[4-(7-chloro-6-fluorobenzothiazol-2-yloxy)phenylcarbamoyl]barbituric acid ;
1,3-dimethyl-5-[4-(6-trifluoromethoxybenzothiazol-2-yloxy)phenylcarbamoyl]barbituric acid ;
1,3-dimethyl-5-[2-isopropyl-4-(5-cyclopropyl-1,3,4-oxadiazol-2-yloxy)phenylcarbamoyl]barbituric acid ;
1,3-dimethyl-5-[4-(5-cyclohexyl-1,3,4-thiadiazol-2-yloxy)phenylcarbamoyl]barbituric acid ;
1,3-dimethyl-5-[4-(1-methyl-3-pentafluoroethyl-1H-1,2,4-triazol-5-yloxy)phenylcarbamoyl]barbituric acid ;
1,3-dimethyl-5-[4-(6-methoxybenzothiazol-2-yloxy)phenylcarbamoyl]barbituric acid ;
1,3-dimethyl-5-[4-(5-cyclopropyl-1,3,4-thiadiazol-2-yloxy)phenylcarbamoyl]barbituric acid ;
1,3-dimethyl-5-[4-(6-chloro-7-fluorobenzothiazol-2-yloxy)phenylcarbamoyl[barbituric acid ;
1-methyl-3-methoxy-5-[4-(6-chloro-7-fluorobenzothiazol-2-yloxy)phenylcarbamoyl]barbituric acid and 1,3-dimethyl-5-[3-methoxy-4-(6-chloro-7-fluorobenzothiazol-2-yloxy)phenylcarbamoyl]barbituric acid.

16. A process for the preparation of a compound of formula I according to any one of claims 1 to 15, which comprises
a) reacting an ester of formula II 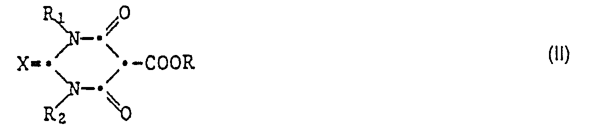 at temperatures of from 50° to 250 °C with an aniline derivative of formula III 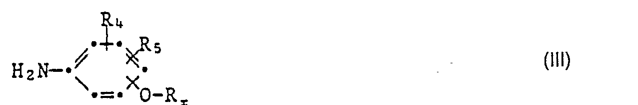 wherein R is a lower alkyl group, or a phenyl group which is unsubstituted or substituted by nitro, or
b) reacting a substituted barbituric acid of formula IV 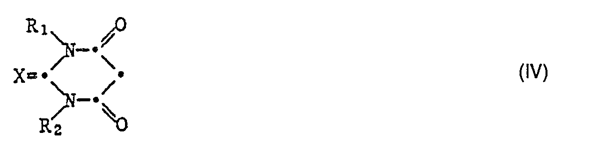 at temperatures of from 0° to 220 °C with a substituted phenylisocyanate of formula V 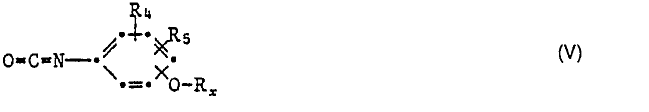 or
c) reacting a substituted barbituric acid for formula IV at temperatures of from 50° to 250 °C with a substituted benzoylazide of formula VI 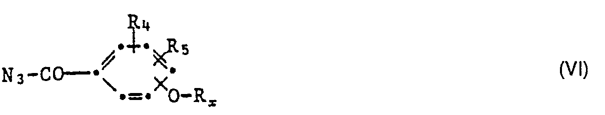 in which formulae II to VI the substituents X, R₁, R₂, R₄ and R₅ are as defined for formula I and Rₓ is hydrogen or a radical as defined for R₃, and, in those cases in which Rₓ is hydrogen either, at the starting material stage, etherifying a hydroxy derivative (Rₓ = H) of formula III, V or VI at temperatures of from 50° to 150 °C with a compound of formula XX 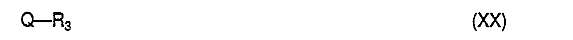 wherein Q is a customary leaving group, and then allowing the etherified product to react further to give the final product, or first reacting a hydroxy derivative of formula III, V or VI with a compound II or IV to give a hydroxy derivative (Rₓ = H) of formula l', wherein X, R₁, R₂, R₄ and R₅ are as defined for formula I and Rₓ is hydrogen, and then etherifying the said hydroxy derivative of formula I' with a compound of formula XX, the reactions (a), (b) and (c) being carried out at normal or elevated pressure in the absence or presence of an inert solvent or diluent.

17. A process according to claim 16, which comprises carrying out processes (a), (b) and (c) in the presence of an inert solvent or diluent.

18. A process according to claims 16 and 17, which comprises carrying out process variants (a) and (c) at temperatures of from 70° to 220 °C, process variant (b) at temperatures of from 0° to 200 °C and the etherification at temperatures of from 80° to 120 °C.

19. A process according to claims 16 to 18, which comprises carrying out processes (a), (b) and (c) in the presence of a base.

20. An anthelmintic composition, which contains as active ingredient at least one compound of formula I according to any one of claims 1 to 15 together with customary formulation assistants.

21. A composition according to claim 20, which composition contains from 0.1 to 99.0 % by weight of active ingredient and from 99.9 to 1 % by weight of carriers and further assistants.

22. A compound according to any one of claims 1 to 15 for use in a method of controlling parasitic helminths in productive livestock.

23. A composition according to claim 20 or claim 21 for use in a method of controlling parasitic helminths in productive livestock.

24. Use of a compound of formula I according to any one of claims 1 to 15 for the preparation of anthelminthics.

## Claims (Claims for the following Contracting State(s) : AT)

1. An anthelminthic composition which, together with the customary formulation assistants, contains as active ingredient at least one compound of formula I 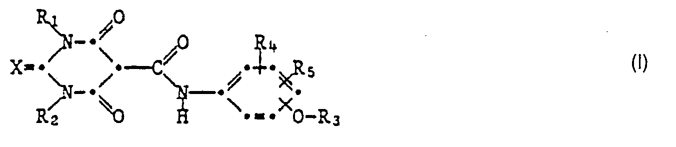 or one of the tautomers thereof, if desired in the form of a salt, wherein
X is oxygen or sulfur ;
R₁ is C₁-C₆alkyl, C₁-C₆alkoxy, C₃-Cₛcycloalkyl or allyl ;
R₂ is C₁-C₆alkyl or allyl ;
R₃ is an unsubstituted or substituted heteroaromatic five-membered azole ring which is bound through carbon and is selected from the series consisting of benzimadazole, benzoxazole, benzothiazole, imidazole, oxazole, thiazole, oxadiazole, thiadiazole and triazole ; and
R₄ and R₅ are each independently of the other hydrogen, C₁-C₆alkyl, C₁-C₆haloalkyl, C₁-C₆alkoxy or Cᵢ-Cₛhaloalkoxy.

2. A composition according to claim 1, which composition contains from 0.1 to 99.0 % by weight of active ingredient and from 99.9 to 1 % by weight of carriers and further assistants.

3. A composition according to claim 1 or claim 2, containing as active ingredient a compound of formula I, wherein X is oxygen or sulfur : R₁ is C₁-C₆alkyl, C₁-C₆alkoxy, C₃-Cₛcycloalkyl or allyl ; R₂ is C₁-C₆alkyl or allyl ; R₄ and R₅ are each independently of the other hydrogen, C₁-C₆alkyl, C₁-C₆alkoxy or C₁-C₆haloalkoxy ; and R₃ is a radical selected from the series consisting of 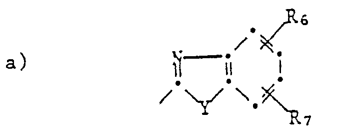 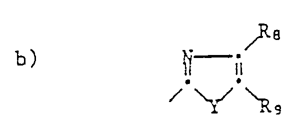 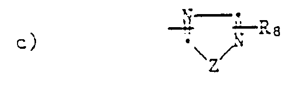 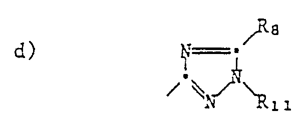 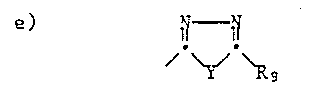 and 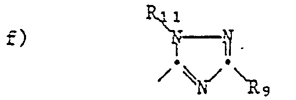 in which formulae
Y is oxygen, sulfur or NR₁₀;
Z is oxygen, sulfur or NH ;
R₆ and R₇ are each independently of the other hydrogen, C₁-C₄alkyl, C₁-C₄haloalkyl, C₁-C₄alkoxy, C₁-C₄haloalkoxy, C₁-C₄alkylthio, halogen, nitro or cyano :
R₈ and Rg are each independently of the other hydrogen, C₁-C₆alkyl, C₁-C₆haloalkyl, C₁-C₆alkoxy, C₁-Cₛalkylthio, halogen or nitro or are each independently of the other C₃-C₇cycloalkyl that is unsubstituted of substituted by halogen or by C₁-C₃alkyl ;
R₁₀ is hydrogen or C₁-C₆alkyl ; and
R₁₁ is C₁-C₆alkyl.

4. A composition according to claim 3, containing as active ingredient a compound of formula I, wherein X is oxygen or sulfur ; R₁ is C₁-C₆alkyl, C₁-C₆alkoxy, C₃-C₆cycloalkyl or allyl ; R₂ is C₁-C₆alkyl or allyl ; R₄ and R₅ are each independently of the other hydrogen, C₁-C₆alkyl, C₁-C₆alkoxy or C₁-C₆haloalkoxy ; and R₃ is a radical selected from the series consisting of 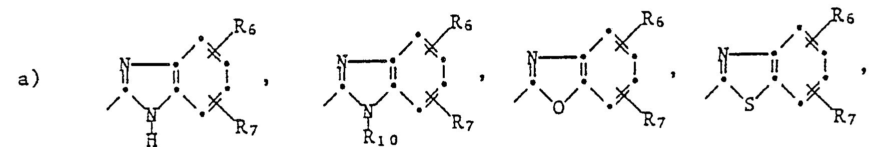 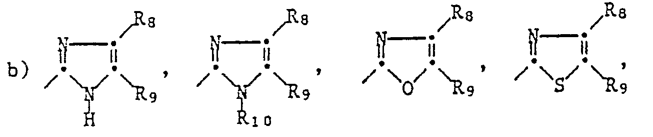 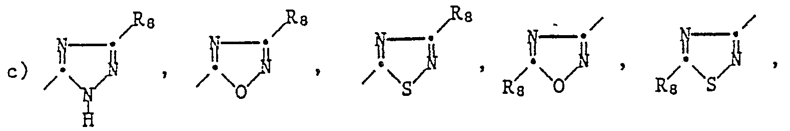 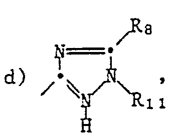 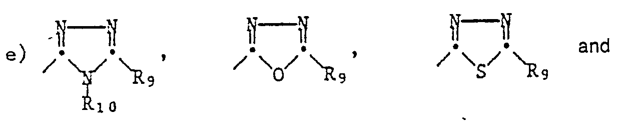 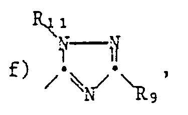 in which formulae
R₆ and R₇ are each independently of the other hydrogen, C₁-C₄alkyl, C₁-C₄haloalkyl, C₁-C₄alkoxy, C₁-C₄haloalkoxy, C₁-C₄alkylthio, halogen, nitro or cyano ;
R₈ and Rg are each independently of the other hydrogen, C₁-C₆alkyl, C₁-C₆haloalkyl, C₁-C₆alkoxy, C₁-Cₛalkylthio, halogen or nitro ;
R₁₀ is hydrogen or C₁-C₆alkyl ; and
R₁₁ is C₁-Cₛalkyl.

5. A composition according to claim 3, containing as active ingredient a compound of formula I, wherein X is oxygen or sulfur ; R₁ is C₁-C₆alkyl, C₁-C₆alkoxy, C₃-C₆cycloalkyl or allyl ; R₂ is C₁-C₆alkyl or allyl ; R₄ and Rₛ are each independently of the other hydrogen, C₁-C₆alkyl, C₁-C₆alkoxy or C₁-C₆haloalkoxy; and R₃ is the radical 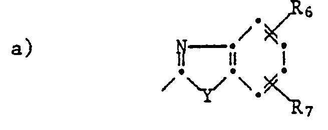 wherein R₆ and R₇ are each independently of the other hydrogen, C₁-C₄alkyl, C₁-C₄haloalkyl, C₁-C₄alkoxy, C₁-C₄haloalkoxy, C₁-C₄alkylthio, halogen, nitro or cyano ; and Y is oxygen, sulfur or NR₁₀, wherein R₁₀ is hydrogen or C₁-C₆alkyl.

6. A composition according to claim 3, containing as active ingredient a compound of formula I, wherein X is oxygen or sulfur ; R₁ is C₁-C₆alkyl, C₁-C₆alkoxy, C₃-C₆cycloalkyl or allyl ; R₂ is C₁-C₆alkyl or allyl ; R₄ and Rₛ are each independently of the other hydrogen, C₁-C₆alkyl, C₁-C₆alkoxy or C₁-C₆haloalkoxy; and R₃ is the radical 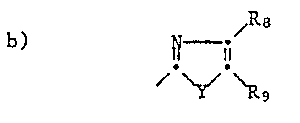 wherein R₈ and Rg are each independently of the other hydrogen, C₁-C₆alkyl, C₁-C₆haloalkyl, C₁-C₆alkoxy, C₁-C₆alkylthio, halogen or nitro ; and Y is oxygen, sulfur or NR₁₀, wherein R₁₀ is hydrogen or C₁-C₆alkyl.

7. A composition according to claim 3, containing as active ingredient'a compound of formula I, wherein X is oxygen or sulfur ; R₁ is C₁-C₆alkyl, C₁-C₆alkoxy, C₃-C₆cycloalkyl or allyl ; R₂ is C₁-C₆alkyl or allyl ; R₄ and R₅ are each independently of the other hydrogen, C₁-C₆alkyl, C₁-C₆alkoxy or C₁-C₆haloalkoxy ; and R₃ is the radical 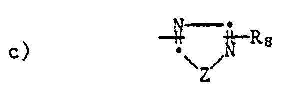 wherein R_{B} is hydrogen, C₁-C₆alkyl, C₁-C₆haloalkyl, C₁-C₆alkoxy, C₁-C₆alkylthio, halogen or nitro; and Z is oxygen, sulfur or NH.

8. A composition according to claim 3, containing as active ingredient a compound of formula I, wherein X is oxygen or sulfur ; R₁ is C₁-C₆alkyl, C₁-C₆alkoxy, C₃-C₆cycloalkyl or allyl ; R₂ is C₁-C₆alkyl or allyl ; R₄ and R₅ are each independently of the other hydrogen, C₁-C₆alkyl, C₁-C₆alkoxy or C₁-C₆haloalkoxy ; and R₃ is the radical 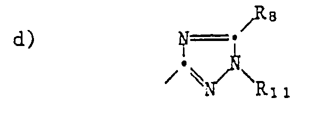 wherein R₈ is hydrogen, C₁-C₆alkyl, C₁-C₆haloalkyl, C₁-C₆alkoxy, C₁-C₆alkylthio, halogen or nitro; and R₁₁ is C₁-C₆alkyl.

9. A composition according to claim 3, containing as active ingredient a compound of formula I wherein X is oxygen or sulfur ; R₁ is C₁-C₆alkyl, C₁-C₆alkoxy, C₃-C₆cycloalkyl or allyl ; R₂ is C₁-C₆alkyl or allyl ; R₄ and R₅ are each independently of the other hydrogen, C₁-C₆alkyl, C₁-C₆alkoxy or C₁-C₆haloalkoxy; and R₃ is the radical 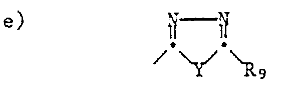 wherein Rg is hydrogen, C₁-C₆alkyl, C₁-C₆haloalkyl, C₁-C₆alkoxy, C₁-C₆alkylthio, halogen or nitro ; and Y is oxygen, sulfur or NR₁₀, wherein R₁₀ is hydrogen or C₁-C₆alkyl.

10. A composition according to claim 1 or claim 2, containing as active ingredient a compound of formula I, wherein X is oxygen or sulfur ; R₁ is C₁-C₆alkyl, C₁-C₆alkoxy, C₃-C₆cycloalkyl or allyl ; R₂ is C₁-C₆alkyl or allyl ; R₄ and Rₛ are each independently of the other hydrogen, C₁-C₆alkyl, C₁-C₆alkoxy or C₁-C₆haloalkoxy ; and R₃ a radical selected from the series consisting of 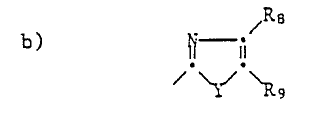 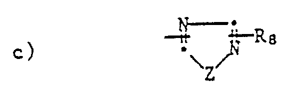 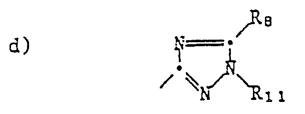 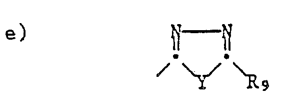 and 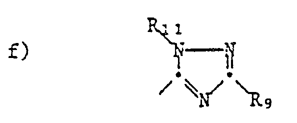 R₈ and Rg are each independently of the other hydrogen, C₁-C₆alkyl, C₁-C₆haloalkyl, C₁-C₆alkoxy, C₁-C₆alkylthio, halogen or nitro or are each independently of the other C₃-C₇cycloalkyl that is unsubstituted or substituted by halogen or by C₁-C₃alkyl ; Y is oxygen, sulfur or NR₁₀ ; Z is oxygen, sulfur or NH, R₁₀ is hydrogen or C₁-C₆alkyl; and R₁₁ is C₁-C₆alkyl.

11. A composition according to claim 3, containing as active ingredient a compound of formula I, wherein R₃ is the radical 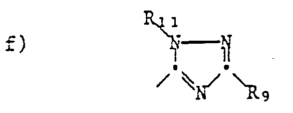 wherein Rg is hydrogen, C₁-C₆alkyl, C₁-C₆haloalkyl, C₁-C₆alkoxy, C₁-C₆alkylthio, halogen or nitro ; and R₁₁ is C₁-C₆alkyl.

12. A composition according to claim 3, containing as active ingredient a compound of formula wherein X is oxygen or sulfur ; R₁ is C₁-C₆alkyl, C₁-C₆alkoxy, C₃-C₆cycloalkyl or allyl ; R₂ is C₁-C₆alkyl or allyl ; R₄ and R₅ are each independently of the other hydrogen, C₁-C₆alkyl, C₁-C₆alkoxy or C₁-C₆haloalkoxy ; and R₃ is the radical 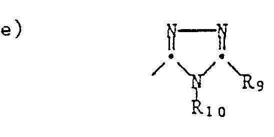 wherein Rg is hydrogen, C₁-C₆alkyl, C₁-C₆haloalkyl, C₁-C₆alkoxy, C₁-C₆alkylthio, halogen or nitro ; and R₁₀ is hydrogen or C₁-C₆alkyl.

13. A composition according to any one of claims 3 to 12, containing a compound of formula I, wherein X is oxygen or sulfur ; R₁ is methyl or methoxy ; R₂ is methyl ; R₄ and R₅ are each independently of the other hydrogen, methyl, CFₛ, OCH₃, OCHF₂ or OCF₃ ; and the remaining substituents are as defined in the relevant claim.

14. A composition according to claim 5, containing as active ingredient a compound of formula I, wherein R₆ and R₇ are each independently of the other hydrogen, methyl, CF₃, methoxy, halomethoxy, fluorine, chlorine or bromine and the remaining substituents are as defined in any one of claims 1 to 12.

15. A composition according to any one of claims 6 to 9 and 11 to 13, containing as active ingredient a compound of formula I, wherein R_{B} and Rg are each independently of the other hydrogen, methyl, ethyl, C₃-C₇cycloalkyl, CFₛ, C₂F₅, C₃F₇, CCl₃, CHCI₂, CH₂CI, SCH₃ or halogen ; R₁₀ is hydrogen or methyl ; R₁₁ is C₁-C₄alkyl ; and the remaining substituents are as defined in claims 5 to 8.

16. A composition according to claim 1 or claim 2 containing as active ingredient a compound of formula I selected from the series consisting of
1,3-dimethyl-5-[4-(6-bromobenzothiazol-2-yloxy)phenylcarbamoyl]barbituric acid ;
1,3-dimethyl-5-[4-(6-chlorobenzothiazol-2-yloxy)phenylcarbamoyl]barbituric acid ;
1,3-dimethyl-5-[4-(6-fluoro(benzothiazol-2-yloxy)phenylcarbomoyl]barbituric acid : 1,3-dimethyl-5-[4-(3-dichloromethyl-1,2,4-thiadiazol-5-yloxy)phenylcarbamoyl]barbituric acid ;
1,3-dimethyl-5-[4-(5-tert.-butyl-1,3,4-oxadiazol-2-yloxy)phenylcarbamoyl]barbituric acid ;
1,3-dimethyl-5-[4-(5-tert.-butyl-1,3,4-thiadiazol-2-yloxy)phenylcarbamoyl]barbituric acid ;
1-methyl-3-methoxy-5-[4-(5-tert.-butyl-1,3,4-thiadiazol-2-yloxy)phenylcarbamoyl]barbituric acid;
1,3-dimethyl-5-[4-(5-isopropyl-1,3,4-oxadiazol-2-yloxy)phenylcarbamoyl]barbituric acid ;
1,3-dimethyl-5-[4-(1-isopropyl-3-trifluoromethyl-1H-1,2,4-triazol-5-yloxy)phenylcarbamoyl]barbituric acid ;
1,3-dimethyl-5-[2,6-dimethyl-4-(1-methyl-3-heptafluoropropyl-1 H-1,2,4-triazol-5-yloxy)phenylcarbamoyl]barbituric acid;
1,3-dimethyl-5-[4-(1-isopropyl-3-pentafluoroethyl-1H-1,2,4-triazol-5-yloxy)phenylcarbamoyl]barbituric acid ;
1,3-dimethyl-5-[4-(1-isopropyl-3-trifluoromethyl-1H-1,2,4-triazol-5-yloxy)-2,6-dimethylphenylcar-
bamoyl]barbituric acid;
1,3-dimethyl-5-[3-(1-methyl-3-trifluoromethyl-1H,1,2,4-triazol-5-yloxy)phenylcarbamoyl]barbituric acid ;
1,3-dimethyl-5-[4-(6-trifluoromethylbenzothiazol-2-yloxy)phenylcarbamoyl]barbituric acid ;
1,3-dimethyl-5-[3-methoxy-4-(5-tert.-butyl-1,3,4-thiadiazol-2-yloxy)phenylcarbamoyl]barbituric acid;
1,3-dimethyl-5-[3-methoxy-4-(6-chlorobenzothiazol-2-yloxy)phenylcarbamoyl]barbituric acid ;
1,3-dimethyl-5-[4-(5,6-dichlorobenzothiazol-2-yloxy)phenylcarbamoyl]barbituric acid;
1,3-dimethyl-5-[4-(6,7-dichlorobenzothiazol-2-yloxy)phenylcarbamoyl]barbituric acid;
1,3-dimethyl-5-[4-(benzothiazol-2-yloxy)phenylcarbamoyl]barbituric acid ;
1,3-dimethyl-5-[4-(5-chloro-6-fluorobenzothiazol-2-yloxy)phenylcarbamoyl]barbituric acid;
1,3-dimethyl-5-[4-(7-chloro-6-fluorobenzothiazol-2-yloxy)phenylcarbamoyl]barbituric acid ;
1,3-dimethyl-5-[4-(6-trifluoromethoxybenzothiazol-2-yloxy)phenylcarbamoyl]barbituric acid ;
1,3-dimethyl-5-[2-isopropyl-4-(5-cyclopropyl-1,3,4-oxadiazol-2-yloxy)phenylcarbamoyl]barbituric acid ;
1,3-dimethyl-5-[4-(5-cyclohexyl-1,3,4-thiadiazol-2-yloxy)phenylcarbamoyl]barbituric acid;
1,3-dimethyl-5-[4-(1-methyl-3-pentafluoroethyl-1H,1,2,4-triazol-5-yloxy)phenylcarbamoyl]barbituric acid ;
1,3-dimethyl-5-[4-(6-methoxybenzothiazol-2-yloxy)phenylcarbamoyl]barbituric acid ;
1,3-dimethyl-5-[4-(5-cyclopropyl-1,3,4-thiadiazol-2-yloxy)phenylcarbamoyl]barbituric acid;
1,3-dimethyl-5-[4-(6-chloro-7-fluorobenzothiazol-2-yloxy)phenylcarbamoyl]barbituric acid;
1-methyl-3-methoxy-5-[4-(6-chloro-7-fluorobenzothiazol-2-yloxy)phenylcarbamoyl]barbituric acid and
1,3-dimethyl-5-[3-methoxy-4-(6-chloro-7-fluorobenzothiazol-2-yloxy)phenylcarbamoyl]barbituric acid.

17. A process for the preparation of a compound of formula I according to any one of claims 1 to 16, which comprises
a) reacting an ester of formula II 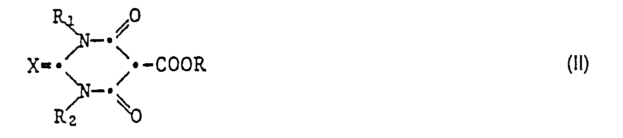 at temperatures of from 50° to 250 °C with an aniline derivative of formula II 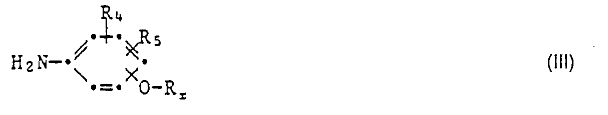 wherein R is a lower alkyl group, or a phenyl group which is unsubstituted or substituted by nitro, or
b) reacting a substituted barbituric acid of formula IV 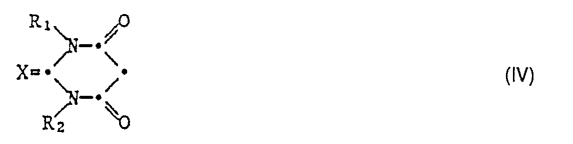 at temperatures of from 0° to 220 °C with a substituted phenylisocyanate of formula V 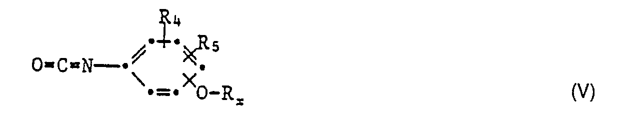 or
c) reacting a substituted barbituric acid of formula IV at temperatures of from 50° to 250 °C with a substituted benzoylazide of formula VI 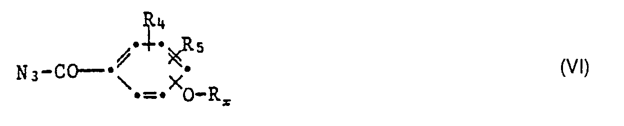 in which formulae II to VI the substituents X, R₁, R₂, R₄ and R₅ are as defined for formula I and Rₓ is hydrogen or a radical as defined for R₃, and, in those cases in which Rₓ is hydrogen either, at the starting material stage, etherifying a hydroxy derivative (Rₓ = H) of formula III, V or VI at temperatures of from 50° to 150 °C with a compound of formula XX 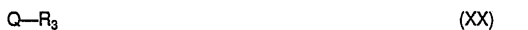 wherein Q is a customary leaving group, and then allowing the etherified product to react further to give the final product, or first reacting a hydroxy derivative of formula III, V or VI with a compound II or IV to give a hydroxy derivative (Rₓ = H) of formula I', wherein X, R₁, R₂, R₄ and Rₛ are as defined for formula I and Rₓ is hydrogen, and then etherifying the hydroxy derivative of formula I' with a compound of formula XX, the reactions (a), (b) and (c) being carried out at normal or elevated pressure in the presence or absence of an inert solvent or diluent.

18. A process according to claim 17, which comprises carrying out processes (a), (b) and (c) in the presence of an inert solvent or diluent.

19. A process according to claims 17 and 18, which comprises carrying out process variants (a) and (c) at temperatures of from 70° to 220 °C, process variant (b) at temperatures of from 0° to 200°C and the etherification at temperatures of from 80° to 120°C.

20. A process according to claims 17 to 19, which comprises carrying out processes (a), (b) and (c) in the presence of a base.

21. A compound of formula I according to any one of claims 1 to 16 for use in a method of controlling parasitic helminths in productive livestock.

22. A composition according to any one of claims 1 to 16 for use in a method of controlling parasitic helminths in productive livestock.

23. Use of a compound of formula I according to any one of claims 1 to 16 for the preparation of anthelmintics.

## Revendications (Revendications pour l'(les) Etat(s) contractant(s) suivant(s) : DE, FR, CH, LI, IT, NL, BE, SE, LU)

1. Composés de formule I 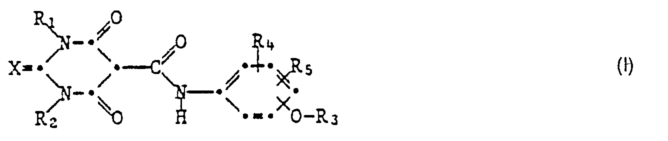 et leurs formes tautomères et sels, dans lesquels
X représente l'oxygène et le soufre ;
R₁ représente un groupe alkyle en C₁-C₆, alcoxy en C₁-C₆, cycloalkyle en C₃-C₆ ou allyle ;
R₂ représente un groupe alkyle en C₁-C₆ ou allyle ;
R₃ représente un azole hétéroaromatique à 5 chaînons, relié par l'intermédiaire du carbone, substitué ou non, et choisi dans la classe formée par le benzimidazole, le benzoxazole, le benzothiazole, l'imidazole, l'oxazole, le thiazole, l'oxadiazole, le thiadiazole et le triazole ; et
R₄ et R₅ représentent chacun, indépendamment l'un de l'autre, l'hydrogène, un groupe alkyle en C₁-C₆, halogénoalkyle en C₁-C₆, alcoxy en C₁-C₆ ou halogénoalcoxy en C₁-C₆.

2. Composés de formule I selon la revendication 1, dans lesquels X représente l'oxygène ou le soufre ; Rᵢ représente un groupe alkyle en C₁-C₆, alcoxy en C₁-C₆, cycloalkyle en C₃-C₆ ou allyle ; R₂ représente un groupe alkyle en C₁-C₆ ou allyle ; R₄ et Rₛ représentent chacun, indépendamment l'un de l'autre, l'hydrogène, un groupe alkyle en C₁-C₆, alcoxy en Ci-C₆ ou halogénoalcoxy en C₁-C₆; R₃ représente un groupe choisi parmi les suivants : 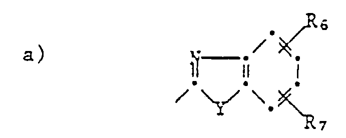 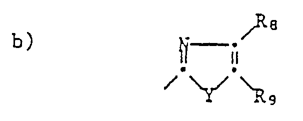 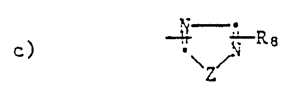 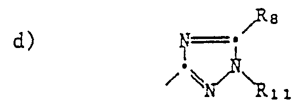 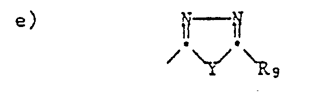 et 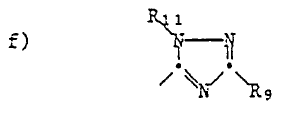 dans lesquels
Y représente l'oxygène, le soufre ou NR₁₀ ;
Z représente l'oxygène, le soufre ou NH ;
R₆ et R₇ représentent chacun, indépendamment l'un de l'autre, l'hydrogène, un groupe alkyle en C₁-C₄, halogénoalkyle en C,-C₄, alcoxy en C₁-C₄, halogénoalcoxy en Ci-C₄, alkylthio en C₁-C₄, un halogène, un groupe nitro ou cyano ;
R₈ et Rg représentent chacun, indépendamment l'un de l'autre, l'hydrogène, un groupe alkyle en C₁-C₆, halogénoalkyle en C₁-C₆, alcoxy en C₁-C₆, alkylthio en C₁-C₆, un halogène, un groupe nitro ou bien, indépendamment l'un de l'autre, un groupe cycloalkyle en C₃-C₇ non substitué ou substitué par des halogènes ou des groupes alkyle en C₁-C₃;
R₁₀ représente l'hydrogène ou un groupe alkyle en C₁-C₆, et
R₁₁ représente un groupe alkyle en C₁-C₆.

3. Composés de formule 1 selon la revendication 2, dans lesquels X représente l'oxygène ou le soufre ; R₁ représente un groupe alkyle en C₁-C₆, alcoxy en C₁-C₆, cycloalkyle en C₃-C₆ ou allyle ; R₂ représente un groupe alkyle en C₁-C₆ ou allyle, R₄ et R₅ représentent chacun, indépendamment l'un de l'autre, l'hydrogène, un groupe alkyle en C₁-C₆, alcoxy en C₁-C₆ ou halogénoalcoxy en C₁-C₆; R₃ représente un groupe choisi parmi les suivants : 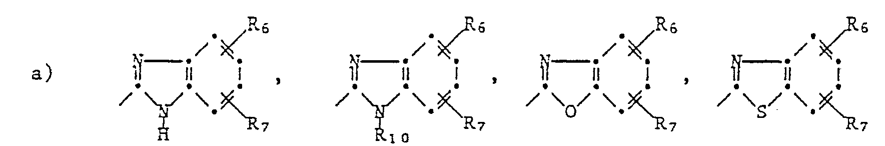 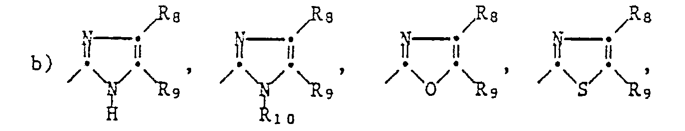 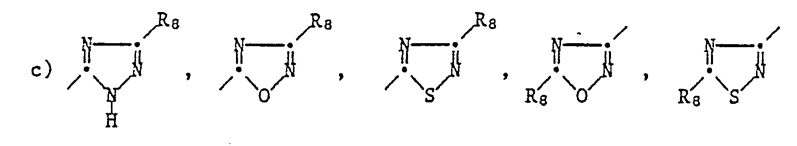 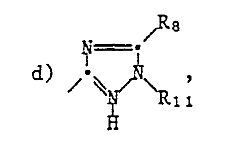 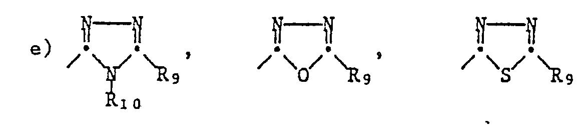 et 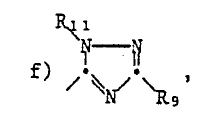 dans lesquels
R₆ et R₇ représentent chacun, indépendamment l'un de l'autre, l'hydrogène, un groupe alkyle en C₁-C₄ halogénoalkyle en C₁-C₄, alcoxy en C₁-C₄ halogénoalcoxy en C₁-C₄, alkylthio en C₁-C₄, un halogène, un groupe nitro ou cyano ;
R₈ et Rg représentent chacun, indépendamment l'un de l'autre, l'hydrogène, un groupe alkyle en C₁-C₆, halogénoalkyle en Ci-Ce, alcoxy C₁-C₆, alkylthio en C₁-C₆, un halogène ou un groupe nitro ;
R₁₀ représente l'hydrogène ou un groupe alkyle en C₁-C₆, et
R₁₁ représente un groupe alkyle en C₁-C₆.

4. Composés de formule 1 selon la revendication 2, dans lesquels X représente l'oxygène ou le soufre ; R₁ représente un groupe alkyle en C₁-C₆, alcoxy en C₁-C₆, cycloalkyle en C₃-C₆ ou allyle ; R₂ représente un groupe alkyle en C₁-C₆ ou allyle, R₄ et R₅ représentent chacun, indépendamment l'un de l'autre, l'hydrogène, un groupe alkyle en C₁-C₆, alcoxy en C₁-C₆ ou halogénoalcoxy en C₁-C₆; R₃ représente le groupe 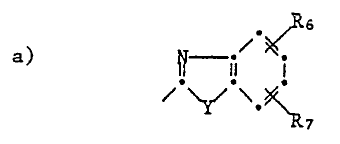 dans lequel R₆ et R₇ représentent chacun, indépendamment l'un de l'autre, l'hydrogène, un groupe alkyle en C₁-C₄, halogénoalkyle en C₁-C₄, alcoxy en C₁-C₄, halogénoalcoxy en C₁-C₄, alkylthio en C₁-C₄, un halogène, un groupe nitro ou cyano ; et Y représente l'oxygène, le soufre ou NR₁₀ dans lequel R₁₀ représente l'hydrogène ou un groupe alkyle en C₁-C₆.

5. Composés de formule 1 selon la revendication 2, dans lesquels X représente l'oxygène ou le soufre ; R₁ représente un groupe alkyle en C₁-C₆, alcoxy en C₁-C₆, cycloalkyle en C₃-C₆ ou allyle ; R₂ représente un groupe alkyle en C₁-C₆ ou allyle ; R₄ et R₅ représentent chacun, indépendamment l'un de l'autre, l'hydrogène, un groupe alkyle en C₁-C₆, alcoxy en C₁-C₆ ou halogénoalcoxy en C₁-C₆; R₃ représente le groupe 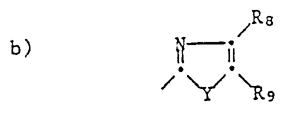 dans lequel R₈ et Rg représentent chacun, indépendamment l'un de l'autre, l'hydrogène, un groupe alkyle en C₁-C₆, halogénoalkyle en C₁-C₆, alcoxy en C₁-C₆, alkylthio en C₁-C₆, un halogène ou un groupe nitro ; et Y représente l'oxygène ou le soufre ou NR₁₀ dans lequel R₁₀ représente l'hydrogène ou un groupe alkyle en C₁-C₆.

6. Composés de formule I selon la revendication 2, dans lesquels X représente l'oxygène ou le soufre ; R₁ représente un groupe alkyle en C₁-C₆, alcoxy en C₁-C₆, cycloalkyle en C₃-C₆ ou allyle ; R₂ représente un groupe alkyle en C₁-C₆ ou allyle ; R₄ et R₅ représentent chacun, indépendamment l'un de l'autre, l'hydrogène, un groupe alkyle en C₁-C₆, alcoxy en C₁-C₆ ou halogénoalcoxy en C₁-C₆; R₃ représente le groupe 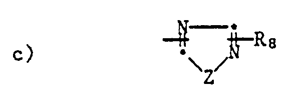 dans lequel R_{B} représente l'hydrogène, un groupe alkyle en C₁-C₆, halogénoalkyle en C₁-C₆, alcoxy en C₁-C₆, alkylthio en C₁-C₆, un halogène ou un groupe nitro ; et Z représente l'oxygène, le soufre ou NH.

7. Composés de formule 1 selon la revendication 2, dans lesquels X représente l'oxygène ou le soufre ; R₁ représente un groupe alkyle en C₁-C₆, alcoxy en C₁-C₆, cycloalkyle en C₃-C₆ ou allyle ; R₂ représente un groupe alkyle en C₁-C₆ ou allyle ; R₄ et R₅ représentent chacun, indépendamment l'un de l'autre, l'hydrogène, un groupe alkyle en C₁-C₆, alcoxy en C₁-C₆ ou halogénoalcoxy en C₁-C₆; R₃ représente le groupe 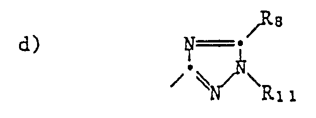 dans lequel R₈ représente l'hydrogène, un groupe alkyle en C₁-C₆, halogénoalkyle en C₁-C₆, alcoxy en C₁-C₆, alkylthio en C₁-C₆, un halogène ou un groupe nitro ; et R₁₁ représente un groupe alkyle en C₁-C₆.

8. Composés de formule 1 selon la revendication 2, dans lesquels X représente l'oxygène ou le soufre ; R₁ représente un groupe alkyle en C₁-C₆, alcoxy en C₁-C₆, cycloalkyle en C₃-C₆ ou allyle ; R₂ représente un groupe alkyle en C₁-C₆ ou allyle ; R₄ et R₅ représentent chacun, indépendamment l'un de l'autre, l'hydrogène, un groupe alkyle en C₁-C₆, alcoxy en C₁-C₆, ou halogénoalcoxy en C₁-C₆ ; R₃ représente le groupe 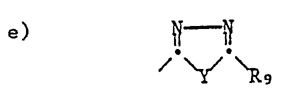 dans lequel Rg représente l'hydrogène, un groupe alkyle en C₁-C₆, halogénoalkyle en C₁-C₆, alcoxy en C₁-C₆, alkylthio en C₁-C₆, un halogène ou un groupe nitro ; Y représente l'oxygène, le soufre ou NR₁₀ ; et R₁₀ représente l'hydrogène ou un groupe alkyle en C₁-C₆.

9. Composés de formule 1 selon la revendication 1, caractérisés en ce que X représente l'oxygène ou le soufre ; R₁ représente un groupe alkyle en C₁-C₆, alcoxy en C₁-C₆, cycloalkyle en C₃-C₆ ou allyle ; R₂ représente un groupe alkyle en C₁-C₆ ou allyle ; R₄ et R₅ représentent chacun, indépendamment l'un de l'autre, l'hydrogène, un groupe alkyle en C₁-C₆, alcoxy en C₁-C₆ ou halogénoalcoxy en C₁-C₆ ; R₃ représente un groupe choisi parmi les suivants : 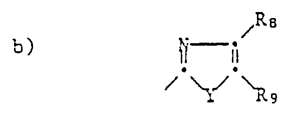 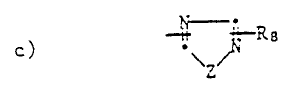 (Suite) 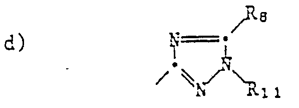 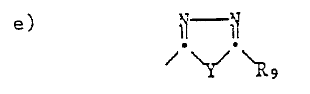 et 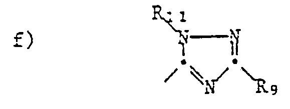 R₈ et Rg représente chacun, indépendamment l'un de l'autre, l'hydrogène, un groupe alkyle en C₁-C₆, halogénoalkyle en C₁-C₆, alcoxy en C₁-C₆, alkylthio en C₁-C₆, un halogène, un groupe nitro ou bien, indépendamment l'un de l'autre, un groupe cycloalkyle en C₃-C₇ non substitué ou substitué par des halogènes ou des groupes alkyle en C₁-C₃ ; Y représente l'oxygène, le soufre ou NR₁₀ ; Z représente l'oxygène, le soufre ou NH ; R₁₀ représente l'hydrogène ou un groupe alkyle en C₁-C₆; R₁₁ représente un groupe alkyle en C₁-C₆.

10. Composés de formule 1 selon la revendication 2, dans lesquels X représente l'oxygène ou le soufre ; R₁ représente un groupe alkyle en C₁-C₆, alcoxy en C₁-C₆, cycloalkyle en C₃-C₆ ou allyle ; R₂ représente un groupe alkyle en C₁-C₆ ou allyle ; R₄ et R₅ représentent chacun, indépendamment l'un de l'autre, l'hydrogène, un groupe alkyle en C₁-C₆, alcoxy en C₁-C₆ ou halogénoalcoxy en C₁-C₆; R₃ représente le groupe 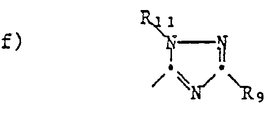 dans lequel Rg représente l'hydrogène, un groupe alkyle en C₁-C₆, halogénoalkyle en C₁-C₆, alcoxy en C₁-C₆, alkylthio en C₁-C₆, un halogène ou un groupe nitro ; et R₁₁ représente un groupe alkyle en C₁-C₆.

11. Composés de formule I selon la revendication 2, dans lesquels R₃ représente le groupe 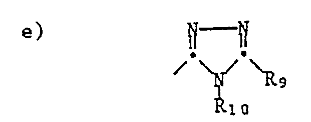 dans lequel Rg représente l'hydrogène, un groupe alkyle en C₁-C₆, halogénoalkyle en C₁-C₆, alcoxy en C₁-C₆, alkylthio en C₁-C₆, un halogène ou un groupe nitro ; et R₁₀ représente l'hydrogène ou un groupe alkyle en C₁-Cₛ.

12. Composés de formule 1 selon l'une des revendications 1 à 11, dans lesquels X représente l'oxygène ou le soufre, R₁ un groupe méthyle ou méthoxy ; R₂ un groupe méthyle ; R₄ et R₅, indépendamment l'un de l'autre l'hydrogène, un groupe méthyle, CF₃, OCH₃, OCHF₂ ou OCF₃; et les autres symboles ont les significations indiquées dans la revendication correspondante.

13. Composés de formule I selon la revendication 4, dans lesquels R₆ et R₇ représentent chacun, indépendamment l'un de l'autre, l'hydrogène, un groupe méthyle, CF₃, méthoxy, halogénométhoxy, le fluor, le chlore ou le brome et les autres symboles ont les significations indiquées dans l'une des revendications 1 à 12.

14. Composés de formule I selon l'une des revendications 5 à 8 et 10 à 11, dans lesquels R_{B} et Rg représentent chacun, indépendamment l'un de l'autre, l'hydrogène, un groupe méthyle, éthyle, cycloalkyle en C₃-C₇, CF₃, C₂F₅, C₃F₇, CCI₃, CHCI₂, CH₂CI, SCH₃ ou un halogène ; R₁₀ représente l'hydrogène ou un groupe méthyle ; R₁₁ représente un groupe alkyle en C₁-C₄ et les autres symboles ont les significations indiquées dans les revendications 5 à 8.

15. Un composé de formule I selon l'une des revendications 1 à 3, choisi parmi les suivants :
acide 1,3-diméthyl-5-[4-(6-bromo-benzothiazole-2-yloxy)-phénylcarbamoyi]-barbiturique,
acide 1,3-diméthyl-5-[4-(6-chloro-benzothiazole-2-yloxy)-phénylcarbamoyl]-barbiturique,
acide 1,3-diméthyl-5-[4-(6-fluoro-benzothiazole-2-yloxy)-phénylcarbamoyl]-barbiturique,
acide 1,3-diméthyl-5-[4-(3-dichlorométhyl-1,2,4-thiadiazole-5-yloxy)-phénylcarbamoyl]-barbiturique,
acide 1,3-diméthyl-5-[4-(5-tert-butyl-1,3,4-oxadiazole-2-yloxy)-phénylcarbamoyl]-barbiturique,
acide 1,3-diméthyl-5-[4-(5-tert-butyl-1,3,4-thiadiazole-2-yloxy)-phénylcarbamoyl]-barbiturique,
acide 1-méthyl-3-méthoxy-5-[4-(5-tert-butyl-1,3,4-thiadiazole-2-yloxy)-phénylcarbamoyl]-barbiturique,
acide 1,3-diméthyl-5-[4-(5-isopropyl-1,3,4-oxadiazole-2-yloxy)-phénylcarbamoyl]-barbiturique,
acide 1,3-diméthyl-5-[4-(1-isopropyl-3-trifluorométhy)-1H-1,2,4-triazole-5-yloxy)-phénylcarbamoyl]-barbiturique,
acide 1,3-diméthyl-5-[2,6-diméthyl-4-(1-méthyl-3-heptafluoropropyl-1H-1,2,4-triazole-5-yloxy)-phénylcarba- moyl]-barbiturique,
acide 1,3-diméthyl-5-[4-(1-isopropyl-3-pentafluoréthyl-1H-1,2,4-triazole-5-yloxy)-phénylcarbamoyl]-barbiturique,
acide 1,3-diméthyl-5-[4-(1-isopropyl-3-trifluorométhyl-1H-1,2,4-triazole-5-yloxy)-2,6-diméthyl-phénylcarba- moylj-barbiturique,
acide 1,3-diméthyl-5-[3-(1-méthyl-3-trifluorométhyl-1H-1,2,4-triazole-5-yloxy)-phénylcarbamoyl]-barbiturique,
acide 1,3-diméthyl-5-[4-(6-trifluorométhyl-benzothiazole-2-yloxy)-phénylcarbamoyl]-barbiturique,
acide 1,3-diméthyl-5-[3-méthoxy-4-(5-tert-butyl-1,3,4-thiadiazole-2-yloxy)-phénylcarbamoyl]-barbiturique, acide 1,3-diméthyl-5-[3-méthoxy-4-(6-chloro-benzothiazole-2-yloxy)-phénylcarbamoyl]-barbiturique,
acide 1,3-diméthyl-5-[4-(5,6-dichloro-benzothiazole-2-yloxy)-phénylcarbamoyl]-barbiturique,
acide 1,3-diméthyl-5-[4-(6,7-dichloro-benzothiazole-2-yloxy)-phénylcarbamoyl]-barbiturique,
acide 1,3-diméthyl-5-[4-(benzothiazole-2-yloxy)-phénylcarbamoyl]-barbiturique,
acide 1,3-diméthyl-5-[4-(5-chloro-6-fluoro-benzothiazole-2-yloxy)-phénylcarbamoyl]-barbiturique,
acide 1,3-diméthyl-5-[4-(7-chloro-6-fluoro-benzothiazole-2-yloxy)-phénylcarbamoyl]-barbiturique,
acide 1,3-diméthyl-5-[4-(6-trifluorométhoxy-benzothiazole-2-yloxy)-phénylcarbamoyl]-barbiturique,
acide 1,3-diméthyl-5-[2-isopropyl-4-(5-cyclopropyl-1,3,4-oxadiazole-2-yloxy)-phénylcarbamoyl]-barbiturique,
acide 1,3-diméthyl-5-[4-(5-cyclohexyl-1,3,4-thiadiazole-2-yloxy)-phénylcarbamoyl]-barbiturique,
acide 1,3-diméthyl-5-[4-(1-méthyl-3-pentafluoréthyl-1H-1,2,4-triazole-5-yloxy)-phénylcarbamoyl]-barbiturique,
acide 1,3-diméthyl-5-[4-(6-méthoxy-benzothiazole-2-yloxy)-phénylcarbamoyl]-barbiturique,
acide 1,3-diméthyl-5-[4-(5-cyclopropyl-1,3,4-thiadiazole-2-yloxy)-phénylcarbamoyl]-barbiturique,
acide 1,3-diméthyl-5-[4-(6-chloro-7-fluoro-benzothiazole-2-yloxy)-phénylcarbamoyl]-barbiturique,
acide 1-méthyl-3-méthoxy-5-[4-(6-chloro-7-fluoro-benzothiazole-2-yloxy)-phénylcarbamoyl]-barbiturique, et
acide 1,3-diméthyl-5-[3-méthoxy-4-(6-chloro-7-fluoro-benzothiazole-2-yloxy)-phénylcarbamoyl]-barbiturique.

16. Procédé de préparation d'un composé de formule 1 selon l'une des revendications 1 à 15, caractérisé en ce que :
a) on fait réagir à des températures de 50 à 250 °C un ester de formule Il 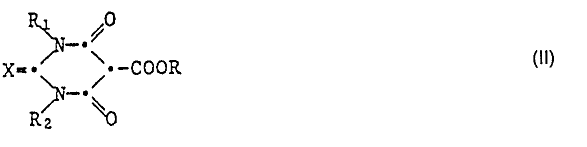 avec un dérivé de l'aniline de formule III 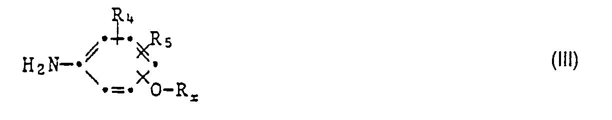 R représentant un groupe alkyle inférieur ou un groupe phényl éventuellement substitué par un groupe nitro, ou bien
b) on fait réagir à des températures de 0 à 220 °C un acide barbiturique substitué de formule IV 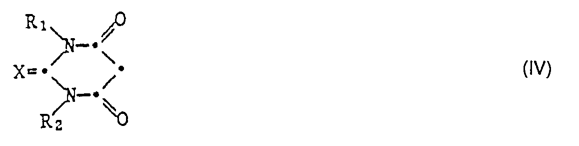 avec un isocyanate de phényle substitué de formule V 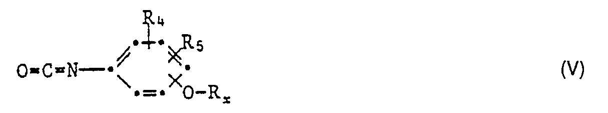 ou bien
c) on fait réagir à des températures de 50 à 250 °C un acide barbiturique substitué de formule IV avec un benzoylazide substitué de formule VI 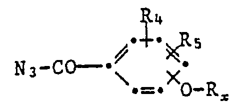 les symboles X, R₁, R₂, R₄ et R₅ ayant dans les formules II à VI les significations indiquées en référence à la formule I et Rₓ représentant l'hydrogène ou l'un des substituants mentionnés en référence à R₃, et dans les cas où Rₓ représente l'hydrogène, soit qu'au stade des produits de départ, on éthérifie à des températures de 50 à 150 °C l'un des dérivés hydroxylés (Rₓ = H) de formules III, V ou VI par un composé de formule XX 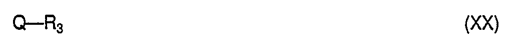 dans laquelle Q représente une fonction éliminable usuelle puis on poursuit les réactions jusqu'au produit final, soit qu'on convertisse d'abord les dérivés hydroxylés de formules III, V ou VI, au moyen de l'un des composés Il ou IV, en un dérivé hydroxylé (Rₓ = H) de formule l', X, R₁, R₂, R₄ et Rₛ ayant les significations indiquées en référence à la formule I et Rₓ représentant l'hydrogène, après quoi, seulement, on éthérifie le dérivé hydroxylé de formule l' par le composé de formule XX, les réactions a), b) et c) étant effectuées à pression normale ou sous pression et en l'absence ou en présence de solvants ou diluants inertes dans les réactions.

17. Procédé selon la revendication 16, caractérisé en ce que les procédés a), b) et c) sont mis en oeuvre en présence de solvants ou diluants inertes dans les réactions.

18. Procédé selon les revendications 16 et 17, caractérisé en ce que les variantes opératoires a) et c) sont mises en oeuvre à des températures de 70 à 220 °C, la variante opératoire b) à des températures de 0 à 200 °C et l'éthérification à des températures de 80 à 120 °C.

19. Procédé selon les revendications 16 à 18, caractérisé en ce que les procédés a), b) et c) sont mis en oeuvre en présence d'une base.

20. Agent anti-helminthique caractérisé en ce qu'il contient en tant que composant actif, avec des produits auxiliaires de formulation usuels, au moins un composé de formule 1 selon l'une des revendications 1 à 15.

21. Produit selon la revendication 20, caractérisé en ce qu'il contient de 0,1 à 99,0 % en poids de substance active et de 99,9 à 1 % en poids de véhicules et autres produits auxiliaires.

22. Un composé selon l'une des revendications 1 à 15, pour l'utilisation dans un procédé pour combattre les helminthes parasitaires chez les animaux utiles.

23. Produit selon l'une des revendications 20 ou 21 pour l'utilisation dans un procédé pour combattre les helminthes parasitaires chez les animaux utiles.

24. Utilisation d'un composé de formule 1 selon l'une des revendications 1 à 15, pour la préparation d'agents anti-helminthiques.

## Revendications (Revendications pour l'(les) Etat(s) contractant(s) suivant(s) : AT)

1. Agent anti-helminthique, caractérisé en ce qu'il contient en tant que composant actif, avec des produits auxiliaires de formulation usuels, au moins un composé de formule I 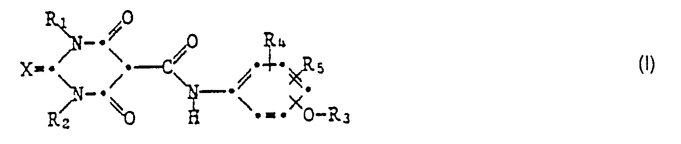 ou l'un de ses tautomères, le cas échéant à l'état de sel, dans lesquels
X représente l'oxygène ou le soufre, Rᵢ représente un groupe alkyle en C₁-C₆, alcoxy en C₁-C₆, cycloalkyle en C₃-C₆ ou allyle ;
R₂ représente un groupe alkyle en C₁-C₆ ou allyle ;
R₃ représente un azole hétéroaromatique à 5 chaînons relié par l'intermédiaire du carbone, non substitué ou substitué, choisi dans la classe formée par le benzimidazole, le benzoxazole, le benzothiazole, l'imidazole, l'oxazole, le thiazole, l'oxadiazole, le thiadiazole et le triazole ; et
R₄ et R₅ représentent chacun, indépendamment l'un de l'autre, l'hydrogène, un groupe alkyle en C₁-C₆, halogénoalkyle en C₁-C₆, alcoxy en C₁-C₆ ou halogénoalcoxy en C₁-C₆.

2. Agent selon la revendication 1, caractérisé en ce qu'il contient de 0,1 à 99,0 % en poids de substance active et de 99,9 à 1 % en poids de véhicules et autres produits auxiliaires.

3. Agent selon l'une des revendications 1 ou 2, caractérisé en ce qu'il contient en tant que substance active un composé de formule I dans laquelle X représente l'oxygène ou le soufre ; R₁ représente un groupe alkyle en C₁-C₆, alcoxy en C₁-C₆, cycloalkyle en C₃-C₆ ou allyle ; R₂ représente un groupe alkyle en C₁-C₆ ou allyle ; R₄ et R₅ représentent chacun, indépendamment l'un de l'autre, l'hydrogène, un groupe alkyle en C₁-C₆, alcoxy en C₁-C₆ ou halogénoalcoxy en C₁-C₆; R₃ représente un groupe choisi parmi les suivants : 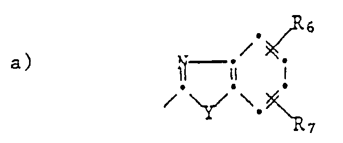 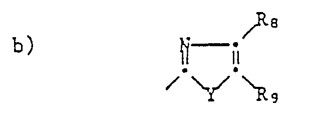 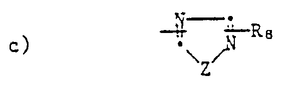 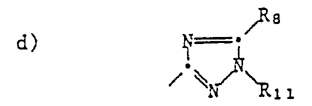 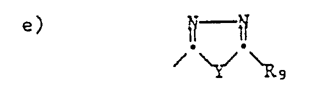 et 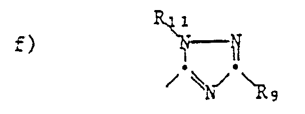 dans lesquels
Y représente l'oxygène, le soufre ou NR₁₀;
Z représente l'oxygène, le soufre ou NH ;
R₆ et R₇ représente chacun, indépendamment l'un de l'autre, l'hydrogène, un groupe alkyle en C₁-C₄, halogénoalkyle en C₁-C₄, alcoxy en C₁-C₄, halogénoalcoxy en C₁-C₄, alkylthio en C₁-C₄, un halogène, un groupe nitro ou cyano ;
R₈ et Rg représentent chacun, indépendamment l'un de l'autre, l'hydrogène, un groupe alkyle en C₁-C₆, halogénoalkyle en C₁-C₆, alcoxy en C₁-C₆, alkylthio en C₁-C₆, un halogène, un groupe nitro, ou bien, indépendamment l'un de l'autre, un groupe cycloalkyle en C₃-C₇ non substitué ou substitué par des halogènes ou des groupes alkyle en C₁-C₃;
R₁₀ représente l'hydrogène ou un groupe alkyle en C₁-C₆, et
R₁₁ représente un groupe alkyle en C₁-C₆.

4. Agent selon la revendication 3, contenant en tant que substance active, l'un des composés de formule I dans lesquels X représente l'oxygène ou le soufre ;
R₁ représente un groupe alkyle en C₁-C₆, alcoxy en C₁-C₆, cycloalkyle en C₃-Cₛ ou allyle ; R₂ représente un groupe alkyle en C₁-C₆ ou allyle ; R₄ et Rg représentent chacun, indépendamment l'un de l'autre, l'hydrogène, un groupe alkyle en C₁-C₆, alcoxy en C₁-C₆ ou halogénoalcoxy en C₁-C₆; R₃ représente un groupe choisi parmi les suivants : 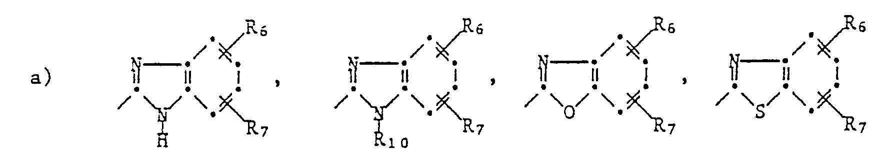 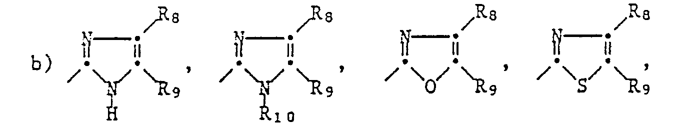 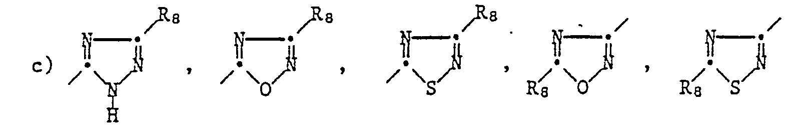 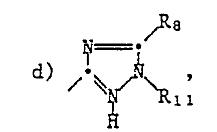 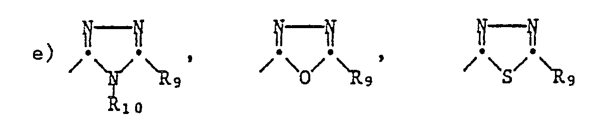 et 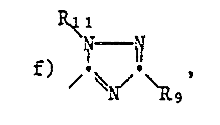 dans lesquels
R₆ et R₇ représentent chacun, indépendamment l'un de l'autre, l'hydrogène, un groupe alkyle en C₁-C₄, halogénoalkyle en C₁-C₄, alcoxy en C₁-C₄, halogénoalcoxy en C₁-C₄, alkylthio en C₁-C₄, un halogène, un groupe nitro ou cyano ;
R₈ et Rg représentent chacun, indépendamment l'un de l'autre, l'hydrogène, un groupe alkyle en C₁-C₆, halogénoalkyle en C₁-C₆, alcoxy en C₁-C₆, alkylthio en C₁-C₆, un halogène ou un groupe nitro ;
R₁₀ représente l'hydrogène ou un groupe alkyle en C₁-C₆, et
R₁₁ représente un groupe alkyle en C₁-C₆.

5. Agent selon la revendication 3, contenant en tant que substance active l'un des composés de formule 1 dans lesquels X représente l'oxygène ou le soufre ; R₁ représente un groupe alkyle en C₁-C₆, alcoxy en C₁-C₆, cycloalkyle en C₃-C₆ ou allyle ; R₂ représente un groupe alkyle en C₁-C₆ ou allyle, R₄ et R₅ représentent chacun, indépendamment l'un de l'autre, l'hydrogène, un groupe alkyle en C₁-C₆, alcoxy en C₁-C₆ ou halogénoalcoxy en C₁-C₆; R₃ représente le groupe 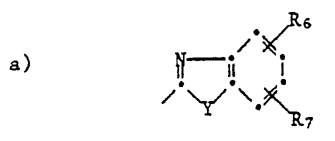 dans lequel R₆ et R₇ représentent chacun, indépendamment l'un de l'autre, l'hydrogène, un groupe alkyle en C₁-C₄, halogénoalkyle en C₁-C₄, alcoxy en C₁-C₄, halogénoalcoxy en C₁-C₄, alkylthio en C₁-C₄, un halogène, un groupe nitro ou cyano ; et Y représente l'oxygène, le soufre ou NR₁₀ dans lequel R₁₀ représente l'hydrogène ou un groupe alkyle en C₁-C₆.

6. Agent selon la revendication 3, contenant en tant que substance active l'un des composés de formule I dans lesquels X représente l'oxygène ou le soufre ; R₁ représente un groupe alkyle en C₁-C₆, alcoxy en C₁-C₆, cycloalkyle en C₃-Cₛ ou allyle ; R₂ représente un groupe alkyle en C₁-C₆ ou allyle ; R₄ et R₅ représentent chacun, indépendamment l'un de l'autre, l'hydrogène, un groupe alkyle en C₁-C₆, alcoxy en C₁-C₆ ou halogénoalcoxy en C₁-C₆; R₃ représente le groupe 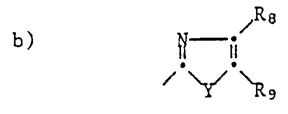 dans lequel R₈ et Rg représentent chacun, indépendamment l'un de l'autre, l'hydrogène, un groupe alkyle en C₁-C₆, halogénoalkyle en C₁-C₆, alcoxy en C₁-C₆, alkylthio en C₁-C₆, un halogène ou un groupe nitro ; et Y représente l'oxygène ou le soufre ou NR₁₀ dans lequel R₁₀ représente l'hydrogène ou un groupe alkyle en C₁-Cₛ.

7. Agent selon la revendication 3, contenant en tant que substance active l'un des composés de formule I dans lesquels X représente l'oxygène ou le soufre ; R₁ représente un groupe alkyle en C₁-C₆, alcoxy en Ci-Ce, cycloalkyle en C₃-C₆ ou allyle ; R₂ représente un groupe alkyle en C₁-C₆ ou allyle ; R₄ et R₅ représentent chacun, indépendamment l'un de l'autre, l'hydrogène, un groupe alkyle en Ci-Ce, alcoxy en Ci-Ce ou halogénoalcoxy en C₁-C₆ ; R₃ représente le groupe 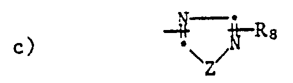 dans lequel R₈ représente |hydrogène, un groupe alkyle en C₁-C₆, halogénoalkyle en C₁-C₆, alcoxy en C₁-C₆, alkylthio en C₁-C₆, un halogène ou un groupe nitro ; et Z représente l'oxygène, le soufre ou NH.

8. Agent selon la revendication 3, contenant en tant que substance active l'un des composés de formule I dans lesquels X représente l'oxygène ou le soufre ; R₁ représente un groupe alkyle en C₁-C₆, alcoxy en Ci-Ce, cycloalkyle en C₃-C₆ ou allyle : R₂ représente un groupe alkyle en C₁-C₆ ou allyle ; R₄ et R₅ représentent chacun, indépendamment l'un de l'autre, l'hydrogène, un groupe alkyle en C₁-C₆, alcoxy en C₁-C₆ ou halogénoalcoxy en C₁-C₆ ; R₃ représente le groupe 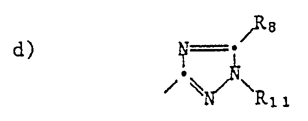 dans lequel R₈ représente l'hydrogène, un groupe alkyle en C₁-C₆, halogénoalkyle en C₁-C₆, alcoxy en C₁-C₆, alkylthio en C₁-C₆, un halogène ou un groupe nitro ; et R₁₁ représente un groupe alkyle en C₁-C₆.

9. Agent selon la revendication 3, contenant en tant que substance active l'un des composés de formule I dans lesquels X représente l'oxygène ou le soufre ; R₁ représente un groupe alkyle en C₁-C₆, alcoxy en C₁-C₆, cycloalkyle en C₃-C₆ ou allyle ; R₂ représente un groupe alkyle en C₁-C₆ ou allyle ; R₄ et R₅ représentent chacun, indépendamment l'un de l'autre, l'hydrogène, un groupe alkyle en C₁-C₆, alcoxy en C₁-C₆, ou halogénoalcoxy en C₁-C₆ ; R₃ représente le groupe 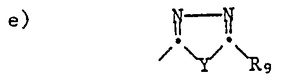 dans lequel Rg représente l'hydrogène, un groupe alkyle en C₁-C₆, halogénoalkyle en C₁-C₆, alcoxy en C₁-C₆, alkylthio en C₁-C₆, un halogène ou un groupe nitro ; Y représente l'oxygène, le soufre ou NR₁₀ ; et R₁₀ représente l'hydrogène ou un groupe alkyle en C₁-C₆.

10. Agent selon l'une des revendications 1 ou 2, contenant en tant que substance active des composés de formule I, et caractérisé en ce que X représente l'oxygène ou le soufre ; R₁ représente un groupe alkyle en C₁-Cₛ, alcoxy en C₁-C₆, cycloalkyle en C₃-Cₛ ou allyle ; R₂ représente un groupe alkyle en C₁-C₆ ou allyle ; R₄ et R₅ représentent chacun, indépendamment l'un de l'autre, l'hydrogène, un groupe alkyle en C₁-C₆, alcoxy en C₁-C₆ ou halogénoalcoxy en C₁-C₆ ; R₃ représente un groupe choisi parmi les suivants : 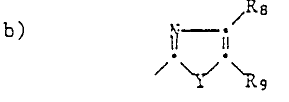 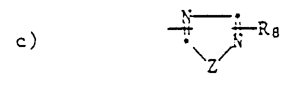 (Suite) 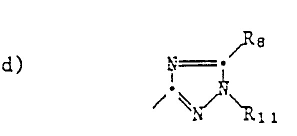 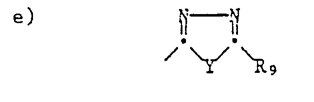 et 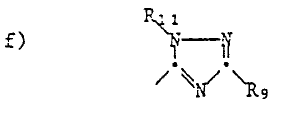 R_{B} et Rg représentent chacun, indépendamment l'un de l'autre, l'hydrogène, un groupe alkyle en C₁-C₆, halogénoalkyle en C₁-C₆, alcoxy en C₁-C₆, alkylthio en C₁-C₆, un halogène, un groupe nitro ou bien, indépendamment l'un de l'autre, un groupe cycloalkyle en C₃-C₇ non substitué ou substitué par des halogènes ou des groupes alkyle en C₁-C₃ ; Y représente l'oxygène, le soufre ou NR₁₀ ; Z représente l'oxygène, le soufre ou NH ; R₁₀ représente l'hydrogène ou un groupe alkyle en C₁-C₆ ; et R₁₁ représente un groupe alkyle en C₁-Cₛ.

11. Agent selon la revendication 3, contenant en tant que substance active l'un des composés de formule I dans lesquels R₃ représente le groupe 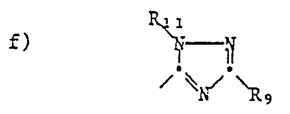 dans lequel Rg représente l'hydrogène, un groupe alkyle en C₁-C₆, halogénoalkyle en C₁-C₆, alcoxy en C₁-C₆, alkylthio en C₁-C₆, un halogène ou un groupe nitro ; et R₁₁ représente un groupe alkyle en C₁-C₆.

12. Agent selon la revendication 3, contenant en tant que substance active l'un des composés de formule 1 dans lesquels X représente l'oxygène ou le soufre ; R₁ représente un groupe alkyle en C₁-C₆, alcoxy en C₁-C₆, cycloalkyle en C₃-C₆ ou R₂ représente un groupe alkyle en C₁-C₆ ou allyle ; R₄ et R₅ représentent chacun, indépendamment l'un de l'autre, l'hydrogène, un groupe alkyle en C₁-C₆, alcoxy en C₁-C₆ ou halogénoalcoxy en C₁-C₆ ; Rₛ représente le groupe 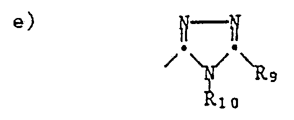 dans lequel Rg représente l'hydrogène, un groupe alkyle en C₁-C₆, halogénoalkyle en C₁-C₆, alcoxy en C₁-C₆, alkylthio en C₁-C₆, un halogène ou un groupe nitro ; et R₁₀ représente l'hydrogène ou un groupe alkyle en C₁-Cₛ.

13. Agent selon l'une des revendications 3 à 12, contenant l'un des composés de formule I dans lesquels X représente l'oxygène ou le soufre, R₁ représente un groupe méthyle ou méthoxy ; R₂ représente un groupe méthyle ; R₄ et R₅ représentent chacun, indépendamment l'un de l'autre, l'hydrogène, un groupe méthyle, CF₃, OCH₃, OCHF₂ ou OCF₃ ; et les autres symboles ont les significations indiquées dans la revendication correspondante.

14. Agent selon la revendication 5, contenant en tant que substance active l'un des composés de formule 1 dans lesquels R₆ et R₇ représentent chacun, indépendamment l'un de l'autre, l'hydrogène, un groupe méthyle, CF₃, méthoxy, halogénométhoxy, le fluor, le chlore ou le brome et les autres symboles ont les significations indiquées dans l'une des revendications 1 à 12.

15. Agent selon l'une des revendications 6 à 9 et 11 à 13, contenant en tant que substance active l'un des composés de formule I dans lesquels R₈ et Rg représentent chacun, indépendamment l'un de l'autre, l'hydrogène, un groupe méthyle, éthyle, cycloalkyle en C₃-C₇, CFₛ, C₂F₅, C₃F₇, OCI₃, CHCI₂, CH₂CI, SCH₃ ou un halogène ; R₁₀ représente l'hydrogène ou un groupe méthyle ; R₁₁ représente un groupe alkyle en C₁-C₄- et les autres symboles ont les significations indiquées dans les revendications 5 à 8.

16. Agent selon l'une des revendications 1 ou 2, contenant en tant que substance active un composé de formule I choisi parmi les suivants :
acide 1,3-diméthyl-5-[4-(6-bromo-benzothiazole-2-yloxy)-phénylcarbamoyl]-barbiturique,
acide 1,3-diméthyl-5-[4-(6-chloro-benzothiazole-2-yloxy)-phénylcarbamoyl]-barbiturique,
acide 1,3-diméthyl-5-[4-(6-fluoro-benzothiazole-2-yloxy)-phénylcarbamoyl]-barbiturique,
acide 1,3-diméthyl-5-[4-(3-dichlorométhyl-1,2,4-thiadiazole-5-yloxy)-phénylcarbamoyl]-barbiturique,
acide 1,3-diméthyl-5-[4-(5-tert-butyl-1,3,4-oxadiazole-2-yloxy)-phénylcarbamoyl]-barbiturique,
acide 1,3-diméthyl-5-[4-(5-tert-butyl-1,3,4-thiadiazole-2-yloxy)-phénylcarbamoyl]-barbiturique,
acide 1-méthyl-3-méthoxy-5-[4-(5-tert-butyl-1,3,4-thiadiazole-2-yloxy)-phénylcarbamoyl]-barbiturique,
acide 1,3-diméthyl-5-[4-(5-isopropyl-1,3,4-oxadiazole-2-yloxy)-phénylcarbamoylj-barbiturique,
acide 1,3-diméthyl-5-[4-(1-isopropyl-3-trifluorométhyl-1H-1,2,4-triazole-5-yloxy)-phénylcarbamoyl]-barbiturique,
acide 1,3-diméthyl-5-[2,6-diméthyl-4-(1-méthyl-3-heptafluoropropyl-1H-1,2,4-triazole-5-yloxy)-phénylcarba- moyl]-barbiturique,
acide 13-diméthyl-5-[4-(1-isopropyl-3-pentafluoréthyl-1H-1,2,4-triazole-5-yloxy)phénylcarbamoyl]-barbiturique,
acide 1,3-diméthyl-5-[4-(1-isopropyl-3-trifluorométhyl-1H-1,2,4-triazole-5-yloxy)-2,6-diméthyl-phénylcarba- moyl]-barbiturique,
acide 1,3-diméthyl-5-[3-(1-méthyl-3-trifluorométhyl-1H-1,2,4-triazole-5-yloxy)-phénylcarbamoyl]-barbiturique,
acide 1,3-diméthyl-5-[4-(6-trifluorométhyl-benzothiazole-2-yloxy)-phénylcarbamoyl]-barbiturique,
acide 1,3-diméthyl-5-[3-méthoxy-4-(5-tert-butyl-1,3,4-thiadiazole-2-yloxy)-phénylcarbamoyl]-barbiturique, acide 1,3-diméthyl-5-[3-méthoxy-4-(6-chloro-benzothiazole-2-yloxy)-phénylcarbamoyl]-barbiturique,
acide 1,3-diméthyl-5-[4-(5,6-dichloro-benzothiazole-2-yloxy)-phénylcarbamoyl]-barbiturique,
acide 1,3-diméthyl-5-[4-(6,7-dichloro-benzothiazole-2-yloxy)-phénylcarbamoylj-barbiturique,
acide 1,3-diméthyl-5-[4-(benzothiazole-2-yloxy)-phénylcarbamoyl]-barbiturique,
acide 1,3-diméthyl-5-[4-(5-chloro-6-fluoro-benzothiazole-2-yloxy)-phénylcarbamoyl]-barbiturique,
acide 1,3-diméthyl-5-[4-(7-chloro-6-fluoro-benzothiazole-2-yloxy)-phénylcarbamoyl]-barbiturique,
acide 1,3-diméthyl-5-[4-(6-trifluorométhoxy-benzothiazole-2-yloxy)-phénylcarbamoyl]-barbiturique,
acide 1,3-diméthyl-5-[2-isopropyl-4-(5-cyclopropyl-1,3,4-oxadiazole-2-yloxy)-phénylcarbamoyl]-barbiturique,
acide 1,3-diméthyl-5-[4-(5-cyclohexyl-1,3,4-thiadiazole-2-yloxy)-phénylcarbamoyl]-barbiturique,
acide 1,3-diméthyt-5-[4-(1-méthyt-3-pentafiuoréthy)-1H-1,2,4-triazote-5-ybxy)-phényicarbamoyl]-barbitun- que,
acide 1,3-diméthyl-5-[4-(6-méthoxy-benzothiazole-2-yloxy)-phénylcarbamoyl]-barbiturique,
acide 1,3-diméthyl-5-[4-(5-cyclopropyl-1,3,4-triadiazole-2-yloxy)-phénylcarbamoyl]-barbiturique,
acide 1,3-diméthyl-5-[4-(6-chloro-7-fluoro-benzothiazole-2-yloxy)-phénylcarbamoyl]-barbiturique,
acide 1-méthyl-3-méthoxy-5-[4-(6-chloro-7-fluoro-benzothiazole-2-yloxy)-phénylcarbamoyl]-barbiturique, et
acide 1,3-diméthyl-5-[3-méthoxy-4-(6-chloro-7-fluorobenzothiazole-2-yloxy)-phénylcarbamoyl]-barbiturique.

17. Procédé de préparation d'un composé de formule 1 selon l'une des revendications 1 à 16, caractérisé en ce que :
a) on fait réagir à des températures de 50 à 250 °C un ester de formule Il 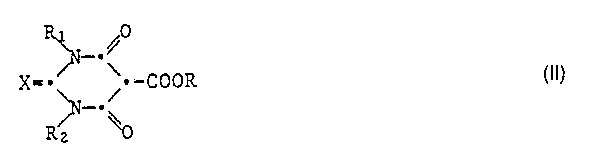 avec un dérivé de l'aniline de formule III 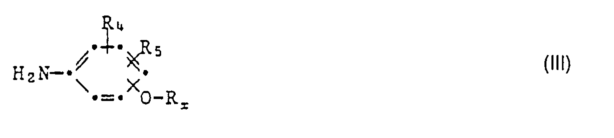 R représentant un groupe alkyle inférieur ou un groupe phényle éventuellement substitué par des groupes nitro, ou bien
b) on fait réagir à des températures de 0 à 220 °C un acide barbiturique substitué de formule IV 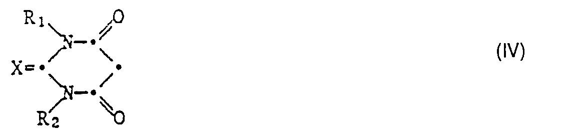 avec un isocyanate de phényle substitué de formule V 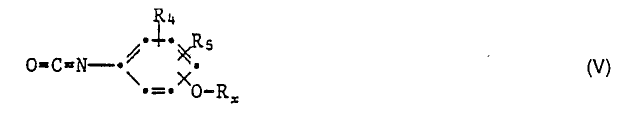 ou bien
c) on fait réagir à des températures de 50 à 250 °C un acide barbiturique substitué de formule IV avec un benzoylazide substitué de formule VI 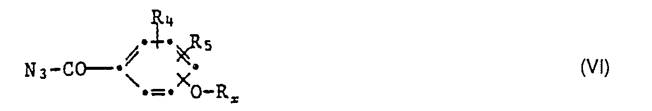 les symboles X, R₁, R₂, R₄ et R₅ ayant dans les formules II à VI les significations indiquées en référence à la formule 1 et Rₓ représentant l'hydrogène ou l'un des substituants mentionnés en référence à R₃, et dans les cas où Rₓ représente l'hydrogène, soit qu'au stade des produits de départ, on éthérifie à des températures de 50 à 150 °C l'un des dérivés hydroxylés (Rₓ = H) de formules III, V ou VI par un composé de formule XX 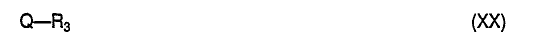 dans laquelle Q représente une fonction éliminable usuelle, et on poursuit les réactions jusqu'au produit final, soit qu'on convertisse d'abord les dérivés hydroxylés de formules III, V ou VI, à l'aide de l'un des composés II ou IV, en un dérivé hydroxylé (Rₓ = H) de formule l' dans laquelle X, R₁, R₂, R₄ et R₅ ont les significations indiquées en référence à la formule I et Rₓ représente l'hydrogène, après quoi seulement, on éthérifie le dérivé hydroxylé de formule l' par le composé de formule XX, les réaction a), b) et c) étant effectuées à pression normale ou sous pression et en présence ou en l'absence de solvants ou diluants inertes dans les réactions.

18. Procédé selon la revendication 17, caractérisé en ce que les réactions a), b) et c) sont effectuées en présence des solvants ou diluants inertes dans la réaction.

19. Procédé selon les revendications 17 et 18, caractérisé en ce que les variantes opératoires a) et c) sont mises en oeuvre à des températures de 70 à 220 °C, la variante opératoire b) à des températures de 0 à 200 °C et l'éthérification à des températures de 80 à 120 °C.

20. Procédé selon les revendications 17 et 19, caractérisé en ce que les réactions a), b) et c) sont effectuées en présence d'une base.

21. Composé de formule 1 selon l'une des revendications 1 à 16, pour l'utilisation dans un procédé pour combattre les helminthes parasitaires chez les animaux utiles.

22. Agent selon l'une des revendications 1 à 16, pour l'utilisation dans un procédé pour combattre les helminthes parasitaires chez les animaux utiles.

23. Utilisation d'un composé de formule 1 selon l'une des revendications 1 à 16, pour la préparation d'agents anti-helminthiques.
